# FASCICULE DE BREVET EUROPEEN

(11) **EP 2 316 925 B1**
(45) Date de publication et mention de la délivrance du brevet: **08.07.2015**
(21) Numéro de dépôt: 10184556.8
(22) Date de dépôt: 04.12.1998
(51) Int. Cl.: C12N 7/00, C12N 7/04, C07K 14/01, C12N 5/10, A01K 67/027, C12Q 1/68, C07K 16/08, G01N 33/569, A61K 39/12

(54) **Séquences de circovirus associé à la maladie de l'amaigrissement du porcelet (MAP)**
Circovirus-Sequenzen im Zusammenhang mit PWMS (Post-weaning-Multi-systemic wasting-syndrom)
Circovirus sequences associated with porcine wasting disease (PWD)

(30) Priorité: 05.12.1997 FR 9715396
(43) Date de publication de la demande: 04.05.2011
(62) Demande divisionnaire de: 08154913.1
(73) Titulaire: Zoetis W LLC, Florham Park NJ 07932 (US)
(72) Inventeur: Jestin, André, 22000, Saint Brieuc (FR); Albina, Emmanuel, 34730, Prades le Lez (FR); Le Cann, Pierre, 56400, Auray (FR); Blanchard, Philippe, 06250, Mougins le Haut (FR); Hutet, Evelyne, 22190, Plerin (FR); Arnault, Claire, 22510, Saint Trimoel (FR); Truong, Catherine, 72000, Le Mans (FR); Mahe, Dominique, 67720, Hoerdt (FR); Cariolet, Roland, 22440, Ploufragan (FR); Madec, François, 22000, Saint Brieuc (FR)
(74) Mandataire: Regimbeau

(56) Documents cités:
- WO-A-99/18214
- WO-A-99/29717
- MEEHAN B M ET AL: "SEQUENCE OF PORCINE CIRCOVIRUS DNA: AFFINITIES WITH PLANT CIRCOVIRUSES", JOURNAL OF GENERAL VIROLOGY., vol. 78, no. 1, janvier 1997 (1997-01), pages 221-227, XP002068398, READING GB
- MANKERTZ, A. ET AL.: "Mapping and characterization of the origin of DNA replication of porcine circovirus", JOURNAL OF VIROLOGY., vol. 71, no. 3, mars 1997 (1997-03), pages 2562-2566, XP002078782, ICAN SOCIETY FOR MICROBIOLOGY US
- HAMEL, A.L. ET AL.: "Nucleotide sequence of Porcine Circovirus associated with postweaning multisystemic wasting syndrome in pigs", JOURNAL OF VIROLOGY, vol. 72, no. 6, juin 1998 (1998-06), pages 5262-5267, XP002078783,
- MOROZOV I ET AL: "Detection of a novel strain of porcine circovirus in pigs with postweaning multisystemic wasting syndrome", JOURNAL OF CLINICAL MICROBIOLOGY, vol. 36, no. 9, septembre 1998 (1998-09), pages 2535-2541, XP002090921,
- MEEHAN B M ET AL: "Characterization of novel circovirus DNAs associated with wasting syndromes in pigs", JOURNAL OF GENERAL VIROLOGY, vol. 79, no. 9, septembre 1998 (1998-09), pages 2171-2179, XP002090386,
- MAHÉ D ET AL: "Differential recognition of ORF2 protein from type 1 and type 2 porcine circoviruses and identification of immunorelevant epitopes", JOURNAL OF GENERAL VIROLOGY, SOCIETY FOR GENERAL MICROBIOLOGY, SPENCERS WOOD, GB, vol. 81, 1 January 2000 (2000-01-01), pages 1815-1824, XP002978779, ISSN: 0022-1317

### LISTAGE DE SEQUENCES

<110> WYETH LLC
<120> SEQUENCES DE CIRCOVIRUS ASSOCIE
   A LA MALADIE DE L'AMAIGRISSEMENT
   DU PORCELET (MAP)
<130> D17221
<140> EP 08154913.1
   <141> 1998-12-04
<150> FR 97 15396
   <151> 1997-12-05
<150> PCT/FR98/02634
   <151> 1998-12-04
<150> EP 98958957.7
   <151> 1998-12-04
<160> 20
<170> PatentIn Vers. 2.0
<210> 1
   <211> 1759
   <212> ADN
   <213> Circovirus MAP type A
<220>
   <223> Brin polarité + (5'-3')
<400> 1
   accagcgcac ttcggcagcg gcagcacctc ggcagcgtca gtgaaaatgc caagcaagaa 60
   aagcggcccg caaccccata agaggtgggt gttcaccctt aataatcctt ccgaggagga 120
   gaaaaacaaa atacgggagc ttccaatctc cctttttgat tattttgttt gtggcgagga 180
   aggtttggaa gagggtagaa ctcctcacct ccaggggttt gcgaattttg ctaagaagca 240
   gacttttaac aaggtgaagt ggtattttgg tgcccgctgc cacatcgaga aagcgaaagg 300
   aaccgaccag cagaataaag aatactgcag taaagaaggc cacatactta tcgagtgtgg 360
   agctccgcgg aaccagggga agcgcagcga cctgtctact gctgtgagta cccttttgga 420
   gacggggtct ttggtgactg tagccgagca gtttcctgta acgtatgtga gaaatttccg 480
   cgggctggct gaacttttga aagtgagcgg gaagatgcag aagcgtgatt ggaagacagc 540
   tgtacacgtc atagtgggcc cgcccggttg tgggaagagc cagtgggccc gtaattttgc 600
   tgagcctagg gacacctact ggaagcctag tagaaataag tggtgggatg gatatcatgg 660
   agaagaagtt gttgttttgg atgattttta tggctggtta ccttgggatg atctactgag 720
   actgtgtgac cggtatccat tgactgtaga gactaaaggg ggtactgttc cttttttggc 780
   ccgcagtatt ttgattacca gcaatcaggc cccccaggaa tggtactcct caactgctgt 840
   cccagctgta gaagctctct atcggaggat tactactttg caattttgga agactgctgg 900
   agaacaatcc acggaggtac ccgaaggccg atttgaagca gtggacccac cctgtgccct 960
   tttcccatat aaaataaatt actgagtctt ttttgttatc acatcgtaat ggtttttatt 1020
   tttattcatt tagagggtct ttcaggataa attctctgaa ttgtacataa atagtcaacc 1080
   ttaccacata attttgggct gtggttgcat tttggagcgc atagcccagg cctgtgtgct 1140
   cgacattggt gtgggtattt aaatggagcc acagctggtt tcttttatta tttggctgga 1200
   accaatcaat tgtttggtct agctctggtt tgggggtgaa gtacctggag tggtaggtaa 1260
   agggctgcct tatggtgtgg cgggaggagt agttaatata ggggtcatag gccaagttgg 1320
   tggagggggt tacaaagttg gcatccaaga taacaacagt ggacccaaca cctctttgat 1380
   tagaggtgat ggggtctctg gggtaaaatt catatttagc ctttctaata cggtagtatt 1440
   ggaaaggtag ggggtagggg ttggtgccgc ctgagggggg gaggaactgg ccgatgttga 1500
   atctcagctc gttaacattc caagatggct gcgagtgtcc tcctcttatg gtgagtacaa 1560
   attctctaga aaggcgggaa ttgaagatac ccgtctttcg gcgccatctg taacggtttc 1620
   tgaaggcggg gtgtaccaaa tatggtcttc tccggaggat gtttccaaga tggctgcggg 1680
   ggcgggtccg tcttctgcgg taacgcctcc ttggccacgt catcctataa aagtgaaaga 1740
   agtgcgctgc tgtagtatt                                              1759
<210> 2
   <211> 1759
   <212> ADN
   <213> Circovirus MAP type A
<220>
   <223> Brin polarité - (5'-3')
<400> 2
   aatactacag cagcgcactt ctttcacttt tataggatga cgtggccaag gaggcgttac 60
   cgcagaagac ggacccgccc ccgcagccat cttggaaacg tcctccggag aagaccatat 120
   ttggtacacc ccgccttcag aaaccgttac agatggcgcc gaaagacggg tatcttcaat 180
   tcccgccttt ctagagaatt tgtactcacc ataagaggag gacactcgca gccatcttgg 240
   aatgttaacg agctgagatt caacatcggc cagttcctcc ccccctcagg cggcaccaac 300
   cccctacccc tacctttcca atactaccgt attagaaagg ctaaatatga attttacccc 360
   agagacccca tcacctctaa tcaaagaggt gttgggtcca ctgttgttat cttggatgcc 420
   aactttgtaa ccccctccac caacttggcc tatgacccct atattaacta ctcctcccgc 480
   cacaccataa ggcagccctt tacctaccac tccaggtact tcacccccaa accagagcta 540
   gaccaaacaa ttgattggtt ccagccaaat aataaaagaa accagctgtg gctccattta 600
   aatacccaca ccaatgtcga gcacacaggc ctgggctatg cgctccaaaa tgcaaccaca 660
   gcccaaaatt atgtggtaag gttgactatt tatgtacaat tcagagaatt tatcctgaaa 720
   gaccctctaa atgaataaaa ataaaaacca ttacgatgtg ataacaaaaa agactcagta 780
   atttatttta tatgggaaaa gggcacaggg tgggtccact gcttcaaatc ggccttcggg 840
   tacctccgtg gattgttctc cagcagtctt ccaaaattgc aaagtagtaa tcctccgata 900
   gagagcttct acagctggga cagcagttga ggagtaccat tcctgggggg cctgattgct 960
   ggtaatcaaa atactgcggg ccaaaaaagg aacagtaccc cctttagtct ctacagtcaa 1020
   tggataccgg tcacacagtc tcagtagatc atcccaaggt aaccagccat aaaaatcatc 1080
   caaaacaaca acttcttctc catgatatcc atcccaccac ttatttctac taggcttcca 1140
   gtaggtgtcc ctaggctcag caaaattacg ggcccactgg ctcttcccac aaccgggcgg 1200
   gcccactatg acgtgtacag ctgtcttcca atcacgctgc tgcatcttcc cgctcacttt 1260
   caaaagttca gccagcccgc ggaaatttct cacatacgtt acaggaaact gctcggctac 1320
   agtcaccaaa gaccccgtct ccaaaagggt actcacagca gtagacaggt cgctgcgctt 1380
   cccctggttc cgcggagctc cacactcgat aagtatgtgg ccttctttac tgcagtattc 1440
   tttattctgc tggtcggttc ctttcgcttt ctcgatgtgg cagcgggcac caaaatacca 1500
   cttcaccttg ttaaaagtct gcttcttagc aaaattcgca aacccctgga ggtgaggagt 1560
   tctaccctct tccaaacctt cctcgccaca aacaaaataa tcaaaaaggg agattggaag 1620
   ctcccgtatt ttgtttttct cctcctcgga aggattatta agggtgaaca cccacctctt 1680
   atggggttgc gggccgcttt tcttgcttgg cattttcact gacgctgccg aggtgctgcc 1740
   gctgccgaag tgcgctggt                                               1759
<210> 3
   <211> 939
   <212> ADN
   <213> Circovirus MAP type A
<220>
   <223> ORF1
<400> 3
   atgccaagca agaaaagcgg cccgcaaccc cataagaggt gggtgttcac ccttaataat 60
   ccttccgagg aggagaaaaa caaaatacgg gagcttccaa tctccctttt tgattatttt 120
   gtttgtggcg aggaaggttt ggaagagggt agaactcctc acctccaggg gtttgcgaat 180
   tttgctaaga agcagacttt taacaaggtg aagtggtatt ttggtgcccg ctgccacatc 240
   gagaaagcga aaggaaccga ccagcagaat aaagaatact gcagtaaaga aggccacata 300
   cttatcgagt gtggagctcc gcggaaccag gggaagcgca gcgacctgtc tactgctgtg 360
   agtacccttt tggagacggg gtctttggtg actgtagccg agcagtttcc tgtaacgtat 420
   gtgagaaatt tccgcgggct ggctgaactt ttgaaagtga gcgggaagat gcagcagcgt 480
   gattggaaga cagctgtaca cgtcatagtg ggcccgcccg gttgtgggaa gagccagtgg 540
   gcccgtaatt ttgctgagcc tagggacacc tactggaagc ctagtagaaa taagtggtgg 600
   gatggatatc atggagaaga agttgttgtt ttggatgatt tttatggctg gttaccttgg 660
   gatgatctac tgagactgtg tgaccggtat ccattgactg tagagactaa agggggtact 720
   gttccttttt tggcccgcag tattttgatt accagcaatc aggcccccca ggaatggtac 780
   tcctcaactg ctgtcccagc tgtagaagct ctctatcgga ggattactac tttgcaattt 840
   tggaagactg ctggagaaca atccacggag gtacccgaag gccgatttga agcagtggac 900
   ccaccctgtg ccctttcccc atataaaata aattactga                        939
<210> 4
   <211> 702
   <212> ADN
   <213> Circovirus MAP type A
<220>
   <223> ORF2
<400> 4
   atgacgtggc caaggaggcg ttaccgcaga agacggaccc gcccccgcag ccatcttgga 60
   aacatcctcc ggagaagacc atatttggta caccccgcct tcagaaaccg ttacagatgg 120
   cgccgaaaga cgggtatctt caattcccgc ctttctagag aatttgtact caccataaga 180
   ggaggacact cgcagccatc ttggaatgtt aacgagctga gattcaacat cggccagttc 240
   ctccccccct caggcggcac caacccccta cccctacctt tccaatacta ccgtattaga 300
   aaggctaaat atgaatttta ccccagagac cccatcacct ctaatcaaag aggtgttggg 360
   tccactgttg ttatcttgga tgccaacttt gtaaccccct ccaccaactt ggcctatgac 420
   ccctatatta actactcctc ccgccacacc ataaggcagc cctttaccta ccactccagg 480
   tacttcaccc ccaaaccaga gctagaccaa acaattgatt ggttccagcc aaataataaa 540
   agaaaccagc tgtggctcca tttaaatacc cacaccaatg tcgagcacac aggcctgggc 600
   tatgcgctcc aaaatgcaac cacagcccaa aattatgtgg taaggttgac tatttatgta 660
   caattcagag aatttatcct gaaagaccct ctaaatgaat aa                    702
<210> 5
   <211> 621
   <212> ADN
   <213> Circovirus MAP type A
<220>
   <223> ORF3
<400> 5
   atgatatcca tcccaccact tatttctact aggcttccag taggtgtccc taggctcagc 60
   aaaattacgg gcccactggc tcttcccaca accgggcggg cccactatga cgtgtacagc 120
   tgtcttccaa tcacgctgct gcatcttccc gctcactttc aaaagttcag ccagcccgcg 180
   gaaatttctc acatacgtta caggaaactg ctcggctaca gtcaccaaag accccgtctc 240
   caaaagggta ctcacagcag tagacaggtc gctgcgcttc ccctggttcc gcggagctcc 300
   acactcgata agtatgtggc cttctttact gcagtattct ttattctgct ggtcggttcc 360
   tttcgctttc tcgatgtggc agcgggcacc aaaataccac ttcaccttgt taaaagtctg 420
   cttcttagca aaattcgcaa acccctggag gtgaggagtt ctaccctctt ccaaaccttc 480
   ctcgccacaa acaaaataat caaaaaggga gattggaagc tcccgtattt tgtttttctc 540
   ctcctcggaa ggattattaa gggtgaacac ccacctctta tggggttgcg ggccgctttt 600
   cttgcttggc attttcactg a                                           621
<210> 6
   <211> 312
   <212> PRT
   <213> Circovirus MAP type A
<400> 6
   Met Pro Ser Lys Lys Ser Gly Pro Gln Pro His Lys Arg Trp Val Phe
   1               5                   10                  15
   Thr Leu Asn Asn Pro Ser Glu Glu Glu Lys Asn Lys Ile Arg Glu Leu
   20 25 30
   Pro Ile Ser Leu Phe Asp Tyr Phe Val Cys Gly Glu Glu Gly Leu Glu
   35 40 45
   Glu Gly Arg Thr Pro His Leu Gln Gly Phe Ala Asn Phe Ala Lys Lys
   50 55 60
   Gln Thr Phe Asn Lys Val Lys Trp Tyr Phe Gly Ala Arg Cys His Ile
   65 70 75 80
   Glu Lys Ala Lys Gly Thr Asp Gln Gln Asn Lys Glu Tyr Cys Ser Lys
   85 90 95
   Glu Gly His Ile Leu Ile Glu Cys Gly Ala Pro Arg Asn Gln Gly Lys
   100 105 110
   Arg Ser Asp Leu Ser Thr Ala Val Ser Thr Leu Leu Glu Thr Gly Ser
   115 120 125
   Leu Val Thr Val Ala Glu Gln Phe Pro Val Thr Tyr Val Arg Asn Phe
   130 135 140
   Arg Gly Leu Ala Glu Leu Leu Lys Val Ser Gly Lys Met Gln Gln Arg
   145 150 155 160
   Asp Trp Lys Thr Ala Val His Val Ile Val Gly Pro Pro Gly Cys Gly
   165 170 175
   Lys Ser Gln Trp Ala Arg Asn Phe Ala Glu Pro Arg Asp Thr Tyr Trp
   180 185 190
   Lys Pro Ser Arg Asn Lys Trp Trp Asp Gly Tyr His Gly Glu Glu Val
   195 200 205
   Val Val Leu Asp Asp Phe Tyr Gly Trp Leu Pro Trp Asp Asp Leu Leu
   210 215 220
   Arg Leu Cys Asp Arg Tyr Pro Leu Thr Val Glu Thr Lys Gly Gly Thr
   225 230 235 240
   Val Pro Phe Leu Ala Arg Ser Ile Leu Ile Thr Ser Asn Gln Ala Pro
   245 250 255
   Gln Glu Trp Tyr Ser Ser Thr Ala Val Pro Ala Val Glu Ala Leu Tyr
   260 265 270
   Arg Arg Ile Thr Thr Leu Gln Phe Trp Lys Thr Ala Gly Glu Gln Ser
   275 280 285
   Thr Glu Val Pro Glu Gly Arg Phe Glu Ala Val Asp Pro Pro Cys Ala
   290 295 300
   Leu Phe Pro Tyr Lys Ile Asn Tyr
   305 310
<210> 7
   <211> 233
   <212> PRT
   <213> Circovirus MAP type A
<400> 7
   Met Thr Trp Pro Arg Arg Arg Tyr Arg Arg Arg Arg Thr Arg Pro Arg
    1               5                  10                  15
   Ser His Leu Gly Asn Ile Leu Arg Arg Arg Pro Tyr Leu Val His Pro
               20                  25                  30
   Ala Phe Arg Asn Arg Tyr Arg Trp Arg Arg Lys Thr Gly Ile Phe Asn
           35                  40                  45
   Ser Arg Leu Ser Arg Glu Phe Val Leu Thr Ile Arg Gly Gly His Ser
       50                  55                  60
   Gln Pro Ser Trp Asn Val Asn Glu Leu Arg Phe Asn Ile Gly Gln Phe
    65                 70                  75                  80
   Leu Pro Pro Ser Gly Gly Thr Asn Pro Leu Pro Leu Pro Phe Gln Tyr
                   85                  90                  95
   Tyr Arg Ile Arg Lys Ala Lys Tyr Glu Phe Tyr Pro Arg Asp Pro Ile
               100                 105                 110
   Thr Ser Asn Gln Arg Gly Val Gly Ser Thr Val Val Ile Leu Asp Ala
           115                 120                 125
   Asn Phe Val Thr Pro Ser Thr Asn Leu Ala Tyr Asp Pro Tyr Ile Asn
       130                 135                 140
   Tyr Ser Ser Arg His Thr Ile Arg Gln Pro Phe Thr Tyr His Ser Arg
   145                 150                 155                 160
   Tyr Phe Thr Pro Lys Pro Glu Leu Asp Gln Thr Ile Asp Trp Phe Gln
                   165                 170                 175
   Pro Asn Asn Lys Arg Asn Gln Leu Trp Leu His Leu Asn Thr His Thr
               180                 185                 190
   Asn Val Glu His Thr Gly Leu Gly Tyr Ala Leu Gln Asn Ala Thr Thr
           195                 200                 205
   Ala Gln Asn Tyr Val Val Arg Leu Thr Ile Tyr Val Gln Phe Arg Glu
       210                 215                 220
   Phe Ile Leu Lys Asp Pro Leu Asn Glu
   225                 230
<210> 8
   <211> 206
   <212> PRT
   <213> Circovirus MAP type A
<400> 8
   Met Ile Ser Ile Pro Pro Leu Ile Ser Thr Arg Leu Pro Val Gly Val
    1               5                  10                  15
   Pro Arg Leu Ser Lys Ile Thr Gly Pro Leu Ala Leu Pro Thr Thr Gly
               20                  25                  30
   Arg Ala His Tyr Asp Val Tyr Ser Cys Leu Pro Ile Thr Leu Leu His
           35                  40                  45
   Leu Pro Ala His Phe Gln Lys Phe Ser Gln Pro Ala Glu Ile Ser His
       50                  55                  60
   Ile Arg Tyr Arg Lys Leu Leu Gly Tyr Ser His Gln Arg Pro Arg Leu
   65                  70                  75                  80
   Gln Lys Gly Thr His Ser Ser Arg Gln Val Ala Ala Leu Pro Leu Val
                   85                  90                  95
   Pro Arg Ser Ser Thr Leu Asp Lys Tyr Val Ala Phe Phe Thr Ala Val
               100                 105                 110
   Phe Phe Ile Leu Leu Val Gly Ser Phe Arg Phe Leu Asp Val Ala Ala
           115                 120                 125
   Gly Thr Lys Ile Pro Leu His Leu Val Lys Ser Leu Leu Leu Ser Lys
       130                 135                 140
   Ile Arg Lys Pro Leu Glu Val Arg Ser Ser Thr Leu Phe Gln Thr Phe
   145                 150                 155                 160
   Leu Ala Thr Asn Lys Ile Ile Lys Lys Gly Asp Trp Lys Leu Pro Tyr
                   165                 170                 175
   Phe Val Phe Leu Leu Leu Gly Arg Ile Ile Lys Gly Glu His Pro Pro
               180                 185                 190
   Leu Met Gly Leu Arg Ala Ala Phe Leu Ala Trp His Phe His
           195                 200                 205
<210> 9
   <211> 1767
   <212> ADN
   <213> Circovirus MAP type B
<220>
   <223> Brin polarité + (5'-3')
<400> 9
   accagcgcac ttcggcagcg gcagcacctc ggcagcacct cagcagcaac atgcccagca 60
   agaagaatgg aagaagcgga ccccaacccc ataaaaggtg ggtgttcact ctgaataatc 120
   cttccgaaga cgagcgcaag aaaatacggg atcttccaat atccctattt gattatttta 180
   ttgttggcga ggagggtaat gaggaaggac gaacacctca cctccagggg ttcgctaatt 240
   ttgtgaagaa gcagactttt aataaagtga agtggtattt gggtgcccgc tgccacatcg 300
   agaaagcgaa aggaacagat cagcagaata aagaatactg cagtaaagaa ggcaacttac 360
   tgatggagtg tggagctcct agatctcagg gacaacggag tgacctgtct actgctgtga 420
   gtaccttgtt ggagagcggg agtctggtga ccgttgcaga gcagcaccct gtaacgtttg 480
   tcagaaattt ccgcgggctg gctgaacttt tgaaagtgag cgggaaaatg cagaagcgtg 540
   attggaagac taatgtacac gtcattgtgg ggccacctgg gtgtggtaaa agcaaatggg 600
   ctgctaattt tgcagacccg gaaaccacat actggaaacc acctagaaac aagtggtggg 660
   atggttacca tggtgaagaa gtggttgtta ttgatgactt ttatggctgg ctgccctggg 720
   atgatctact gagactgtgt gatcgatatc cattgactgt agagactaaa ggtggaactg 780
   tacctttttt ggcccgcagt attctgatta ccagcaatca gaccccgttg gaatggtact 840
   cctcaactgc tgtcccagct gtagaagctc tttatcggag gattacttcc ttggtatttt 900
   ggaagaatgc tacagaacaa tccacggagg aagggggcca gttcgtcacc ctttcccccc 960
   catgccctga atttccatat gaaataaatt actgagtctt ttttatcact tcgtaatggt 1020
   ttttattatt cattaagggt taagtggggg gtctttaaaa ttaaattctc tgaattgtac 1080
   atacatggtt acacggatat tgtattcctg gtcgtatata ctgttttcga acgcagtgcc 1140
   gaggcctacg tggtctacat ttccagcagt ttgtagtctc agccacagct ggtttctttt 1200
   gttgtttggt tggaagtaat caatagtgaa atctaggaca ggtttggggg taaagtaccg 1260
   ggagtggtag gagaagggct gggttatggt atggcgggag gagtagttta cataggggtc 1320
   ataggtgagg gctgtggcct ttgttacaaa gttatcatct aaaataacag cactggagcc 1380
   cactcccctg tcaccctggg tgatcgggga gcagggccag aattcaacct taacctttct 1440
   tattctgtag tattcaaagg gcacagagcg ggggtttgac ccccctcctg ggggaagaaa 1500
   gtcattaata ttgaatctca tcatgtccac cgcccaggag ggcgttctga ctgtggttcg 1560
   cttgacagta tatccgaagg tgcgggagag gcgggtgttg aagatgccat ttttccttct 1620
   ccagcggtaa cggtggcggg ggtggacgag ccaggggcgg cggcggagga tctggccaag 1680
   atggctgcgg gggcggtgtc ttcttcttcg gtaacgcctc cttggatacg tcatatctga 1740
   aaacgaaaga agtgcgctgt aagtatt                                1767
<210> 10
   <211> 1767
   <212> ADN
   <213> Circovirus MAP type B
<220>
   <223> Brin polarité - (5'-3')
<400> 10
   aatacttaca gcgcacttct ttcgttttca gatatgacgt atccaaggag gcgttaccga 60
   agaagaagac accgcccccg cagccatctt ggccagatcc tccgccgccg cccctggctc 120
   gtccaccccc gccaccgtta ccgctggaga aggaaaaatg gcatcttcaa cacccgcctc 180
   tcccgcacct tcggatatac tgtcaagcga accacagtca gaacgccctc ctgggcggtg 240
   gacatgatga gattcaatat taatgacttt cttcccccag gaggggggtc aaacccccgc 300
   tctgtgccct ttgaatacta cagaataaga aaggttaagg ttgaattctg gccctgctcc 360
   ccgatcaccc agggtgacag gggagtgggc tccagtgctg ttattttaga tgataacttt 420
   gtaacaaagg ccacagccct cacctatgac ccctatgtaa actactcctc ccgccatacc 480
   ataacccagc ccttctccta ccactcccgg tactttaccc ccaaacctgt cctagatttc 540
   actattgatt acttccaacc aaacaacaaa agaaaccagc tgtggctgag actacaaact 600
   gctggaaatg tagaccacgt aggcctcggc actgcgttcg aaaacagtat atacgaccag 660
   gaatacaata tccgtgtaac catgtatgta caattcagag aatttaattt taaagacccc 720
   ccacttaacc cttaatgaat aataaaaacc attacgaagt gataaaaaag actcagtaat 780
   ttatttcata tggaaattca gggcatgggg gggaaagggt gacgaactgg cccccttcct 840
   ccgtggattg ttctgtagca ttcttccaaa ataccaagga agtaatcctc cgataaagag 900
   cttctacagc tgggacagca gttgaggagt accattccaa cggggtctga ttgctggtaa 960
   tcagaatact gcgggccaaa aaaggtacag ttccaccttt agtctctaca gtcaatggat 1020
   atcgatcaca cagtctcagt agatcatccc agggcagcca gccataaaag tcatcaataa 1080
   caaccacttc ttcaccatgg taaccatccc accacttgtt tctaggtggt ttccagtatg 1140
   tggtttccgg gtctgcaaaa ttagcagccc atttgctttt accacaccca ggtggcccca 1200
   caatgacgtg tacattagtc ttccaatcac gcttctgcat tttcccgctc actttcaaaa 1260
   gttcagccag cccgcggaaa tttctgacaa acgttacagg gtgctgctct gcaacggtca 1320
   ccagactccc gctctccaac aaggtactca cagcagtaga caggtcactc cgttgtccct 1380
   gagatctagg agctccacac tccatcagta agttgccttc tttactgcag tattctttat 1440
   tctgctgatc tgttcctttc gctttctcga tgtggcagcg ggcacccaaa taccacttca 1500
   ctttattaaa agtctgcttc ttcacaaaat tagcgaaccc ctggaggtga ggtgttcgtc 1560
   cttcctcatt accctcctcg ccaacaataa aataatcaaa tagggatatt ggaagatccc 1620
   gtattttctt gcgctcgtct tcggaaggat tattcagagt gaacacccac cttttatggg 1680
   gttggggtcc gcttcttcca ttcttcttgc tgggcatgtt gctgctgagg tgctgccgag 1740
   gtgctgccgc tgccgaagtg cgctggt                                    1767
<210> 11
   <211> 945
   <212> ADN
   <213> Circovirus MAP type B
<220>
   <223> ORF1
<400> 11
   atgcccagca agaagaatgg aagaagcgga ccccaacccc ataaaaggtg ggtgttcact 60
   ctgaataatc cttccgaaga cgagcgcaag aaaatacggg atcttccaat atccctattt 120
   gattatttta ttgttggcga ggagggtaat gaggaaggac gaacacctca cctccagggg 180
   ttcgctaatt ttgtgaagaa gcagactttt aataaagtga agtggtattt gggtgcccgc 240
   tgccacatcg agaaagcgaa aggaacagat cagcagaata aagaatactg cagtaaagaa 300
   ggcaacttac tgatggagtg tggagctcct agatctcagg gacaacggag tgacctgtct 360
   actgctgtga gtaccttgtt ggagagcggg agtctggtga ccgttgcaga gcagcaccct 420
   gtaacgtttg tcagaaattt ccgcgggctg gctgaacttt tgaaagtgag cgggaaaatg 480
   cagaagcgtg attggaagac taatgtacac gtcattgtgg ggccacctgg gtgtggtaaa 540
   agcaaatggg ctgctaattt tgcagacccg gaaaccacat actggaaacc acctagaaac 600
   aagtggtggg atggttacca tggtgaagaa gtggttgtta ttgatgactt ttatggctgg 660
   ctgccctggg atgatctact gagactgtgt gatcgatatc cattgactgt agagactaaa 720
   ggtggaactg tacctttttt ggcccgcagt attctgatta ccagcaatca gaccccgttg 780
   gaatggtact cctcaactgc tgtcccagct gtagaagctc tttatcggag gattacttcc 840
   ttggtatttt ggaagaatgc tacagaacaa tccacggagg aagggggcca gttcgtcacc 900
   ctttcccccc catgccctga atttccatat gaaataaatt actga                945
<210> 12
   <211> 702
   <212> ADN
   <213> Circovirus MAP type B
<220>
   <223> ORF2
<400> 12
   atgacgtatc caaggaggcg ttaccgaaga agaagacacc gcccccgcag ccatcttggc 60
   cagatcctcc gccgccgccc ctggctcgtc cacccccgcc accgttaccg ctggagaagg 120
   aaaaatggca tcttcaacac ccgcctctcc cgcaccttcg gatatactgt caagcgaacc 180
   acagtcagaa cgccctcctg ggcggtggac atgatgagat tcaatattaa tgactttctt 240
   cccccaggag gggggtcaaa cccccgctct gtgccctttg aatactacag aataagaaag 300
   gttaaggttg aattctggcc ctgctccccg atcacccagg gtgacagggg agtgggctcc 360
   agtgctgtta ttttagatga taactttgta acaaaggcca cagccctcac ctatgacccc 420
   tatgtaaact actcctcccg ccataccata acccagccct tctcctacca ctcccggtac 480
   tttaccccca aacctgtcct agatttcact attgattact tccaaccaaa caacaaaaga 540
   aaccagctgt ggctgagact acaaactgct ggaaatgtag accacgtagg cctcggcact 600
   gcgttcgaaa acagtatata cgaccaggaa tacaatatcc gtgtaaccat gtatgtacaa 660
   ttcagagaat ttaattttaa agacccccca cttaaccctt aa                    702
<210> 13
   <211> 315
   <212> ADN
   <213> Circovirus MAP type B
<220>
   <223> ORF3
<400> 13
   atggtaacca tcccaccact tgtttctagg tggtttccag tatgtggttt ccgggtctgc 60
   aaaattagca gcccatttgc ttttaccaca cccaggtggc cccacaatga cgtgtacatt 120
   agtcttccaa tcacgcttct gcattttccc gctcactttc aaaagttcag ccagcccgcg 180
   gaaatttctg acaaacgtta cagggtgctg ctctgcaacg gtcaccagac tcccgctctc 240
   caacaaggta ctcacagcag tagacaggtc actccgttgt ccctgagatc taggagctcc 300
   acactccatc agtaa                                                  315
<210> 14
   <211> 314
   <212> PRT
   <213> Circovirus MAP type B
<400> 14
   Met Pro Ser Lys Lys Asn Gly Arg Ser Gly Pro Gln Pro His Lys Arg
     1               5                  10                  15
   Trp Val Phe Thr Leu Asn Asn Pro Ser Glu Asp Glu Arg Lys Lys Ile
                20                  25                  30
   Arg Asp Leu Pro Ile Ser Leu Phe Asp Tyr Phe Ile Val Gly Glu Glu
            35                  40                  45
   Gly Asn Glu Glu Gly Arg Thr Pro His Leu Gln Gly Phe Ala Asn Phe
        50                  55                  60
   Val Lys Lys Gln Thr Phe Asn Lys Val Lys Trp Tyr Leu Gly Ala Arg
    65                  70                  75                  80
   Cys His Ile Glu Lys Ala Lys Gly Thr Asp Gln Gln Asn Lys Glu Tyr
                    85                  90                  95
   Cys Ser Lys Glu Gly Asn Leu Leu Met Glu Cys Gly Ala Pro Arg Ser
               100                 105                 110
   Gln Gly Gln Arg Ser Asp Leu Ser Thr Ala Val Ser Thr Leu Leu Glu
           115                 120                 125
   Ser Gly Ser Leu Val Thr Val Ala Glu Gln His Pro Val Thr Phe Val
       130                 135                 140
   Arg Asn Phe Arg Gly Leu Ala Glu Leu Leu Lys Val Ser Gly Lys Met
   145                 150                 155                 160
   Gln Lys Arg Asp Trp Lys Thr Asn Val His Val Ile Val Gly Pro Pro
                   165                 170                 175
   Gly Cys Gly Lys Ser Lys Trp Ala Ala Asn Phe Ala Asp Pro Glu Thr
               180                 185                 190
   Thr Tyr Trp Lys Pro Pro Arg Asn Lys Trp Trp Asp Gly Tyr His Gly
           195                 200                 205
   Glu Glu Val Val Val Ile Asp Asp Phe Tyr Gly Trp Leu Pro Trp Asp
       210                 215                 220
   Asp Leu Leu Arg Leu Cys Asp Arg Tyr Pro Leu Thr Val Glu Thr Lys
   225                 230                 235                 240
   Gly Gly Thr Val Pro Phe Leu Ala Arg Ser Ile Leu Ile Thr Ser Asn
                   245                 250                 255
   Gln Thr Pro Leu Glu Trp Tyr Ser Ser Thr Ala Val Pro Ala Val Glu
               260                 265                 270
   Ala Leu Tyr Arg Arg Ile Thr Ser Leu Val Phe Trp Lys Asn Ala Thr
           275                 280                 285
   Glu Gln Ser Thr Glu Glu Gly Gly Gln Phe Val Thr Leu Ser Pro Pro
       290                 295                 300
   Cys Pro Glu Phe Pro Tyr Glu Ile Asn Tyr
   305                 310
<210> 15
   <211> 233
   <212> PRT
   <213> Circovirus MAP type B
<400> 15
   Met Thr Tyr Pro Arg Arg Arg Tyr Arg Arg Arg Arg His Arg Pro Arg
    1               5                  10                  15
   Ser His Leu Gly Gln Ile Leu Arg Arg Arg Pro Trp Leu Val His Pro
               20                  25                  30
   Arg His Arg Tyr Arg Trp Arg Arg Lys Asn Gly Ile Phe Asn Thr Arg
           35                  40                  45
   Leu Ser Arg Thr Phe Gly Tyr Thr Val Lys Arg Thr Thr Val Arg Thr
       50                  55                  60
   Pro Ser Trp Ala Val Asp Met Met Arg Phe Asn Ile Asn Asp Phe Leu
   65                  70                  75                  80
   Pro Pro Gly Gly Gly Ser Asn Pro Arg Ser Val Pro Phe Glu Tyr Tyr
                   85                  90                  95
   Arg Ile Arg Lys Val Lys Val Glu Phe Trp Pro Cys Ser Pro Ile Thr
               100                 105                 110
   Gln Gly Asp Arg Gly Val Gly Ser Ser Ala Val Ile Leu Asp Asp Asn
           115                 120                 125
   Phe Val Thr Lys Ala Thr Ala Leu Thr Tyr Asp Pro Tyr Val Asn Tyr
       130                 135                 140
   Ser Ser Arg His Thr Ile Thr Gln Pro Phe Ser Tyr His Ser Arg Tyr
   145                 150                 155                 160
   Phe Thr Pro Lys Pro Val Leu Asp Phe Thr Ile Asp Tyr Phe Gln Pro
                   165                 170                 175
   Asn Asn Lys Arg Asn Gln Leu Trp Leu Arg Leu Gln Thr Ala Gly Asn
               180                 185                 190
   Val Asp His Val Gly Leu Gly Thr Ala Phe Glu Asn Ser Ile Tyr Asp
           195                 200                 205
   Gln Glu Tyr Asn Ile Arg Val Thr Met Tyr Val Gln Phe Arg Glu Phe
       210                 215                 220
   Asn Phe Lys Asp Pro Pro Leu Asn Pro
   225                 230
<210> 16
   <211> 104
   <212> PRT
   <213> Circovirus MAP type B
<400> 16
   Met Val Thr Ile Pro Pro Leu Val Ser Arg Trp Phe Pro Val Cys Gly
    1               5                   10                  15
   Phe Arg Val Cys Lys Ile Ser Ser Pro Phe Ala Phe Thr Thr Pro Arg
               20                  25                  30
   Trp Pro His Asn Asp Val Tyr Ile Ser Leu Pro Ile Thr Leu Leu His
           35                  40                  45
   Phe Pro Ala His Phe Gln Lys Phe Ser Gln Pro Ala Glu Ile Ser Asp
       50                  55                  60
   Lys Arg Tyr Arg Val Leu Leu Cys Asn Gly His Gln Thr Pro Ala Leu
   65                  70                  75                  80
   Gln Gln Gly Thr His Ser Ser Arg Gln Val Thr Pro Leu Ser Leu Arg
                   85                  90                  95
   Ser Arg Ser Ser Thr Leu His Gln
               100
<210> 17
   <211> 15
   <212> PRT
   <213> Circovirus MAP type B
<400> 17
   Val Asp Met Met Arg Phe Asn Ile Asn Asp Phe Leu Pro Pro Gly
    1               5                  10                  15
<210> 18
   <211> 15
   <212> PRT
   <213> Circovirus MAP type B
<400> 18
   Gln Gly Asp Arg Gly Val Gly Ser Ser Ala Val Ile Leu Asp Asp
    1               5                  10                  15
<210> 19
   <211> 15
   <212> PRT
   <213> Circovirus MAP type B
<400> 19
   Gly Val Gly Ser Ser Ala Val Ile Leu Asp Asp Asn Phe Val Thr
    1               5                  10                  15
<210> 20
   <211> 15
   <212> PRT
   <213> Circovirus MAP type B
<400> 20
   Val Asp His Val Gly Leu Gly Thr Ala Phe Glu Asn Ser Ile Tyr
     1             5                  10                  15

## Description

L'invention concerne des procédés de détection des polypeptides, des kits de diagnostic d'infection par le circovirus MAP de type B et des anticorps mono- ou polyclonaux capables de reconnaître de manière spécifique polypeptides isolé ayant la séquence SEQ ID N°17-20. La description a pour objet la séquence génomique et des séquences nucléotidiques codant pour des polypeptides de circovirus MAP, tels que les polypeptides structuraux et non structuraux dudit circovirus, ainsi que des vecteurs incluant lesdites séquences et cellules ou animaux transformés par ces vecteurs. La description concerne des procédés de détection de ces acides nucléiques, L'invention concerne des procédés de détection et/ou d'identification d'un circovirus MAP de type B dans un échantillon biologique, comprenant la mise en contact de l'échantillon biologique avec un anticorps mono-ou polyclonal selon l'invention et la mise en évidence des complexes antigène-anticorps éventuellement formés. L'invention concerne également des kits de diagnostic d'infection par le circovirus MAP de type B dans un échantillon biologique, comprenant un anticorps mono-ou polyclonal selon l'invention et un réactif pour la constitution d'un milieu propice à la réalisation d'une réaction antigéniques.

La description vise aussi une méthode de sélection de composés capables de moduler l'infection virale. La description comprend enfin des compositions pharmaceutiques, notamment vaccinales, pour la prévention et/ou le traitement d'infections virales par circovirus MAP ainsi que l'utilisation de vecteur selon la description pour la prévention et/ou le traitement de maladies par thérapie génique.

La maladie de l'amaigrissement du porcelet (MAP) ou encore appelé dépérissement fatal du porcelet (DFP) a été largement décrite en Amérique du Nord (Harding, J.C., 1997), et des auteurs ont rapporté l'existence d'une relation entre cette pathologie et la présence de circovirus porcin (Daft, B. et al., 1996 ; Clark, E.G., 1997 ; Harding, J.C., 1997 ; Harding, J.C. et Clark, E.G., 1997 ; Nayar, G.P. et al., 1997). Un circovirus porcin a déjà été mis en évidence dans des cultures cellulaires dérivées de porc établies en lignée et infectées chroniquement (Tischer, I., 1986, 1988, 1995 ; Dulac, G.C., 1989 ; Edwards, S., 1994 ; Allan, G.M., 1995 et McNeilly, F., 1996). Ce virus, lors d'infection expérimentale de porcelets, ne se révélait pas pathogène pour le porc (Tischer, I., 1986, Homer, G.W., 1991) et sa séquence nucléotidique a été déterminée et caractérisée (Tischer, I., 1982 ; Meehan, B.M.et al., 1997 ; Mankertz, A., 1997). Le circovirus porcin, dénommé virus PCV, fait partie du genre circovirus de la famille des circoviridae (Murphy, F.A. et al., 1995) dont le virion possède un ADN circulaire de taille compris entre 1,7 et 2,3 kb, ADN qui comprend 3 cadres ouverts de lecture (ORF1 à ORF3), codant pour une protéine de réplication REP impliquée dans la phase d'initiation et de terminaison de la réplication circulaire déroulante (RCR) (Heyraud-Nitschke, F., et al., 1995 ; Harding, M.R. et al., 1993 ; Hanson, S.F. et al., 1995 ; Fontes, E.P.B. et al., 1994), codant pour une protéine de capside (Boulton, L.H. et al., 1997 ; Hackland, A.F. et al., 1994 ; Chu, P.W.G. et al., 1993 et codant pour une protéine non structurale dite de dissémination (Lazarowitz, S.G. et al., 1989).

Les auteurs de la présente invention ont remarqué que les manifestations cliniques perceptibles chez le porc et liées à l'infection par le circovirus MAP, sont très individualisées. Ces manifestations apparaissent en général sur des porcs de 8 à 12 semaines d'âge, sevrés depuis 4 à 8 semaines. Les premiers signes sont l'hypotonie sans qu'on puisse parler de prostration. Rapidement (48 heures), les flancs se creusent, la ligne du dos se dessine, les porcs « blanchissent ». Ces signes s'accompagnent en général d'hyperthermie, d'anorexie et le plus souvent de manifestations respiratoires (toux, dyspnée, polypnée). Des diarrhées transitoires peuvent également apparaître. La phase d'état de la maladie dure environ un mois au terme de laquelle les taux de mortalité varient de 5 à 20 %. A ces mortalités il convient d'ajouter une proportion variable (5-10 %) d'animaux cadavériques ne pouvant plus représenter un avenir économique. Il est à noter qu'en dehors de ce stade critique de fin de post-sevrage, aucune anomalie n'apparaît dans les élevages. En particulier, la fonction de reproduction est parfaitement maintenue.

Sur le plan épidémiologique, les premières manifestations de cette pathologie sont apparues au début de 1995 dans l'Est du département des Côtes d'Armor en France, et les élevages atteints sont surtout cantonnés dans cette zone du département. En décembre 1996, le nombre des élevages concernés ne peut être évalué avec précision en raison de l'absence de méthode de diagnostic spécifique au laboratoire ni de dispositif d'épidémiosurveillance du cheptel. En se basant sur les faits cliniques ainsi que sur des résultats d'examens nécropsiques fournis par les vétérinaires, on peut estimer ce nombre à plusieurs dizaines (80-100). La contagiosité de la maladie est faible à modérée. Des cas sont signalés en dehors de la zone initiale et font suite pour la plupart au transfert d'animaux venant d'élevages connaissant le problème. En revanche, une particularité de l'affection est sa forte rémanence. Ainsi, des élevages atteints depuis une année sont-ils toujours concernés en dépit de l'application massive de thérapeutiques. Les élevages à expression clinique se recrutent dans les différentes catégories de spécialisation (naisseurs-engraisseurs, post-sevreurs-engraisseurs) et différentes structures économiques sont concernées. Par ailleurs, les troubles apparaissent même dans des élevages où les règles de la zootechnie sont respectées.

De nombreux examens nécropsiques ont été réalisés soit dans les élevages soit au laboratoire. Les éléments du tableau lésionnel sont disparates. Les lésions macroscopiques les plus constantes sont de la pneumonie qui se présente parfois en damier ainsi qu'une hypertrophie des ganglions lymphatiques. Les autres lésions concernent surtout les viscères thoraciques dont notamment péricardite et pleurésie. Mais des arthrites, des ulcères gastriques sont également observés. Les lésions révélées à l'examen histologique se situent essentiellement au niveau pulmonaire (pneumonie interstitielle), ganglionnaire (déplétion lymphoïde des noeuds lymphatiques, cellules géantes) et rénal (glomérulonéphrite, vascularite). Les agents infectieux ont fait l'objet de recherches larges. L'intervention des pestivirus et de la maladie d'Aujeszky a pu être exclue. Les troubles apparaissent dans les troupeaux SDRP (Syndrome Dysgénésique et Respiratoire Porcin, infection liée à un artériovirus) séropositif, mais le rôle de ce dernier dans la genèse des troubles n'a pu être établi (la majorité des élevages de Bretagne sont SDRP séropositifs).

Les auteurs de la présente invention, dans le but d'identifier l'agent étiologique responsable de la MAP, ont réalisé des épreuves de « contact » entre porcelets manifestement « malades » et des porcs EOPS (Exempts d'Organismes Pathogènes Spécifiques) du CNEVA (Centre National d'Etudes Vétérinaires et Alimentaires, France). Ces épreuves ont permis d'observer le développement dans les animaleries protégées des manifestations comparables à celles observées en élevage. Les manifestations discrètes telles que l'hyperthermie modérée, l'anorexie et la diarrhée intermittente, sont apparues après une semaine de contact. Il faut noter que le virus SDRP n'a diffusé que postérieurement aux manifestations cliniques. Par ailleurs, des inoculations de broyats d'organes d'animaux malades à des porcs sains a permis de reproduire des manifestations apparentées à celles observées dans les élevages, avec cependant une incidence moins forte liée aux conditions favorables d'entretien des animaux dans les installations expérimentales.

Ainsi, les auteurs de la présente invention ont pu mettre en évidence que les manifestations pathologiques se présentent comme une entité bien définie affectant le porc à un stade particulier de sa croissance.

Cette pathologie n'a jamais été décrite en France. Toutefois, des informations éparses notamment canadiennes relatent de faits apparentés.

Les troubles ne peuvent être maîtrisés par les thérapeutiques existantes.

Les données récoltées tant en élevage qu'en expérimentation ont permis de mettre en relief les points suivants :
- la maladie MAP est transmissible mais sa contagiosité est peu élevée,
- son origine étiologique est de nature infectieuse et probablement virale,
- la maladie MAP présente un caractère persistant dans les élevages atteints.

Il s'ensuit des conséquences économiques considérables pour les élevages.

Ainsi, un besoin important à ce jour concerne un diagnostic spécifique et sensible, de réalisation pratique et rapide, permettant le dépistage précoce de l'infection. Un test fiable, sensible et pratique, qui permette la distinction entre souches de circovirus porcin (PCV) est donc fortement désiré.

D'autre part, un besoin de traitement efficace, et bien toléré des infections à circovirus MAP reste également désiré, aucun vaccin aujourd'hui n'est disponible contre le circovirus MAP.

S'agissant du circovirus MAP, il faudra probablement comprendre le rôle de la défense immunitaire dans la physiologie et la pathologie de la maladie pour développer des vaccins satisfaisants.

Une information plus ample concernant la biologie de ces souches, leurs interactions avec leurs hôtes, les phénomènes d'infectivité associés et ceux d'échappement aux défenses immunitaires de l'hôte notamment, et leur implication enfin dans le développement des pathologies associées, permettra une meilleure compréhension de ces mécanismes. Compte tenu de ce qui précède et qui montre en particulier les limitations des moyens de lutter contre l'infection par le circovirus MAP, il est donc primordial aujourd'hui d'une part de développer des outils moléculaires, notamment à partir d'une meilleure connaissance génétique du circovirus MAP, mais également de mettre au point de nouveaux traitements préventifs et thérapeutiques, des nouvelles méthodes de diagnostic et de nouvelles stratégies vaccinales spécifiques, efficaces et tolérées. La présente invention a précisément pour objet une nouvelle méthode de diagnostic.

La présente invention a précisément pour objet une nouvelle méthode de diagnostic.

La présente description a pour objet les séquences nucléotidiques du génome de circovirus MAP choisies parmi les séquences SEQ ID N° 1, SEQ ID N° 2, SEQ ID N° 9, SEQ ID N° 10 ou un de leurs fragments.

Les séquences nucléotidiques de séquences SEQ ID N° 1 et SEQ ID N° 2 correspondent respectivement à la séquence génomique du brin de polarité (+) et du brin de polarité (-) du circovirus MAP de type A (ou PCVA), la séquence SEQ ID N° 2 étant représentée suivant l'orientation 5' → 3'.

Les séquences nucléotidiques de séquences SEQ ID N° 9 et SEQ ID N° 10 correspondent respectivement à la séquence génomique du brin de polarité (+) et du brin de polarité (-) du circovirus MAP de type B (ou PCVB) la séquence SEQ ID N° 10 étant représentée suivant l'orientation 5' → 3'.

La présente description a également pour objet des séquences nucléotidiques caractérisées en ce qu'elles sont choisies parmi :
a) une séquence nucléotidique de fragment spécifique d'une séquence SEQ ID N° 1, SEQ ID N° 2, SEQ ID N° 9, SEQ ID N° 10 ou un de leurs fragments ;
b) une séquence nucléotidique homologue à une séquence nucléotidique telle que définie en a) ;
c) une séquence nucléotidique complémentaire d'une séquence nucléotidique telle que définie en a) ou b), et une séquence nucléotidique de leur ARN correspondant ;
d) une séquence nucléotidique capable de s'hybrider dans des conditions stringentes avec une séquence telle que définie en a), b), ou c) ;
e) une séquence nucléotidique comprenant une séquence telle que définie en a), b), c) ou d) ; et
f) une séquence nucléotidique modifiée d'une séquence nucléotidique telle que définie en a), b), c), d) ou e).

On entendra par séquence nucléotidique, polynucléotide ou acide nucléique, selon la présente description, aussi bien un ADN double brin ou simple brin dans des formes monomériques et dimériques (dites en tandem) que des produits de transcription desdits ADNs.

Il doit être compris que la présente description ne concerne pas les séquences nucléotidiques génomiques prises dans leur environnement naturel, c'est-à-dire à l'état naturel. Il s'agit de séquences qui ont pu être isolées, purifiées ou partiellement purifiées, à partir de méthodes de séparation telles que par exemple la chromatographie par échange d'ions, par exclusion basée sur la taille moléculaire, ou par affinité, ou encore les techniques de fractionnement basées sur la solubilité dans différents solvants, ou à partir de méthodes du génie génétique telles que l'amplification, le clonage et le sous-clonage, les séquences de la description pouvant être portées par des vecteurs.

Les séquences nucléotidiques SEQ ID N° 1 et SEQ ID N° 9 ont été obtenues par séquençage du génome par la méthode de Sanger.

Le terme « fragment de séquence nucléotidique » tel qu'utilisé dans la description désigne tout fragment nucléotidique du circovirus MAP, type A ou B, de longueur d'au moins 8 nucléotides, de préférence au moins 12 nucléotides, et encore plus préférentiellement au moins 20 nucléotides consécutifs de la séquence dont il est issu.

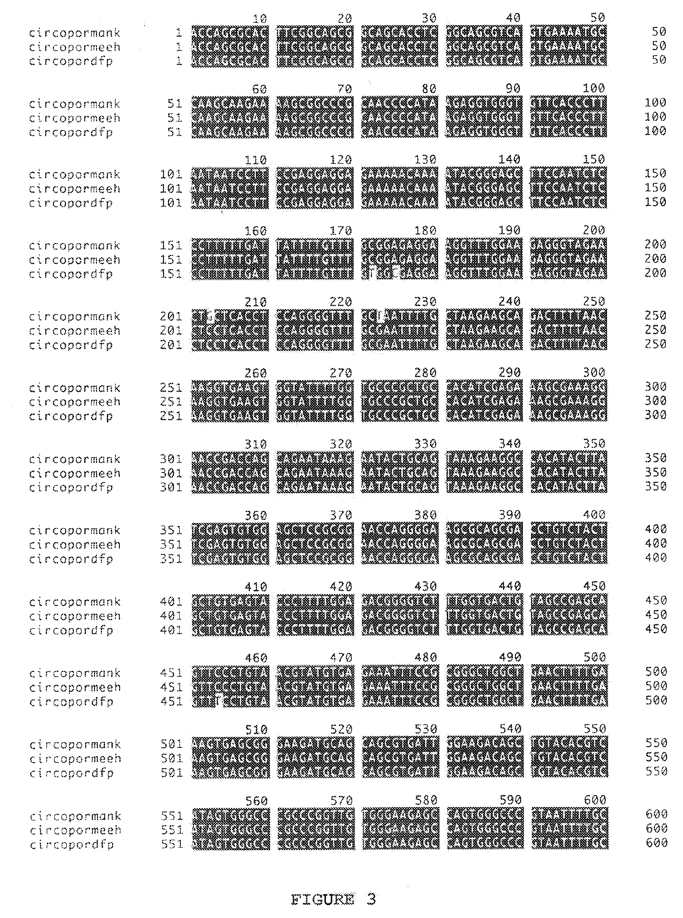

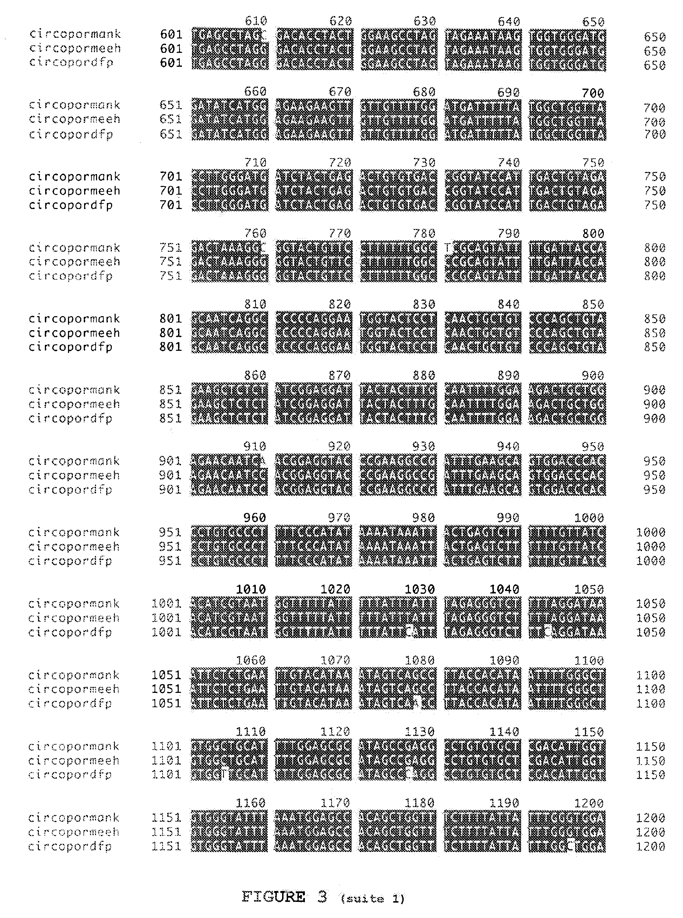

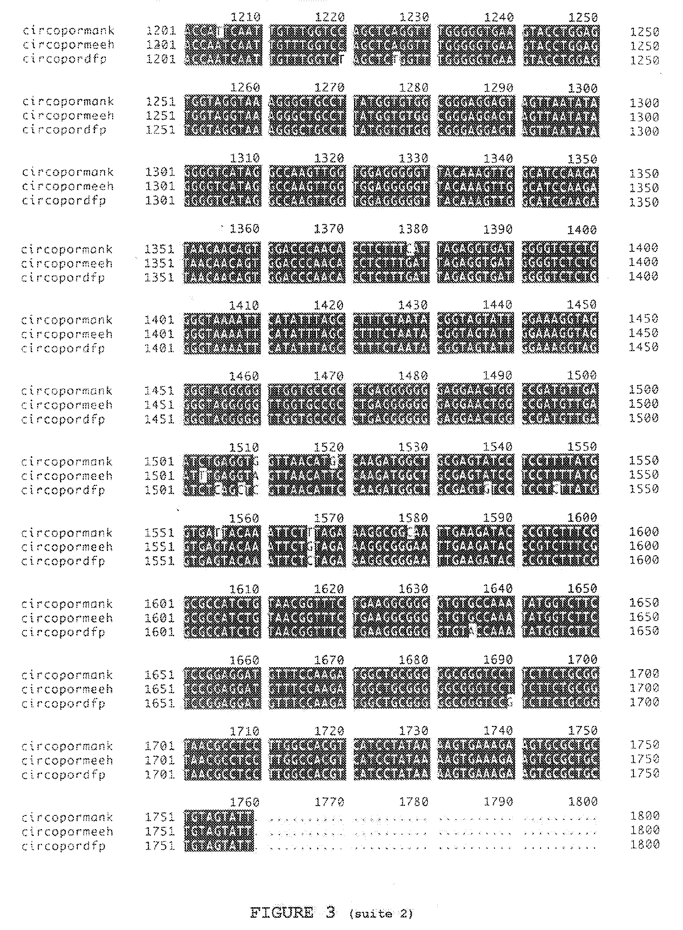

Par fragment spécifique de séquence nucléotidique selon la description, on entendra désigner tout fragment nucléotidique du circovirus MAP, type A ou B, présentant, après alignement et comparaison avec les fragments correspondants de circovirus porcins connus, au moins un nucléotide ou base de nature différente. Par exemple, les fragments nucléotidiques spécifiques du circovirus MAP de type A peuvent facilement être déterminés en se reportant à la figure 3 de la présente description dans laquelle sont mis en évidence les nucléotides ou bases de la séquence SEQ ID N° 1 (circopordfp) qui sont de nature différente, après alignement de ladite séquence SEQ ID N° 1 avec les deux autres séquences de circovirus porcin connues (circopormeeh et circopormank).

Par séquence nucléotidique homologue au sens de la présente description, on entend une séquence nucléotidique présentant au moins un pourcentage d'identité avec les bases d'une séquence nucléotidique selon la description d'au moins 80 %, de préférence 90 % et 95 %, ce pourcentage étant purement statistique et les différences entre les deux séquences nucléotidiques pouvant être réparties au hasard et sur toute leur longueur.

Par séquence nucléotidique homologue spécifique au sens de la présente description, on entend une séquence nucléotidique homologue présentant au moins une séquence nucléotidique de fragment spécifique, telle que définie précédemment. Lesdites séquences homologues « spécifiques » peuvent comprendre, par exemple, les séquences correspondant à la séquence génomique ou aux séquences de ses fragments représentatifs de variants de circovirus MAP de type A ou B. Ces séquences homologues spécifiques peuvent ainsi correspondre à des variations liées à des mutations au sein des souches de circovirus MAP de type A et B, et correspondre notamment à des troncatures, substitutions, délétions et/ou additions d'au moins un nucléotide. Lesdites séquences homologues peuvent également correspondre à des variations liées à la dégénérescence du code génétique.

Dans la présente description, on entendra désigner par circovirus MAP, les circovirus associés à la maladie de l'amaigrissement du porcelet (MAP) de type A (PCVA) ou de type B (PCVB), ci-après définis par leur séquence génomique, ainsi que les circovirus dont les séquences nucléiques sont homologues aux séquences des circovirus MAP de type A ou B, tels que notamment les circovirus correspondant à des variants du type A ou du type B.

Par séquence nucléotidique complémentaire d'une séquence de la description, on entend tout ADN dont les nucléotides sont complémentaires de ceux de la séquence de la description, et dont l'orientation est inversée (séquence antiparallèle).

On entend par hybridation dans des conditions de stringence avec une séquence nucléotidique selon la description, une hybridation dans des conditions de température et de force ionique choisies de telle manière qu'elles permettent le maintien de l'hybridation entre deux fragments d'ADN complémentaires.

A titre illustratif, des conditions de forte stringence de l'étape d'hybridation aux fins de définir les fragments nucléotidiques décrits ci-dessus, sont avantageusement les suivantes.

L'hybridation est réalisée à une température préférentielle de 65°C en présence de tampon SSC, 1 x SSC correspondant à 0,15 M NaCl et 0,05 M citrate de Na. Les étapes de lavage peuvent, par exemple, être les suivantes :
- 2 x SSC, à température ambiante suivi de 2 lavages à 2 x SSC, 0,5 % SDS à 65°C ; 2 x 0,5 x SSC, 0,5 % SDS ; à 65°C pendant 10 minutes chacun.

Les conditions de stringence intermédiaire, en utilisant par exemple une température de 42°C en présence d'un tampon 2 x SSC, ou de faible stringence, par exemple une température de 37°C en présence d'un tampon 2 x SSC, requièrent respectivement pour l'hybridation entre les deux séquences une complémentarité globale moins importante.

Les conditions stringentes d'hybridation décrites ci-avant pour un polynucléotide d'une taille d'environ 350 bases, seront adaptées par l'homme du métier pour des oligonucléotides de taille plus grande ou plus petite, selon l'enseignement de Sambrook et al., 1989.

Parmi les séquences nucléotidiques selon la description, on préfère également celles utilisables comme amorce ou sonde dans des méthodes permettant d'obtenir les séquences homologues selon la description, ces méthodes telles que la réaction en chaîne à la polymérase (PCR), le clonage et le séquençage d'acide nucléique étant bien connues de l'homme de l'art.

Parmi lesdites séquences nucléotidiques selon la description, on préfère encore celles utilisables comme amorce ou sonde dans des méthodes permettant de diagnostiquer la présence de circovirus MAP ou l'un de ses variants telles que définies ci-après.

On préfère également les séquences nucléotidiques selon la description capables de moduler, d'inhiber ou d'induire l'expression de gène de circovirus MAP, et/ou capables de moduler le cycle de réplication de circovirus MAP dans la cellule et/ou l'organisme hôte. On entendra désigner par cycle de réplication, l'invasion, la multiplication de circovirus MAP, et sa propagation cellules hôtes à cellules hôtes dans l'organisme hôte.

Parmi lesdites séquences nucléotidiques selon la description, on préfère enfin celles correspondant à des cadres ouverts de lecture, dénommés séquences ORF (ORF pour « open reading frame »), et codant pour des polypeptides, telles que par exemple les séquences SEQ ID N° 3 (ORF1), SEQ ID N° 4 (ORF2) et SEQ ID N° 5 (ORF3) correspondant respectivement aux séquences nucléotidiques comprises entre les positions 47 à 985 déterminées par rapport à la position des nucléotides sur la séquence SEQ ID N° 1, les positions 1723 à 1022 et les positions 658 à 38 par rapport à la position des nucléotides sur la séquence SEQ ID N° 2 (représentée suivant l'orientation 3' → 5'), les extrémités étant comprises, ou encore les séquences SEQ ID N° 11 (ORF'1), SEQ ID N° 12 (ORF'2) et SEQ ID N° 13 (ORF'3), correspondant respectivement aux séquences comprises entre les positions 51 à 995 déterminées par rapport à la position des nucléotides sur la séquence SEQ ID N° 9, les positions 1734 à 1033 et les positions 670 à 357, les positions étant déterminées par rapport à la position des nucléotides sur la séquence SEQ ID N° 10 (représentée suivant l'orientation 3' → 5'), les extrémités étant comprises.

Les fragments de séquence nucléotidique selon la description peuvent être obtenus par exemple par amplification spécifique, telle que la PCR, ou après digestion par des enzymes de restriction appropriées de séquences nucléotidiques selon la description, ces méthodes sont en particulier décrites dans l'ouvrage de Sambrook et al., 1989. Cesdits fragments représentatifs peuvent également être obtenus par synthèse chimique lorsque leur taille n'est pas trop importante et selon des méthodes bien connues de l'homme de l'art.

Par séquence nucléotidique modifiée, on entendra toute séquence nucléotidique obtenue par mutagenèse selon des techniques bien connues de l'homme de l'art, et comportant des modifications par rapport aux séquences normales selon la description, par exemple des mutations dans les séquences régulatrices et/ou promotrices de l'expression de polypeptide, notamment conduisant à une modification du taux d'expression dudit polypeptide ou à une modulation du cycle réplicatif.

Par séquence nucléotidique modifiée, on entendra également toute séquence nucléotidique codant pour un polypeptide modifié tel que défini ci-après.

La présente invention a pour objet des séquences nucléotidiques de circovirus MAP selon la description, caractérisées en ce qu'elles sont choisies parmi les séquences SEQ ID N° 3, SEQ ID N° 4, SEQ ID N° 5, SEQ ID N° 11, SEQ ID N° 12, SEQ ID N° 13 ou un de leurs fragments.

La description concerne également les séquences nucléotidiques caractérisées en ce qu'elles comprennent une séquence nucléotidique choisie parmi :
a) une séquence nucléotidique SEQ ID N° 3, SEQ ID N° 4, SEQ ID N° 5, SEQ ID N° 11, SEQ ID N° 12, SEQ ID N° 13 ou un de leurs fragments ;
b) une séquence nucléotidique de fragment spécifique d'une séquence telle que définie en a) ;
c) une séquence nucléotidique homologue comportant au moins 80 % d'identité avec une séquence telle que définie en a) ou b) ;
d) une séquence nucléotidique complémentaire ou d'ARN correspondant à une séquence telle que définie en a), b) ou c) ; et
e) une séquence nucléotidique modifiée d'une séquence telle que définie en a), b), c), ou d).

En ce qui concerne l'homologie avec les séquences nucléotidiques SEQ ID N° 3, SEQ ID N° 4, SEQ ID N° 5, SEQ ID N° 11, SEQ ID N° 12, SEQ ID N° 13 ou un de leurs fragments, on préfère les séquences homologues, notamment spécifiques, présentant un pourcentage d'identité avec l'une des séquences SEQ ID N° 3, SEQ ID N° 4, SEQ ID N° 5, SEQ ID N° 11, SEQ ID N° 12, SEQ ID N° 13 ou un de leurs fragments d'au moins 80 %, de préférence 90 % et 95 %. Lesdites séquences homologues spécifiques peuvent comprendre, par exemple, les séquences correspondant aux séquences ORF1, ORF2, ORF3, ORF'1, ORF'2 et ORF'3 de variant de circovirus MAP de type A ou de type B. Ces séquences homologues spécifiques peuvent de la même manière correspondre à des variations liées à des mutations au sein des souches de circovirus MAP de type A ou de type B et correspondre notamment à des troncatures, substitutions, délétions et/ou additions d'au moins un nucléotide.

Parmi les séquences nucléotidiques selon la description, on préfère notamment la séquence SEQ ID N° 11 qui présente une homologie comportant plus de 80 % d'identité avec la séquence SEQ ID N° 3, ainsi que la séquence SEQ ID N° 12.

De manière préférée, la description est relative aux séquences nucléotidiques selon la description, caractérisées en ce qu'elles comprennent une séquence nucléotidique choisie parmi les séquences suivantes :
a) 170 5'TGTGGCGA 3' ;
b) 450 5' AGTTTCCT 3' ;
c) 1026 5' TCATTTAGAGGGTCTTTCAG 3' ;
d) 1074 5' GTCAACCT 3' ;
e) 1101 5' GTGGTTGC 3' ;
f) 1123 5' AGCCCAGG 3' ;
g) 1192 5' TTGGCTGG 3' ;
h) 1218 5' TCTAGCTCTGGT 3' ;
i) 15015' ATCTCAGCTCGT 3' ;
j) 1536 5' TGTCCTCCTCTT 3' ;
k) 1563 5' TCTCTAGA 3' ;
l) 1632 5' TGTACCAA 3' ;
m) 1686 5' TCCGTCTT 3' ; et leur séquence complémentaire.

Dans la liste des séquences nucléotidiques a)-m) ci-dessus, les nucléotides soulignés sont mutés par rapport aux deux séquences connues de circovirus non pathogènes pour le porc. Le nombre précédent la séquence nucléotidique représente la position du premier nucléotide de ladite séquence sur la séquence SEQ ID N° 1.

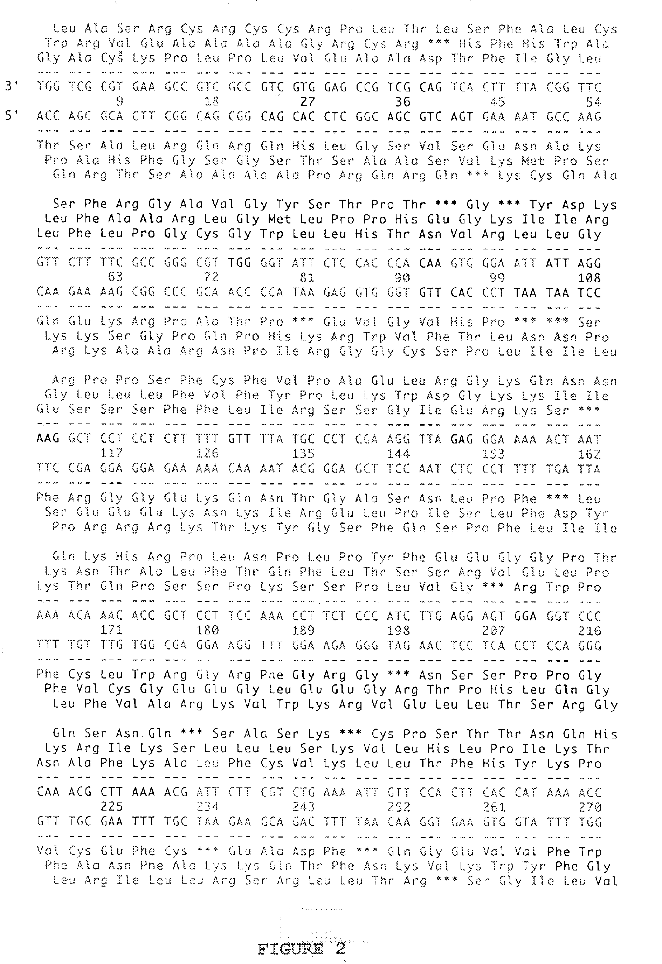

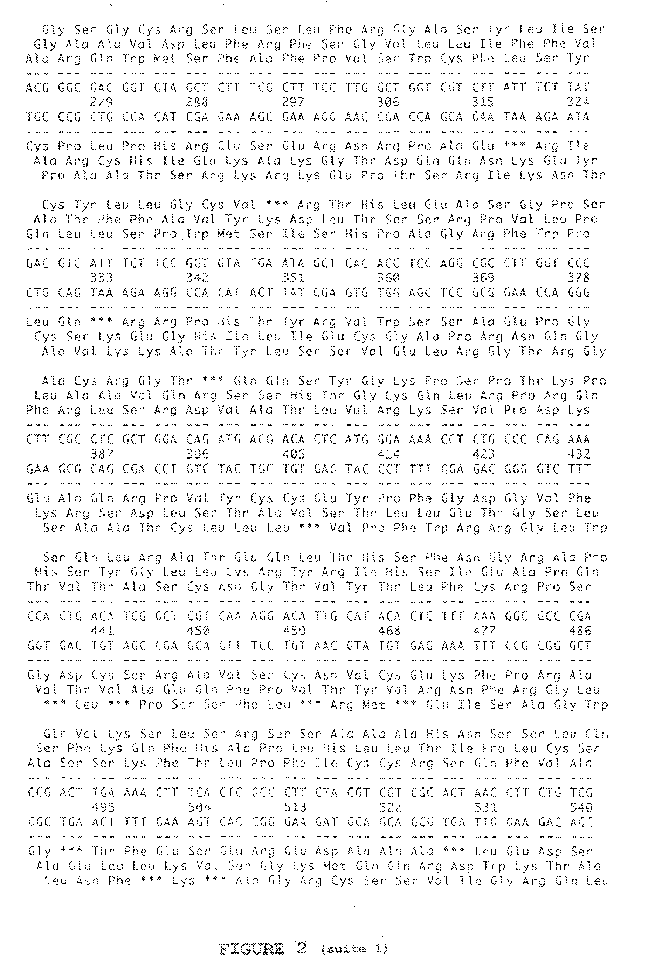

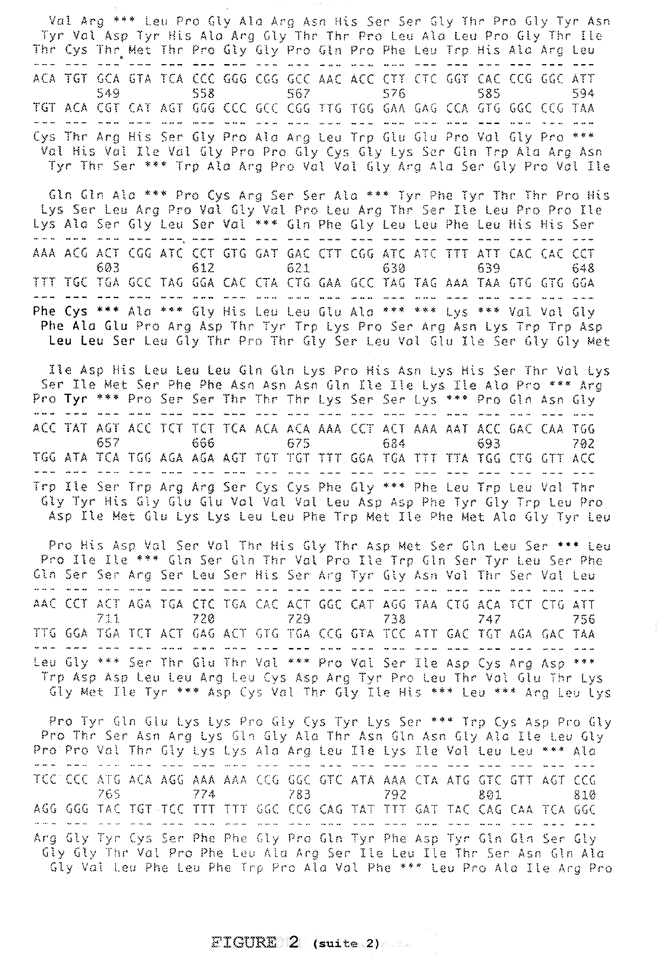

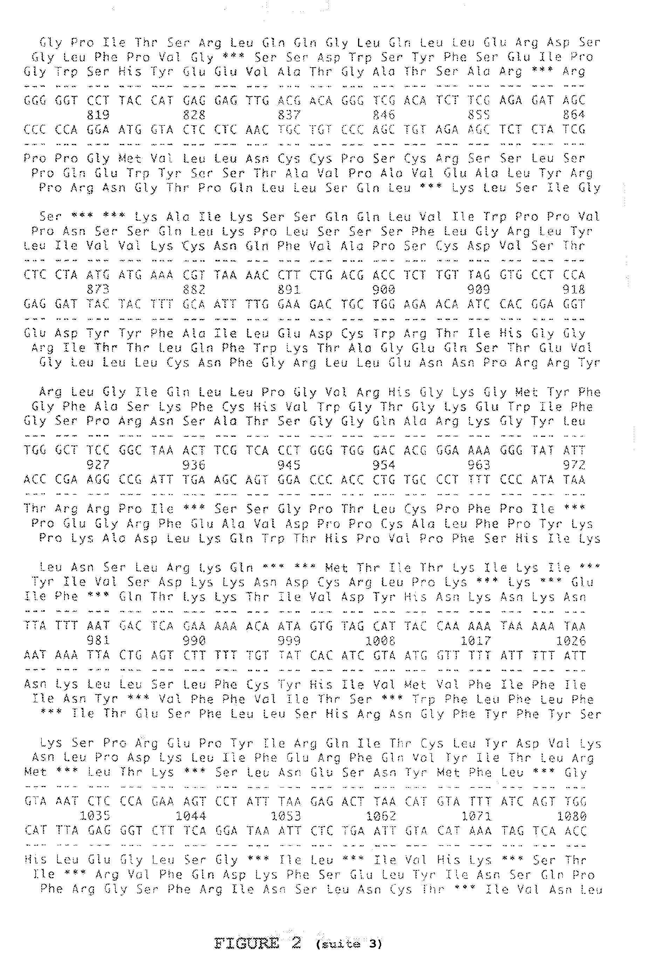

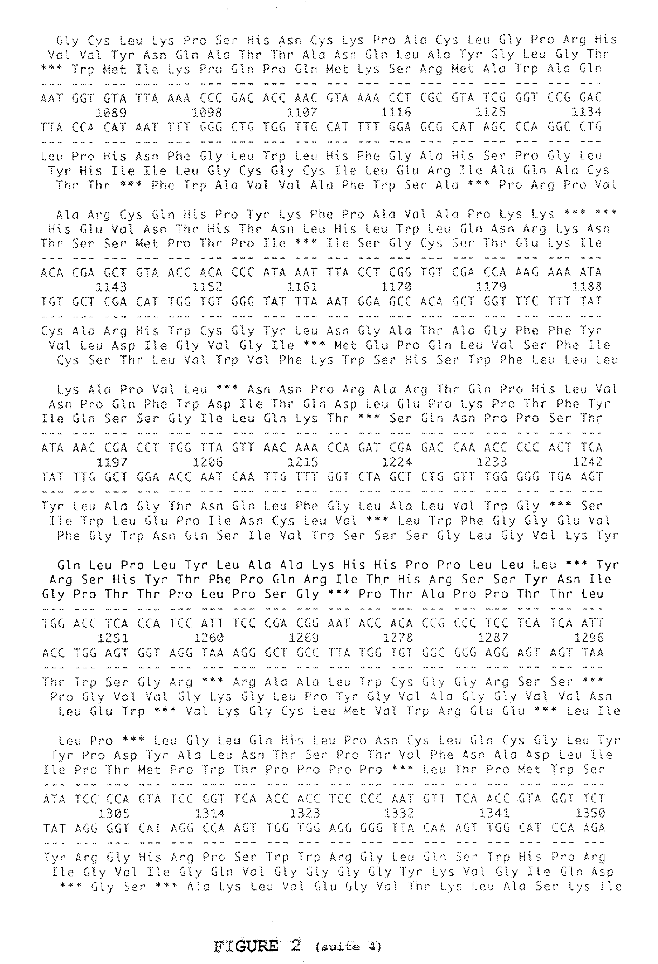

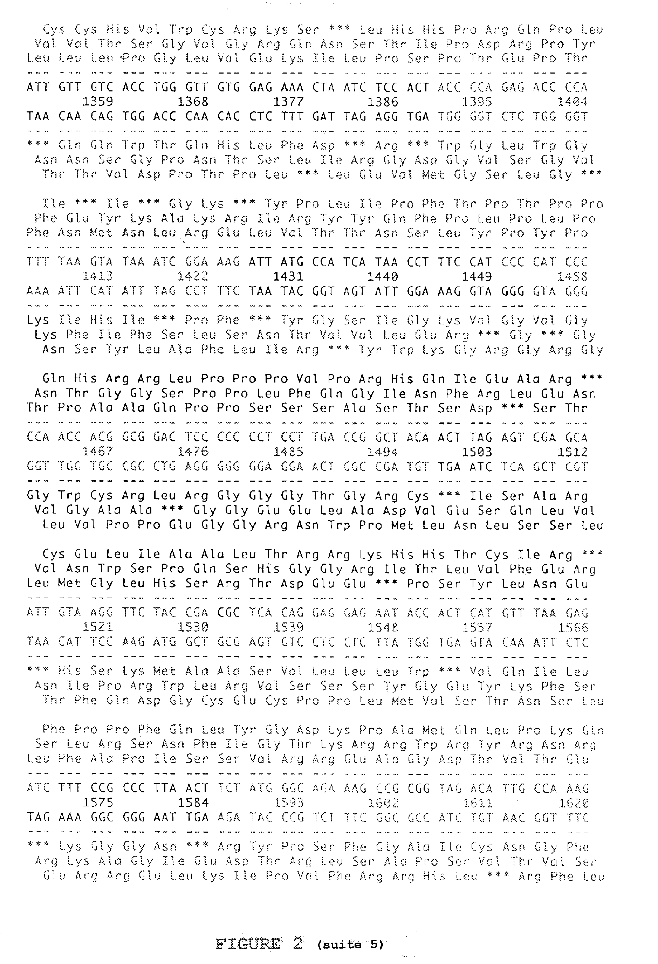

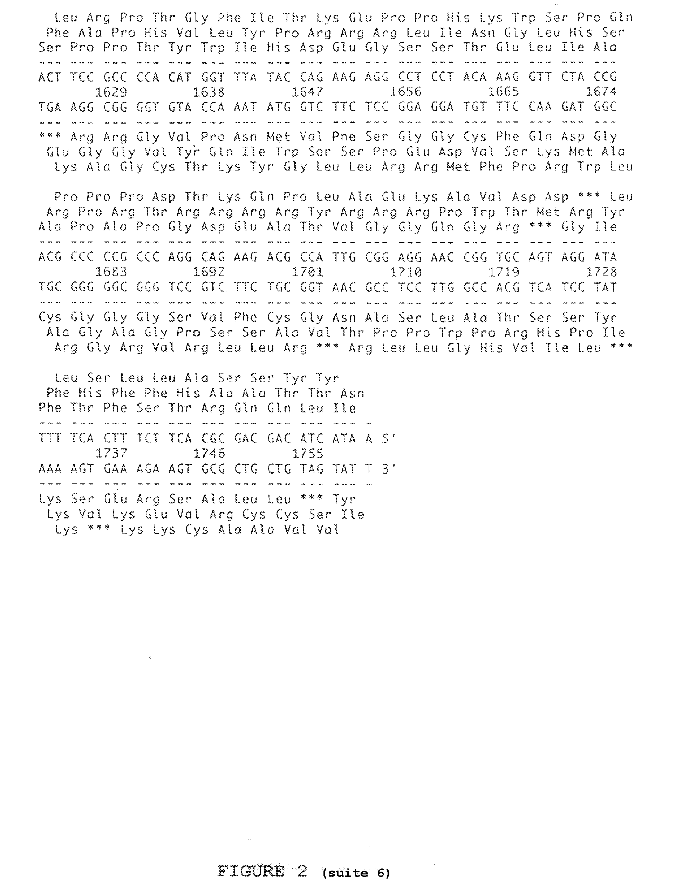

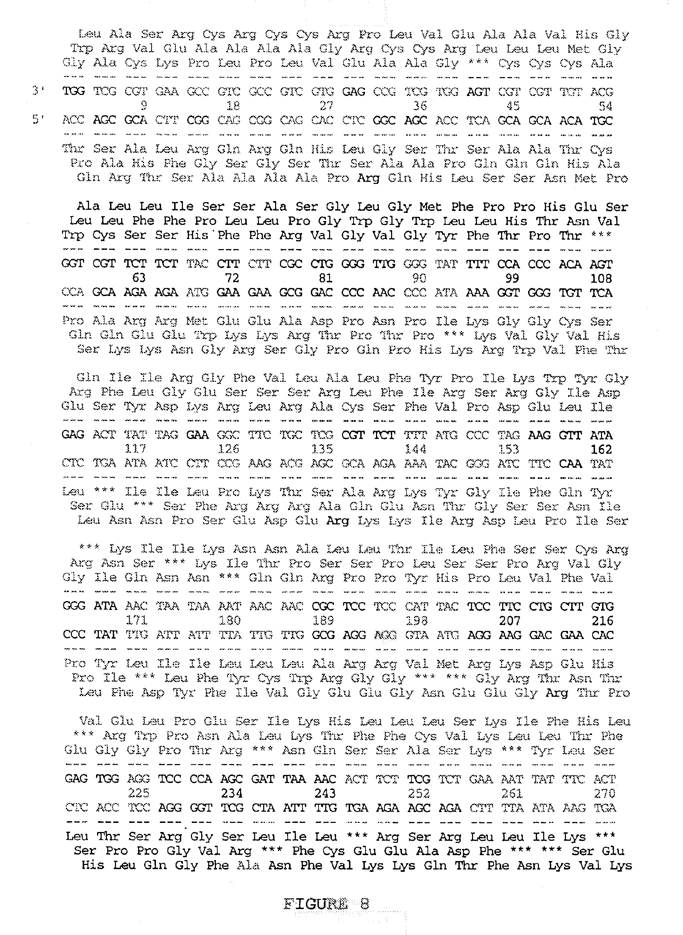

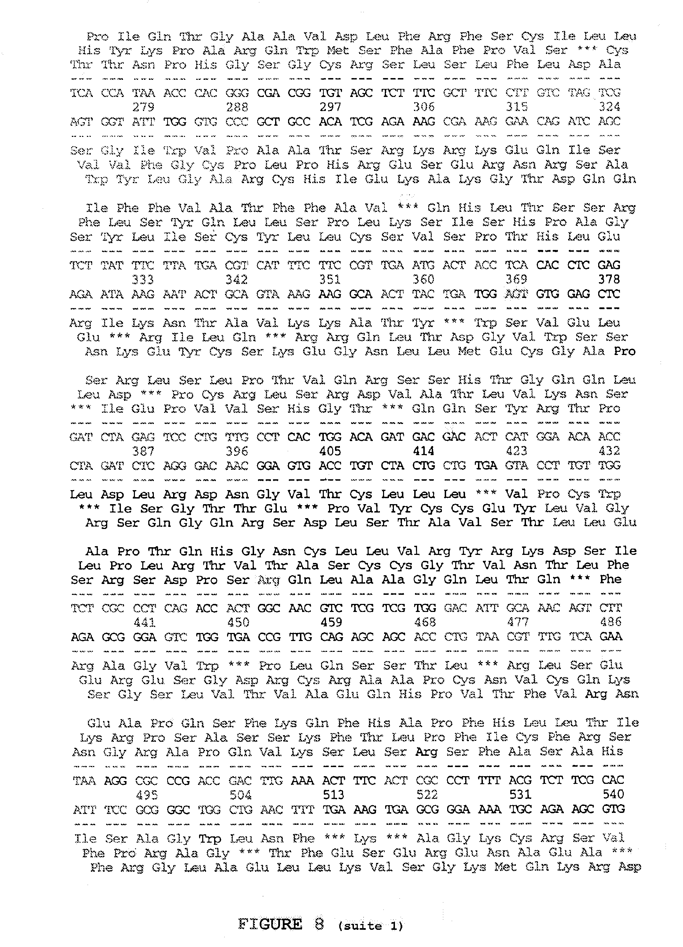

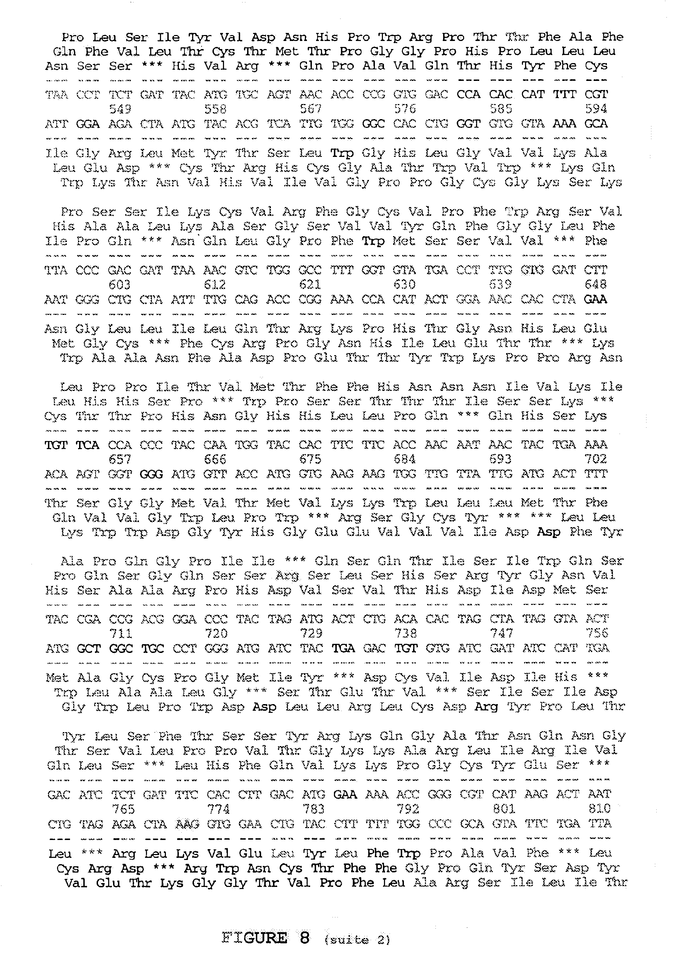

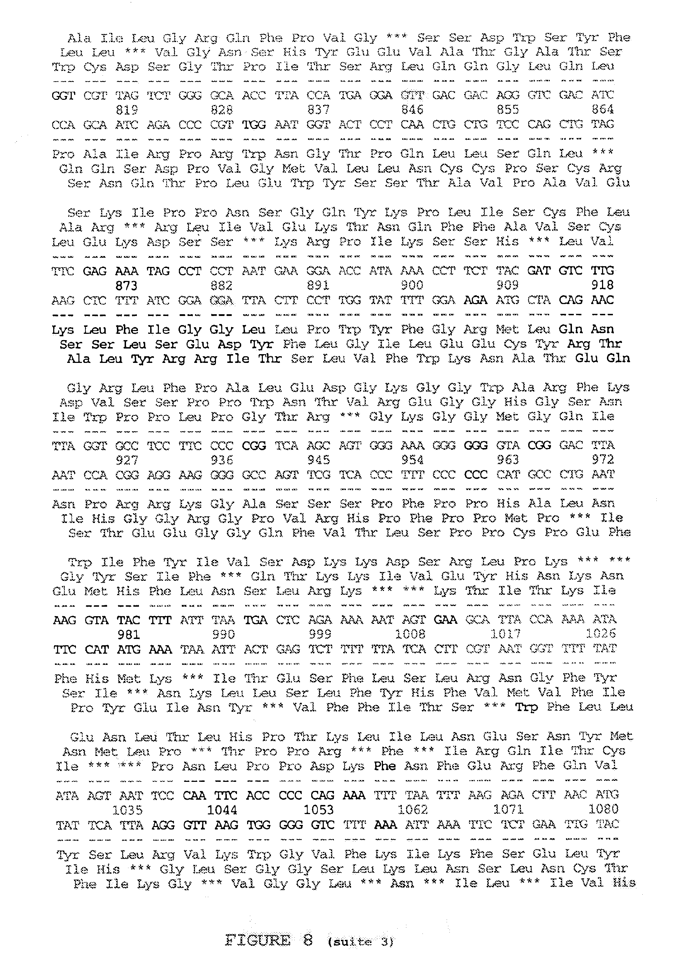

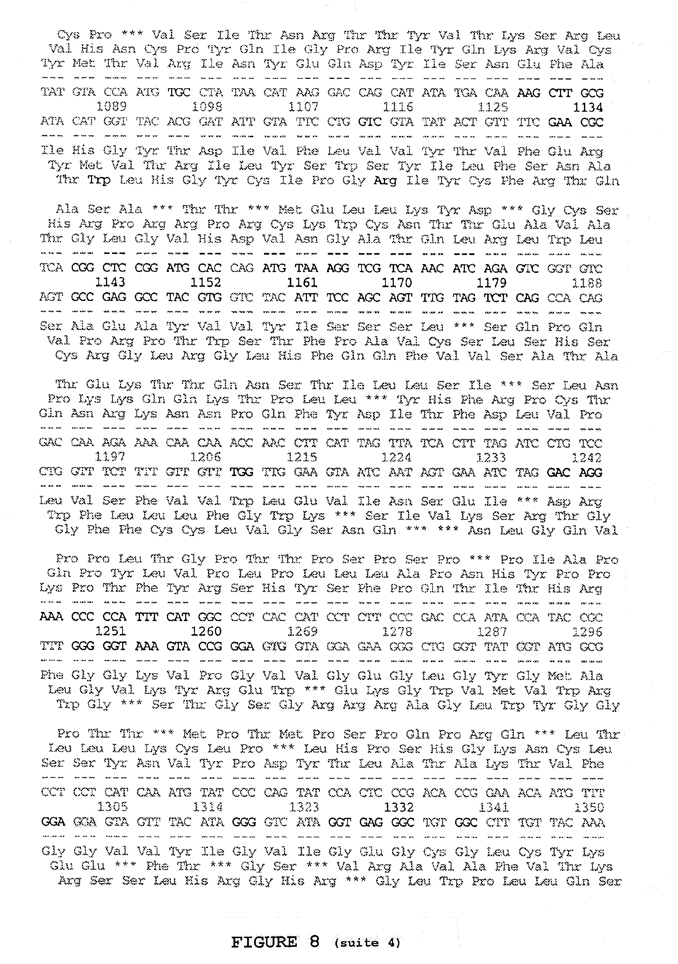

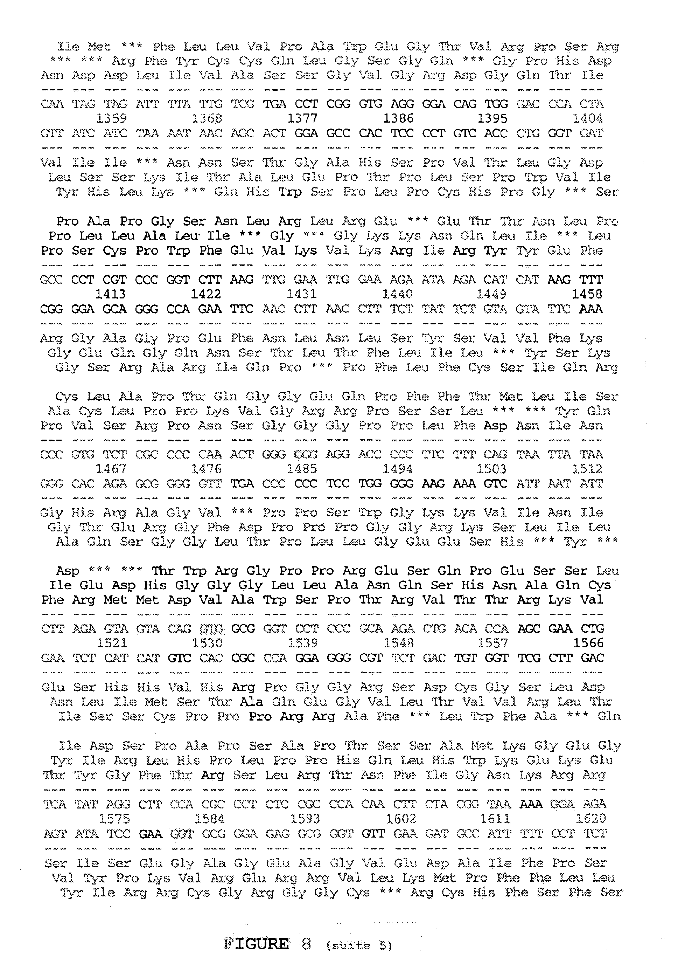

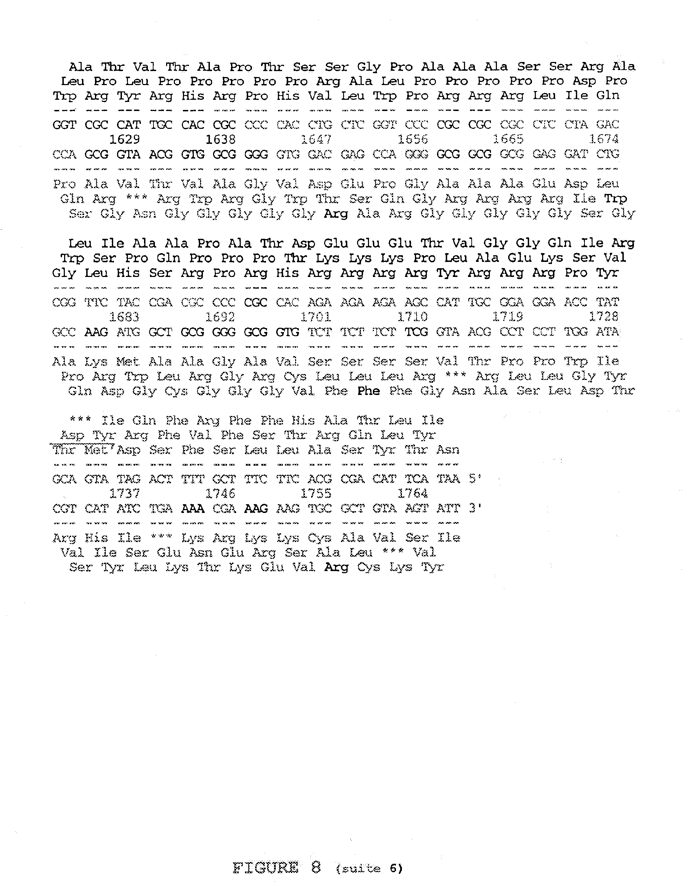

La description comprend les polypeptides codés par une séquence nucléotidique selon la description, de préférence un polypeptide dont la séquence est représentée par un fragment, notamment spécifique, d'une des 6 séquences d'acides aminés représentées à la figure 2, ces 6 séquences d'acides aminés correspondant aux polypeptides pouvant être codés suivant l'un des 3 cadres de lecture possibles de la séquence SEQ ID N° 1 ou de la séquence SEQ ID N° 2, ou un polypeptide dont la séquence est représentée par un fragment, notamment spécifique, d'une des 6 séquences d'acides aminés représentées à la figure 8, ces 6 séquences d'acides aminés correspondant aux polypeptides pouvant être codés suivant l'un des 3 cadres de lecture possible de la séquence SEQ ID N° 9 ou de la séquence SEQ ID N° 10.

La description concerne également les polypeptides caractérisés en ce qu'ils comprennent un polypeptide choisi parmi les séquences d'acides aminés SEQ ID N° 6, SEQ ID N° 7, SEQ ID N° 8, SEQ ID N° 14, SEQ ID N° 15, SEQ ID N° 16 ou un de leurs fragments.

Parmi les polypeptides selon la description, on préfère notamment le polypeptide de séquence d'acides aminés SEQ ID N° 14 qui présente une homologie comportant plus de 80 % d'identité avec la séquence SEQ ID N° 6, ainsi que le polypeptide de séquence SEQ ID N° 15.

La description concerne aussi les polypeptides caractérisés en ce qu'ils comprennent un polypeptide choisi parmi :
a) un fragment spécifique d'au moins 5 acides aminés d'un polypeptide de séquence d'acides aminés selon la description ;
b) un polypeptide homologue à un polypeptide tel que défini en a) ;
c) un fragment spécifique biologiquement actif d'un polypeptide tel que défini en a) ou b) ; et
d) un polypeptide modifié d'un polypeptide tel que défini en a), b) ou c).

Le terme « polypeptides » tel qu'utilisé dans la présente description désigne de préférence les polypeptides de séquences d'acides aminés SEQ ID N° 17, SEQ ID N° 18, SEQ ID N° 19 et SEQ ID N° 20, ces polypeptides étant notamment capables de reconnaître de manière spécifique les anticorps produits lors de l'infection par le circovirus MAP de type B. Ces polypeptides présentent ainsi des épitopes spécifiques du circovirus MAP de type B et peuvent donc en particulier être utilisés dans le domaine diagnostic ou comme agent immunogène pour conférer une protection chez le porc contre l'infection par circovirus MAP, notamment de type B.

Dans la présente description, les termes polypeptide, peptide et protéine sont interchangeables.

Il doit être compris que la description ne concerne pas les polypeptides sous forme naturelle, c'est-à-dire qu'ils ne sont pas pris dans leur environnement naturel mais qu'ils ont pu être isolés ou obtenus par purification à partir de sources naturelles, ou bien obtenus par recombinaison génétique, ou encore par synthèse chimique et qu'ils peuvent alors comporter des acides aminés non naturels, comme cela sera décrit ci-après.

Le terme « fragment de polypeptide »tel qu'utilisé dans la description désigne un polypeptide comportant au minimum 5 acides aminés, de préférence 10 acides aminés et 15 acides aminés.

Le terme « fragment spécifique de polypeptide » tel qu'utilisé dans la descriptiondésigne un fragment de polypeptide codé par une séquence nucléotidique de fragment spécifique selon la description.

Par polypeptide homologue, on entendra désigner les polypeptides présentant, par rapport au polypeptide naturel, certaines modifications comme en particulier une délétion, addition ou substitution d'au moins un acide aminé, une troncation, un allongement, une fusion chimérique, et/ou une mutation. Parmi les polypeptides homologues, on préfère ceux dont la séquence d'acides aminés présente au moins 80 %, de préférence 90 %, d'homologie avec les séquences d'acides aminés des polypeptides selon la description.

Par polypeptide homologue spécifique, on entendra désigner les polypeptides homologues tels que définis précédemment et présentant un fragment spécifique de polypeptide selon la description.

Dans le cas d'une substitution, un ou plusieurs acides aminés consécutifs ou non consécutifs, sont remplacés par des acides aminés « équivalents ». L'expression acide aminé « équivalent » vise ici à désigner tout acide aminé susceptible d'être substitué à l'un des acides aminés de la structure de base sans cependant modifier essentiellement les activités biologiques des peptides correspondants et telles qu'elles seront définies par la suite.

Ces acides aminés équivalents peuvent être déterminés soit en s'appuyant sur leur homologie de structure avec les acides aminés auxquels ils se substituent, soit sur des résultats d'essais comparatifs d'activité biologique entre les différents polypeptides susceptibles d'être effectués.

A titre d'exemple, on mentionnera les possibilités de substitutions susceptibles d'être effectuées sans qu'il en résulte une modification approfondie de l'activité biologique des polypeptides modifiés correspondants, les remplacements, par exemple, de la leucine par la valine ou l'isoleucine, de l'acide aspartique par l'acide glutamique, de la glutamine par l'asparagine, de l'arginine par la lysine etc., les substitutions inverses étant naturellement envisageables dans les mêmes conditions.

Les polypeptides homologues spécifiques correspondent également aux polypeptides codés par les séquences nucléotidiques homologues spécifiques telles que définies précédemment et comprennent ainsi dans la présente définition les polypeptides mutés ou correspondant à des variants, pouvant exister chez circovirus MAP, et qui correspondent notamment à des troncatures, substitutions, délétions et/ou additions d'au moins un résidu d'acide aminé.

Le terme « fragment spécifique biologiquement actif d'un polypeptide » tel qu'utilisé dans la description désigne en particulier un fragment spécifique de polypeptide, tel que défini ci-avant, présentant au moins une des caractéristiques des polypeptides selon la description, notamment en ce qu'il est :
- capable d'induire une réaction d'immunogénicité dirigée contre un circovirus MAP ; et/ou
- capable d'être reconnu par un anticorps selon l'invention, spécifique d'un polypeptide selon la description ; et/ou
- capable de se lier à un polypeptide ou à une séquence nucléotidique de circovirus MAP ; et/ou
- capable d'exercer une activité physiologique, même partielle, telle que par exemple une activité de dissémination ou structurelle (capside) ; et/ou
- capable de moduler, d'induire ou d'inhiber l'expression de gène de circovirus MAP ou l'un de ses variants, et/ou capable de moduler le cycle de réplication de circovirus MAP dans la cellule et/ou l'organisme hôte.

Les fragments de polypeptide selon la description peuvent correspondre à des fragments isolés ou purifiés naturellement présents dans un circovirus MAP ou correspondre à des fragments pouvant être obtenus par clivage dudit polypeptide par une enzyme protéolytique, telle que la trypsine ou la chymotrypsine ou la collagénase, ou par un réactif chimique, tel que le bromure de cyanogène (CNBr) ou encore en plaçant ledit polypeptide dans un environnement très acide, par exemple à pH 2,5. De tels fragments polypeptidiques peuvent être également préparés indifféremment par synthèse chimique, à partir d'hôtes transformés par un vecteur d'expression selon la description contenant un acide nucléique permettant l'expression desdits fragments, placé sous le contrôle des éléments de régulation et/ou d'expression appropriés.

Par « polypeptide modifié » d'un polypeptide selon la description, on entend désigner un polypeptide obtenu par recombinaison génétique ou par synthèse chimique comme cela sera décrit ci-après, présentant au moins une modification par rapport à la séquence normale. Ces modifications pourront notamment porter sur des acides aminés à l'origine d'une spécificité, de la pathogénicité et/ou de virulence, ou à l'origine de la conformation structurale, et de la capacité d'insertion membranaire du polypeptide selon la description. On pourra ainsi créer des polypeptides d'activité équivalente, augmentée ou diminuée, et de spécificité équivalente, plus étroite, ou plus large. Parmi les polypeptides modifiés, il faut citer les polypeptides dans lesquels jusqu'à 5 acides aminés peuvent être modifiés, tronqués à l'extrémité N- ou C-terminale, ou bien délétés, ou bien ajoutés.

Comme cela est indiqué, les modifications du polypeptide auront pour objectif notamment :
- de le rendre capable de moduler, d'inhiber ou d'induire l'expression de gène de circovirus MAP et/ou capable de moduler le cycle de réplication de circovirus MAP dans la cellule et/ou l'organisme hôte,
- de permettre son incorporation dans des compositions vaccinales,
- de modifier sa biodisponibilité en tant que composé à usage thérapeutique.

Les méthodes permettant de mettre en évidence lesdites modulations sur des cellules eucaryotes ou procaryotes sont bien connues de l'homme de l'art. Il est également bien entendu que les séquences nucléotidiques codant pour lesdits polypeptides modifiés pourront être utilisées pour lesdites modulations, par exemple par l'intermédiaire de vecteurs selon la description et décrits ci-après, afin, par exemple, de prévenir ou de traiter les pathologies liées à l'infection.

Les polypeptides modifiés précédents peuvent être obtenus en utilisant la chimie combinatoire, dans laquelle il est possible de faire varier systématiquement des parties de polypeptide avant de les tester sur des modèles, cultures cellulaires ou des micro-organismes par exemple, pour sélectionner les composés les plus actifs ou présentant les propriétés recherchées.

La synthèse chimique présente également l'avantage de pouvoir utiliser :
- des acides aminés non naturels, ou
- des liaisons non peptidiques.

Ainsi, afin d'améliorer la durée de vie des polypeptides selon la description, il pourra être intéressant d'utiliser des acides aminés non naturels, par exemple sous forme D, ou bien des analogues d'acides aminés, notamment des formes soufrées par exemple.

Enfin, la structure des polypeptides selon la description, ses formes homologues spécifiques ou modifiées, pourra être intégrée dans des structures chimiques de type polypeptidique ou autres. Ainsi, il pourra être intéressant de prévoir aux extrémités Net C-terminales des composés non reconnus par les protéases.

Font également partie de la description les séquences nucléotidiques codant pour un polypeptide selon la description.

La description concerne également des séquences nucléotidiques utilisables comme amorce ou sonde, caractérisées en ce que lesdites séquences sont choisies parmi les séquences nucléotidiques selon la description.

Parmi les couples de séquences nucléotidiques utilisables comme couple d'amorces selon la description, on préfère les couples d'amorces choisis parmi les couples suivants :
a) 5' GTG TGC TCG ACA TTG GTG TG 3', et
   5' TGG AAT GTT AAC GAG CTG AG 3' ;
b) 5' GTG TGC TCG ACA TTG GTG TG 3', et
   5' CTC GCA GCC ATC TTG GAA TG 3' ;
c) 5' CGC GCG TAA TAC GAC TCA CT 3', et
   5' GTG TGC TCG ACA TTG GTG TG 3' ;
d) 5' CGC GCG TAA TAC GAC TCA CT 3', et
   5' CTC GCA GCC ATC TTG GAA TG 3' ; et
e) 5' CCT GTC TAC TGC TGT GAG TAC CTT GT 3', et
   5' GCA GTA GAC AGG TCA CTC CGT TGT CC 3'.

Le clonage et le séquençage du circovirus MAP, type A et B, a permis d'identifier, après analyse comparative avec les séquences nucléotidiques des autres circovirus porcins, quelles étaient parmi les séquences de fragments de ces acides nucléiques, celles qui sont strictement spécifiques du circovirus MAP de type A, de type B ou de type A et B, et celles qui correspondent à une séquence consensus de circovirus porcins autres que les circovirus MAP de type A et/ou B.

Un grand besoin existe également de pouvoir disposer de séquences nucléotidiques utilisables comme amorce ou sonde spécifiques de l'ensemble de circovirus porcins autres connus et non pathogènes.

Cesdites séquences nucléotidiques consensus spécifiques de l'ensemble de circovirus, autres que circovirus MAP de type A et B, sont facilement identifiables à partir de la figure 3 et de la séquence SEQ ID N° 9, et font partie de la description.

Parmi cesdites séquences nucléotidiques consensus, on préfère celle caractérisée en ce qu'elle fait partie du couple d'amorces suivant :
a) 5' GTG TGC TCG ACA TTG GTG TG 3', et
   5' TGG AAT GTT AAC TAC CTC AA 3'.

La description comprend également une séquence nucléotidique selon la description, caractérisée en ce que ladite séquence est une séquence consensus spécifique de circovirus porcin autre que circovirus MAP de type B et en ce qu'elle est l'une des amorces du couple d'amorces suivant :
a) 5' GGC GGC GCC ATC TGT AAC GGT TT 3', et
   5' GAT GGC GCC GAA AGA CGG GTA TC 3'.

Il est bien entendu que la présente invention a également pour objet les polypeptides spécifiques de circovirus porcins connus autres que circovirus MAP, codés par lesdites séquences nucléotidiques consensus, susceptibles d'être obtenus par purification à partir des polypeptides naturels, par recombinaison génétique ou par synthèse chimique par des procédés bien connus de l'homme de l'art et tels que décrits notamment ci-après. De la même manière, les anticorps mono ou polyclonaux, marqués ou non marqués dirigés contre lesdits polypeptides spécifiques codés par lesdites séquences nucléotidiques consensus, font aussi partie de la description.

Lesdites séquences nucléotidiques consensus, lesdits polypeptides correspondants ainsi que lesdits anticorps dirigés contre lesdits polypeptides, pourront être utilisés dans des procédés ou nécessaires de détection et/ou d'identification tels que décrits ci-après, à la place ou en outre des séquences nucléotidiques, des polypeptides selon la description ou des anticorps selon l'invention, spécifiques de circovirus MAP type A et/ou B.

Ces protocoles ont été améliorés pour détecter de façon différentielle les formes monomériques circulaires de formes réplicatives spécifiques du virion ou de l'ADN en réplication et les formes dimériques retrouvées dans les constructions moléculaires dites en tandem.

La description concerne en outre l'utilisation d'une séquence nucléotidique selon la description, comme amorce ou sonde, pour la détection et/ou l'amplification de séquences d'acide nucléique.

Les séquences nucléotidiques selon la description peuvent ainsi être utilisées pour amplifier des séquences nucléotidiques, notamment par la technique PCR (réaction en chaîne à la polymérase) (Erlich, 1989 ; Innis et al., 1990 ; Rolfs et al., 1991 ; et White et al., 1997).

Ces amorces oligodésoxyribonucléotidiques ou oligo-ribonucléotidiques ont avantageusement une longueur d'au moins 8 nucléotides, de préférence d'au moins 12 nucléotides, et encore plus préférentiellement au moins 20 nucléotides.

D'autres techniques d'amplification de l'acide nucléique cible peuvent être avantageusement employées comme alternatives à la PCR.

Les séquences nucléotidiques de la description, en particulier les amorces selon la description, peuvent également être mises en oeuvre dans d'autres procédés d'amplification d'un acide nucléique cible, tels que :
- la technique TAS (Transcription-based Amplification System), décrite par Kwoh et al. en 1989 ;
- la technique 3SR (Self-Sustained Sequence Replication), décrite par Guatelli et al. en 1990 ;
- la technique NASBA (Nucleic Acid Sequence Based Amplification), décrite par Kievitis et al. en 1991 ;
- la technique SDA (Strand Displacement Amplification) ou technique d'amplification à déplacement de brin (Walker et al., 1992) ;
- la technique TMA (Transcription Mediated Amplification).

Les polynucléotides de la description peuvent aussi être employés dans des techniques d'amplification ou de modification de l'acide nucléique servant de sonde, telles que :
- la technique LCR (Ligase Chain Reaction), décrite par Landegren et al. en 1988 et perfectionnée par Barany et al. en 1991, qui emploie une ligase thermostable ;
- la technique de RCR (Repair Chain Reaction), décrite par Segev en 1992 ;
- la technique CPR (Cycling Probe Reaction), décrite par Duck et al. en 1990 ;
- la technique d'amplification à la Q-beta-réplicase, décrite par Miele et al. en 1983 et perfectionnée notamment par Chu et al. en 1986, Lizardi et al. en 1988, puis par Burg et al., ainsi que par Stone et al. en 1996.

Dans le cas où éventuellement le polynucléotide cible à détecter est un ARN, par exemple un ARNm, on pourra utiliser, préalablement à la mise en oeuvre d'une réaction d'amplification à l'aide d'au moins une amorce selon la description ou à la mise en oeuvre d'un procédé de détection à l'aide d'au moins une sonde de la description, une enzyme de type transcriptase inverse afin d'obtenir un ADNc à partir de l'ARN contenu dans l'échantillon biologique. L'ADNc obtenu servira alors de cible pour la ou les amorces ou la ou les sondes mises en oeuvre dans le procédé d'amplification ou de détection selon la description.

La sonde de détection sera choisie de telle manière à ce qu'elle hybride avec la séquence cible ou l'amplicon généré à partir de la séquence cible. Une telle sonde de détection aura avantageusement pour séquence une séquence d'au moins 12 nucléotides, en particulier d'au moins 20 nucléotides, et de préférence d'au moins 100 nucléotides.

La description comprend aussi les séquences nucléo-tidiques utilisables comme sonde ou amorce selon la description, caractérisées en ce qu'elles sont marquées par un composé radioactif ou par un composé non radioactif.

Les séquences nucléotidiques non marquées peuvent être utilisées directement comme sondes ou amorces, cependant les séquences sont généralement marquées par un élément radioactif (³²P, ³⁵S, ³H, ¹²⁵I) ou par une molécule non radioactive (biotine, acétylaminofluorène, digoxigénine, 5-bromo-désoxyuridine, fluorescéine) pour obtenir des sondes utilisables pour de nombreuses applications.

Des exemples de marquage non radioactifs de séquences nucléotidiques sont décrits, par exemple, dans le brevet français N° 78.10975 ou par Urdea et al. ou par Sanchez-Pescador et al. en 1988.

Dans ce dernier cas, on pourra aussi utiliser l'une des méthodes de marquage décrites dans les brevets FR-2 422 956 et FR-2 518 755.

La technique d'hybridation peut être réalisée de manières diverses (Matthews et al., 1988). La méthode la plus générale consiste à immobiliser l'acide nucléique extrait des cellules sur un support (tel que nitro-cellulose, nylon, polystyrène) et à incuber, dans des conditions bien définies, l'acide nucléique cible immobilisé avec la sonde. Après l'hybridation, l'excès de sonde est éliminé et les molécules hybrides formées sont détectées par la méthode appropriée (mesure de la radioactivité, de la fluorescence ou de l'activité enzymatique liée à la sonde).

La description comprend également les séquences nucléotidiques selon la description, caractérisées en ce qu'elles sont immobilisées sur un support, de manière covalente ou non covalente.

Selon un autre mode avantageux de mise en oeuvre des séquences nucléotidiques selon la description, ces dernières peuvent être utilisées immobilisées sur un support et servir ainsi à capturer par hybridation spécifique l'acide nucléique cible obtenu à partir de l'échantillon biologique à tester. Si nécessaire, le support solide est séparé de l'échantillon et le complexe d'hybridation formé entre la sonde dite de capture et l'acide nucléique cible est ensuite détecté grâce à une seconde sonde, dite sonde de détection, marquée par un élément facilement détectable.

Un autre objet de la présente invention est un vecteur pour le clonage et/ou l'expression d'une séquence, caractérisé en ce qu'il contient une séquence nucléotidique selon la description.

Les vecteurs selon la description, caractérisés en ce qu'ils comportent les éléments permettant l'expression et/ou la sécrétion desdites séquences nucléotidiques dans une cellule hôte déterminée, font également partie de la description.

Le vecteur doit alors comporter un promoteur, des signaux d'initiation et de terminaison de la traduction, ainsi que des régions appropriées de régulation de la transcription. Il doit pouvoir être maintenu de façon stable dans la cellule hôte et peut éventuellement posséder des signaux particuliers spécifiant la sécrétion de la protéine traduite. Ces différents éléments sont choisis en fonction de l'hôte cellulaire utilisé. A cet effet, les séquences nucléotidiques selon la description peuvent être insérées dans des vecteurs à réplication autonome au sein de l'hôte choisi, ou des vecteurs intégratifs de l'hôte choisi.

De tels vecteurs seront préparés selon les méthodes couramment utilisées par l'homme du métier, et les clones en résultant pourront être introduits dans un hôte approprié par des méthodes standard, telles que par exemple la lipofection, l'électroporation, le choc thermique.

Les vecteurs selon la description sont par exemple des vecteurs d'origine plasmidique ou virale.

Un vecteur préféré pour l'expression des polypeptides de la description est le baculovirus.

On préfère également le vecteur pBS KS dans lequel est inséré la séquence d'ADN en tandem du circovirus MAP type A (ou DFP) et tel que déposé à la CNCM le 3 juillet 1997, sous le numéro I-1891.

Ces vecteurs sont utiles pour transformer des cellules hôtes afin de cloner ou d'exprimer les séquences nucléo-tidiques de la description.

La description comprend également les cellules hôtes transformées par un vecteur selon la description.

Ces cellules peuvent être obtenues par l'introduction dans des cellules hôtes d'une séquence nucléotidique insérée dans un vecteur tel que défini ci-dessus, puis la mise en culture desdites cellules dans des conditions permettant la réplication et/ou l'expression de la séquence nucléotidique transfectée.

L'hôte cellulaire peut être choisi parmi des systèmes procaryotes ou eucaryotes, comme par exemple les cellules bactériennes (Olins et Lee, 1993), mais également les cellules de levure (Buckholz, 1993), de même que les cellules animales, en particulier les cultures de cellules de mammifères (Edwards et Aruffo, 1993), et notamment les cellules d'ovaire de hamster chinois (CHO), mais également les cellules d'insectes dans lesquelles on peut utiliser des procédés mettant en oeuvre des baculovirus par exemple (Luckow, 1993).

Une cellule hôte préférée pour l'expression des protéines de la description est constituée par les cellules d'insectes sf9.

Une cellule hôte encore préférée selon la description est E-coli, telle que déposée à la CNCM le 3 juillet 1997, sous le numéro 1-1891.

La description concerne également les animaux, comprenant une desdites cellules transformées selon la description.

L'obtention d'animaux transgéniques selon la description surexprimant un ou plusieurs des gènes de circovirus MAP ou partie de gènes sera menée de façon préférée sur des rats, des souris ou des lapins selon des méthodes bien connues de l'homme de l'art telles que par transfections, virales ou non virales. Les animaux transgéniques surexprimant l'un ou plusieurs desdits gènes pourront être obtenus par transfection de copies multiples desdits gènes sous contrôle d'un promoteur puissant de nature ubiquitaire, ou sélectif d'un type de tissu. Les animaux transgéniques pourront être également obtenus par recombinaison homologue sur cellules souches embryonnaires, transfert de ces cellules souches à des embryons, sélection des chimères affectées au niveau des lignées reproductrices, et croissance desdites chimères.

Les cellules transformées ainsi que les animaux transgéniques selon la description sont utilisables dans des procédés de préparation de polypeptide recombinant.

Il est aujourd'hui possible de produire des polypeptides recombinants en quantité relativement importante par génie génétique en utilisant les cellules transformées par des vecteurs d'expression selon la description ou en utilisant des animaux transgéniques selon la description.

Les procédés de préparation d'un polypeptide de la description sous forme recombinante, caractérisés en ce qu'ils mettent en oeuvre un vecteur et/ou une cellule transformée par un vecteur selon la description et/ou un animal transgénique comprenant une desdites cellules transformées selon la description, sont eux-mêmes compris dans la présente invention.

Parmi lesdits procédés de préparation d'un polypeptide de la description sous forme recombinante, on préfère les procédés de préparation mettant en oeuvre un vecteur, et/ou une cellule transformée par ledit vecteur et/ou un animal transgénique comprenant une desdites cellules transformées, contenant une séquence nucléotidique selon la description codant pour un polypeptide de circovirus MAP.

Les polypeptides recombinants obtenus comme indiqué ci-dessus, peuvent aussi bien se présenter sous forme glycosylée que non glycosylée et peuvent présenter ou non la structure tertiaire naturelle.

Une variante préférée consiste à produire un polypeptide recombinant fusionné à une protéine « porteuse » (protéine chimère). L'avantage de ce système est qu'il permet une stabilisation et une diminution de la protéolyse du produit recombinant, une augmentation de la solubilité au cours de la renaturation in vitro et/ou une simplification de la purification lorsque le partenaire de fusion possède une affinité pour un ligand spécifique.

Plus particulièrement, la description concerne un procédé de préparation d'un polypeptide de la description comprenant les étapes suivantes :
a) culture des cellules transformées dans des conditions permettant l'expression d'un polypeptide recombinant de séquence nucléotidique selon la description ;
b) le cas échéant, récupération dudit polypeptide recombinant.

Lorsque le procédé de préparation d'un polypeptide de la description met en oeuvre un animal transgénique selon la description, le polypeptide recombinant est ensuite extrait dudit animal.

La description a aussi pour objet un polypeptide susceptible d'être obtenu par un procédé de la description tel que décrit précédemment.

La description comprend aussi un procédé de préparation d'un polypeptide synthétique, caractérisé en ce qu'il utilise une séquence d'acides aminés de polypeptides selon la description.

La description concerne également un polypeptide synthétique obtenu par un procédé selon la description.

Les polypeptides selon la description peuvent également être préparés par les techniques classiques, dans le domaine de la synthèse des peptides. Cette synthèse peut être réalisée en solution homogène ou en phase solide.

Par exemple, on aura recours à la technique de synthèse en solution homogène décrite par Houbenweyl en 1974.

Cette méthode de synthèse consiste à condenser successivement deux à deux les acides aminés successifs dans l'ordre requis, ou à condenser des acides aminés et des fragments préalablement formés et contenant déjà plusieurs acides aminés dans l'ordre approprié, ou encore plusieurs fragments préalablement ainsi préparés, étant entendu que l'on aura eu soin de protéger au préalable toutes les fonctions réactives portées par ces acides aminés ou fragments, à l'exception des fonctions amines de l'un et carboxyles de l'autre ou vice-versa, qui doivent normalement intervenir dans la formation des liaisons peptidiques, notamment après activation de la fonction carboxyle, selon les méthodes bien connues dans la synthèse des peptides.

Selon une autre technique préférée de la description, on a recours à celle décrite par Merrifield.

Pour fabriquer une chaîne peptidique selon le procédé de Merrifield, on a recours à une résine polymère très poreuse, sur laquelle on fixe le premier acide aminé C-terminal de la chaîne. Cet acide aminé est fixé sur une résine par l'intermédiaire de son groupe carboxylique et sa fonction amine est protégée. On fixe ainsi, les uns après les autres, les acides aminés qui vont constituer la chaîne peptidique sur le groupe amine chaque fois déprotégé au préalable de la portion de la chaîne peptidique déjà formée, et qui est rattachée à la résine. Lorsque la totalité de la chaîne peptidique désirée est formée, on élimine les groupes protecteurs des différents acides aminés constituant la chaîne peptidique et on détache le peptide de la résine à l'aide d'un acide.

La description est en outre relative à des polypeptides hybrides présentant au moins un polypeptide selon la description, et une séquence d'un polypeptide susceptible d'induire une réponse immunitaire chez l'homme ou l'animal.

Avantageusement, le déterminant antigénique est tel qu'il est susceptible d'induire une réponse humorale et/ou cellulaire.

Un tel déterminant pourra comprendre un polypeptide selon la description sous forme glycosylée utilisé en vue d'obtenir des compositions immunogènes susceptibles d'induire la synthèse d'anticorps dirigés contre des épitopes multiples. Lesdits polypeptides ou leurs fragments glycosylés font également partie de la description.

Ces molécules hybrides peuvent être constituées en partie d'une molécule porteuse de polypeptides ou de leurs fragments selon la description, associée à une partie éventuellement immunogène, en particulier un épitope de la toxine diphtérique, la toxine tétanique, un antigène de surface du virus de l'hépatite B (brevet FR 79 21811), l'antigène VP1 du virus de la poliomyélite ou toute autre toxine ou antigène viral ou bactérien.

Les procédés de synthèse des molécules hybrides englobent les méthodes utilisées en génie génétique pour construire des séquences nucléotidiques hybrides codant pour les séquences polypeptidiques recherchées. On pourra, par exemple, se référer avantageusement à la technique d'obtention de gènes codant pour des protéines de fusion décrite par Minton en 1984.

Lesdites séquences nucléotidiques hybrides codant pour un polypeptide hybride ainsi que les polypeptides hybrides selon la description caractérisés en ce qu'il s'agit de polypeptides recombinants obtenus par l'expression desdites séquences nucléotidiques hybrides, font également partie de la description.

La description comprend également les vecteurs caractérisés en ce qu'ils contiennent une desdites séquences nucléotidiques hybrides. Les cellules hôtes transformées par lesdits vecteurs, les animaux transgéniques comprenant une desdites cellules transformées ainsi que les procédés de préparation de polypeptides recombinants utilisant lesdits vecteurs, lesdites cellules transformées et/ou lesdits animaux transgéniques font bien entendu également partie de la description.

Les polypeptides selon la description, les anticorps selon l'invention ci-après décrits et les séquences nucléotidiques selon la description peuvent avantageusement être mis en oeuvre dans des procédés pour la détection et/ou l'identification de circovirus MAP, ou de circovirus porcin autre qu'un circovirus MAP, dans un échantillon biologique (tissu ou fluide biologique) susceptible de les contenir. Ces procédés, suivant la spécificité des anticorps selon l'invention qui seront utilisés, pourront en particulier détecter et/ou identifier un circovirus MAP ou un circovirus porcin autre qu'un circovirus MAP ou autre que le circovirus MAP de type B.

Les polypeptides selon la description peuvent avantageusement être mis en oeuvre dans un procédé pour la détection et/ou l'identification de circovirus MAP de type A, de type B, de type A ou B, de circovirus porcin autre que le circovirus MAP de type B, ou de circovirus porcin autre que le circovirus MAP de type A ou B, dans un échantillon biologique (tissu ou fluide biologique) susceptible de les contenir, caractérisé en ce qu'il comprend les étapes suivantes :
a) mise en contact de cet échantillon biologique avec un polypeptide ou un de ses fragments selon la description (dans des conditions permettant une réaction immunologique entre ledit polypeptide et les anticorps éventuellement présents dans l'échantillon biologique) ;
b) mise en évidence des complexes antigène-anticorps éventuellement formés.

Dans la présente description, on entendra désigner par circovirus MAP, sauf si une mention particulière est indiquée, un circovirus MAP de type A ou de type B, et par circovirus porcin autre que MAP, sauf si une mention particulière est indiquée, un circovirus porcin autre qu'un circovirus MAP de type A et B.

De préférence, l'échantillon biologique est constitué par un fluide, par exemple un sérum de porc, du sang total ou des biopsies.

Toute procédure classique peut être mise en oeuvre pour réaliser une telle détection des complexes antigène-anticorps éventuellement formés.

A titre d'exemple, une méthode préférée met en jeu des processus immunoenzymatiques selon la technique ELISA, par immunofluorescence, ou radio-immunologiques (RIA) ou équivalent.

Ainsi, la description concerne également les polypeptides selon la description, marqués à l'aide d'un marqueur adéquat tel que du type enzymatique, fluorescent, radioactif.

De telles méthodes comprennent par exemple les étapes suivantes :
- dépôt de quantités déterminées d'une composition polypeptidique selon la description dans les puits d'une plaque de microtitration,
- introduction dans lesdits puits de dilutions croissantes de sérum, ou d'échantillon biologique autre tel que défini précédemment, devant être analysé,
- incubation de la microplaque,
- introduction dans les puits de la plaque de micro-titration d'anticorps marqués dirigés contre des immunoglobulines de porc, le marquage de ces anticorps ayant été réalisé à l'aide d'une enzyme sélectionnée parmi celles qui sont capables d'hydrolyser un substrat en modifiant l'absorption des radiations de ce dernier, au moins à une longueur d'onde déterminée, par exemple à 550 nm,
- détection, en comparaison avec un témoin de contrôle, de la quantité de substrat hydrolysé.

La description concerne également un kit ou nécessaire pour la détection et/ou l'identification de circovirus MAP, de circovirus porcin autre qu'un circovirus MAP ou de circovirus porcin autre que le circovirus MAP de type B, caractérisé en ce qu'il comprend les éléments suivants :
- un polypeptide selon la description,
- le cas échéant, les réactifs pour la constitution du milieu propice à la réaction immunologique ou spécifique,
- le cas échéant, les réactifs permettant la détection des complexes antigène-anticorps produits par la réaction immunologique entre le ou les polypeptides de la description et les anticorps éventuellement présents dans l'échantillon biologique, ces réactifs pouvant également porter un marqueur, ou être susceptibles d'être reconnus à leur tour par un réactif marqué, plus particulièrement dans le cas où le polypeptide selon la description n'est pas marqué,
- le cas échéant, un échantillon biologique de référence (témoin négatif) dépourvu d'anticorps reconnus par un polypeptide selon la description,
- le cas échéant, un échantillon biologique de référence (témoin positif) contenant une quantité prédéterminée d'anticorps reconnus par un polypeptide selon la description.

Les polypeptides selon la description permettent de préparer des anticorps monoclonaux ou polyclonaux caractérisés en ce qu'ils reconnaissent spécifiquement les polypeptides selon la description. Les anticorps monoclonaux pourront avantageusement être préparés à partir d'hybridomes selon la technique décrite par Kohler et Milstein en 1975. Les anticorps polyclonaux pourront être préparés, par exemple, par immunisation d'un animal, en particulier une souris, avec un polypeptide ou un ADN selon la description associé à un adjuvant de la réponse immunitaire, puis purification des anticorps spécifiques contenus dans le sérum des animaux immunisés sur une colonne d'affinité sur laquelle a préalablement été fixé le polypeptide ayant servi d'antigène. Les anticorps polyclonaux selon l'invention peuvent aussi être préparés par purification, sur une colonne d'affinité, sur laquelle a préalablement été immobilisé un polypeptide selon la description, des anticorps contenus dans le sérum de porcs infectés par un circovirus MAP.

L'invention a également pour objet des anticorps mono ou polyclonaux ou leurs fragments, ou anticorps chimériques, caractérisés en ce qu'ils sont capables de reconnaître spécifiquement un polypeptide isolé ayant une séquence choisie parmi les séquences SEQ ID N°17, SEQ ID N°18, SEQ ID N°19 et SEQ ID N°20.

L'invention a également pour objet des anticorps monoclonaux ou leurs fragments, ou anticorps chimériques, capables de reconnaître spécifiquement un polypeptide isolé ayant une séquence choisie parmi les séquences SEQ ID N°17, SEQ ID N°18, SEQ ID N°19 et SEQ ID N°20.

Les anticorps de l'invention pourront également être marqués de la même manière que décrit précédemment pour les sondes nucléiques de la description tels qu'un marquage de type enzymatique, fluorescent ou radioactif.

L'invention vise en outre un procédé pour la détection et/ou l'identification de circovirus MAP, de circovirus porcin autre qu'un circovirus MAP, ou autre que le circovirus MAP de type B, dans un échantillon biologique, caractérisé en ce qu'il comprend les étapes suivantes :
a) mise en contact de l'échantillon biologique (tissu ou fluide biologique) avec un anticorps mono ou polyclonal selon l'invention (dans des conditions permettant une réaction immunologique entre lesdits anticorps et les polypeptides de circovirus MAP, de circovirus porcin autre qu'un circovirus MAP, de circovirus porcin autre que le circovirus MAP de type B, éventuellement présents dans l'échantillon biologique) ;
b) mise en évidence du complexe antigène-anticorps éventuellement formé.

Entre également dans le cadre de l'invention, un kit ou nécessaire pour la détection et/ou l'identification de circovirus MAP, de circovirus porcin autre qu'un circovirus MAP ou de circovirus porcin autre que le circovirus MAP de type B, caractérisé en ce qu'il comprend les éléments suivants :
- un anticorps polyclonal ou monoclonal selon l'invention, le cas échéant marqué ;
- un réactif pour la constitution d'un milieu propice à la réalisation de la réaction immunologique ;
- le cas échéant, un réactif permettant la détection des complexes antigène-anticorps produits par la réaction immunologique, ce réactif pouvant également porter un marqueur, ou être susceptible d'être reconnu à son tour par un réactif marqué, plus particulièrement dans le cas où ledit anticorps monoclonal ou polyclonal n'est pas marqué ;
- le cas échéant, des réactifs pour effectuer la lyse des cellules de l'échantillon testé.

La présente description a également pour objet un procédé pour la détection et/ou l'identification de MAP, de circovirus porcin autre qu'un circovirus MAP ou de circovirus porcin autre que le circovirus MAP de type B, dans un échantillon biologique, caractérisé en ce qu'il met en oeuvre une séquence nucléotidique selon la description.

Plus particulièrement, la description concerne un procédé pour la détection et/ou l'identification de circovirus MAP, de circovirus porcin autre qu'un circovirus MAP ou de circovirus porcin autre que le circovirus MAP de type B, dans un échantillon biologique, caractérisé en ce qu'il comporte les étapes suivantes :
a) le cas échéant, isolement de l'ADN à partir de l'échantillon biologique à analyser ;
b) amplification spécifique de l'ADN de l'échantillon à l'aide d'au moins une amorce, ou un couple d'amorces, selon la description ;
c) mise en évidence des produits d'amplification.

Ceux-ci peuvent être détectés par exemple par la technique d'hybridation moléculaire en utilisant une sonde nucléique selon la description. Cette sonde sera avantageuse-ment marquée par un élément non radioactif (sonde froide) ou radioactif.

Aux fins de la présente invention, on entendra par « ADN de l'échantillon biologique » ou « ADN contenu dans l'échantillon biologique », soit l'ADN présent dans l'échantillon biologique considéré, soit éventuellement l'ADNc obtenu après l'action d'une enzyme de type transcriptase inverse sur l'ARN présent dans ledit échantillon biologique.

Un autre but de la présente invention consiste en un procédé selon la description, caractérisé en ce qu'il comprend les étapes suivantes :
a) mise en contact d'une sonde nucléotidique selon la description avec un échantillon biologique, l'ADN contenu dans l'échantillon biologique ayant, le cas échéant, préalablement été rendu accessible à l'hybridation, dans des conditions permettant l'hybridation de la sonde à l'ADN de l'échantillon ;
b) mise en évidence de l'hybride formé entre la sonde nucléotidique et l'ADN de l'échantillon biologique.

La présente invention concerne aussi un procédé selon la description, caractérisé en ce qu'il comprend les étapes suivantes :
a) mise en contact d'une sonde nucléotidique immobilisée sur un support selon la description avec un échantillon biologique, l'ADN de l'échantillon ayant, le cas échéant, été préalablement rendu accessible à l'hybridation, dans des conditions permettant l'hybridation de la sonde à l'ADN de l'échantillon ;
b) mise en contact de l'hybride formé entre la sonde nucléotidique immobilisée sur un support et l'ADN contenu dans l'échantillon biologique, le cas échéant après élimination de l'ADN de l'échantillon biologique n'ayant pas hybridé avec la sonde, avec une sonde nucléotidique marquée selon la description ;
c) mise en évidence du nouvel hybride formé à l'étape b).

Selon un mode de réalisation avantageux du procédé pour la détection et/ou l'identification défini précédemment, celui-ci est caractérisé en ce que, pré-alablement à l'étape a), l'ADN de l'échantillon biologique est préalablement amplifié à l'aide d'au moins une amorce selon la description.

La description vise en outre un kit ou nécessaire pour la détection et/ou l'identification de circovirus MAP, de circovirus porcin autre que le circovirus MAP ou de circovirus porcin autre que le circovirus MAP de type B, caractérisé en ce qu'il comprend les éléments suivants :
a) une sonde nucléotidique selon la description ;
b) le cas échéant, les réactifs nécessaires à la mise en oeuvre d'une réaction d'hybridation ;
c) le cas échéant, au moins une amorce selon la description ainsi que les réactifs nécessaires à une réaction d'amplification de l'ADN.

La description concerne également un kit ou nécessaire pour la détection et/ou l'identification de circovirus MAP, de circovirus porcin autre qu'un circovirus MAP ou de circovirus porcin autre que le circovirus MAP de type B, caractérisé en ce qu'il comprend les éléments suivants :
a) une sonde nucléotidique, dite sonde de capture, selon la description ;
b) une sonde oligonucléotidique, dite sonde de révélation, selon la description ;
c) le cas échéant, au moins une amorce selon la description ainsi que les réactifs nécessaires à une réaction d'amplification de l'ADN.

La description est relative aussi à un kit ou nécessaire pour la détection et/ou l'identification de circovirus MAP, de circovirus porcin autre qu'un circovirus MAP ou de circovirus porcin autre que le circovirus MAP de type B, caractérisé en ce qu'il comprend les éléments suivants :
a) au moins une amorce selon la description ;
b) le cas échéant, les réactifs nécessaires pour effectuer une réaction d'amplification d'ADN ;
c) le cas échéant, un composant permettant de vérifier la séquence du fragment amplifié, plus particulièrement une sonde oligonucléotidique selon la description.

Un anticorps selon l'invention peut être utilisé pour la sélection de composé organique ou inorganique capable de moduler, d'induire ou d'inhiber l'expression de gènes, et/ou de modifier la réplication cellulaire de circovirus MAP ou capable d'induire ou d'inhiber les pathologies liées à une infection par un circovirus MAP.

La description comprend également une méthode de sélection de composés capables de se lier à un polypeptide ou un de ses fragments selon la description, capables de se lier à une séquence nucléotidique selon la description, ou capables de reconnaître un anticorps selon l'invention, et/ou capables de moduler, d'induire ou d'inhiber l'expression de gènes, et/ou de modifier la réplication cellulaire de circovirus MAP ou capables d'induire ou d'inhiber les pathologies liées à une infection par un circovirus MAP, caractérisée en ce qu'elle comprend les étapes suivantes :
a) mise en contact dudit composé avec ledit polypeptide, ladite séquence nucléotidique, avec une cellule transformée selon la description et/ou administration dudit composé à un animal transformé selon la description ;
b) détermination de la capacité dudit composé à se lier avec ledit polypeptide ou ladite séquence nucléotidique, ou de moduler, d'induire ou d'inhiber l'expression de gènes, ou de moduler la croissance ou la réplication de circovirus MAP, ou d'induire ou d'inhiber chez ledit animal transformé les pathologies liées à une infection par circovirus MAP (dénommée activité dudit composé).

Les composés susceptibles d'être sélectionnés peuvent être des composés organiques tels que des polypeptides ou hydrates de carbone ou tous autres composés organiques ou inorganiques déjà connus, ou des composés organiques nouveaux élaborés à partir de techniques de modélisation moléculaire et obtenus par synthèse chimique ou bio-chimique, ces techniques étant connues de l'homme de l'art.

Lesdits composés sélectionnés pourront être utilisés pour moduler la réplication cellulaire de circovirus MAP et ainsi pour contrôler l'infection par ce virus. Les méthodes permettant de déterminer lesdites modulations étant bien connues de l'homme de l'art.

Cette modulation peut être réalisée par exemple par un agent capable de se lier à une protéine et ainsi d'inhiber ou de potentialiser son activité biologique, ou capable de se lier à une protéine d'enveloppe de la surface externe dudit virus et de bloquer la pénétration dudit virus dans la cellule hôte ou de favoriser l'action du système immunitaire de l'organisme infecté dirigé à l'encontre dudit virus. Cette modulation peut être également réalisée par un agent capable de se lier à une séquence nucléotidique d'un ADN dudit virus et de bloquer par exemple l'expression d'un polypeptide dont l'activité biologique ou structurelle est nécessaire à la réplication ou à la prolifération dudit virus cellules hôtes à cellules hôtes dans l'animal hôte.

La description concerne les composés susceptibles d'être sélectionnés par une méthode de sélection selon la description.

La description concerne également une composition pharmaceutique comprenant un composé choisi parmi les composés suivants :
a) une séquence nucléotidique selon la description ;
b) un polypeptide selon la description ;
c) un vecteur, une particule virale ou une cellule transformée selon la description ;
d) un anticorps selon l'invention ;
e) un composé susceptible d'être sélectionné par une méthode de sélection selon la description ;
éventuellement en association avec un véhicule pharmaceutiquement acceptable et, le cas échéant, avec un ou plusieurs adjuvants de l'immunité appropriés.

La description concerne aussi une composition immunogène et/ou vaccinale, caractérisée en ce qu'elle comprend un composé choisi parmi les composés suivants :
a) une séquence nucléotidique selon la description ;
b) un polypeptide selon la description ;
c) un vecteur ou une particule virale selon la description ; et
d) une cellule selon la description.

La description concerne en outre une composition vaccinale selon la description, caractérisée en ce qu'elle comprend un mélange d'au moins deux desdits composés a), b), c) et d) précédents et en ce que l'un des deux desdits composés est relatif au circovirus MAP de type A et l'autre relatif au circovirus MAP de type B.

Par composé relatif au circovirus MAP de type A ou de type B, on entend désigner ici respectivement un composé obtenu à partir de la séquence génomique du circovirus MAP de type A ou de type B.

La description vise en outre une composition immunogène et/ou vaccinale, caractérisée en ce qu'elle comprend au moins l'un des composés suivants :
- une séquence nucléotidique SEQ ID N° 11, SEQ ID N° 12, ou un de leurs fragments ;
- un polypeptide de séquence SEQ ID N° 14, SEQ ID N° 15, ou un de leurs fragments ;
- un vecteur ou une particule virale comprenant une séquence nucléotidique SEQ ID N° 11, SEQ ID N° 12, ou un de leurs fragments ;
- une cellule transformée capable d'exprimer un polypeptide de séquences SEQ ID N° 14, SEQ ID N° 15, ou un de leurs fragments ; ou
- un mélange de deux au moins desdits composés.

La description comprend aussi une composition immunogène et/ou vaccinale selon la description, caractérisée en ce qu'elle comprend ledit mélange de deux au moins desdits composés comme produit de combinaison pour une utilisation simultanée, séparée ou étalée dans le temps pour la prévention ou le traitement d'infection par un circovirus MAP, notamment de type B.

Dans un mode de réalisation préférée, la composition vaccinale selon la description comprend le mélange de composés suivants :
- un plasmide pcDNA3 contenant un acide nucléique de séquence SEQ ID N°11;
- un plasmide pcDNA3 contenant un acide nucléique de séquence SEQ ID N° 12 ;
- un plasmide pcDNA3 contenant un acide nucléique codant pour la protéine GM-CSF ;
- un baculovirus recombinant contenant un acide nucléique de séquence SEQ ID N° 11 ;
- un baculovirus recombinant contenant un acide nucléique de séquence SEQ ID N° 12 ; et
- le cas échéant, un adjuvant de l'immunité approprié, notamment l'adjuvant AIF™.

La description vise également une composition pharmaceutique selon la description, pour la prévention ou le traitement d'une infection par un circovirus MAP.

La description vise aussi une composition pharmaceutique selon la description, pour la prévention ou le traitement d'une infection par le circovirus MAP de type B.

La description concerne également l'utilisation d'une composition selon la description, pour la préparation d'un médicament destinée à la prévention ou le traitement d'infection par un circovirus MAP, de préférence par le circovirus MAP de type B.

Sous un autre aspect, la description a pour objet un vecteur, une particule virale ou une cellule selon la description, pour le traitement et/ou la prévention d'une maladie par thérapie génique.

Enfin, la description comprend l'utilisation d'un vecteur, d'une particule virale ou d'une cellule selon la description, pour la préparation d'un médicament destinée au traitement et/ou la prévention d'une maladie par thérapie génique.

Les polypeptides de la description entrant dans les compositions immunogènes ou vaccinales selon la description peuvent être sélectionnés par des techniques connues de l'homme de l'art comme par exemple sur la capacité desdits polypeptides à stimuler les cellules T, qui se traduit par exemple par leur prolifération ou la sécrétion d'interleukines, et qui aboutit à la production d'anticorps dirigés contre lesdits polypeptides.

Chez le porc, comme chez la souris, chez lesquelles une dose pondérale de la composition vaccinale comparable à la dose utilisée chez l'homme est administrée, la réaction anticorps est testée par prélèvement du sérum suivi d'une étude de la formation d'un complexe entre les anticorps présents dans le sérum et l'antigène de la composition vaccinale, selon les techniques usuelles.

Les compositions pharmaceutiques selon la description contiendront une quantité efficace des composés de la description, c'est-à-dire en quantité suffisante du ou desdits composés permettant d'obtenir l'effet désiré, comme par exemple la modulation de la réplication cellulaire de circovirus MAP. L'homme de l'art saura déterminer cette quantité, en fonction par exemple de l'âge et du poids de l'individu à traiter, de l'état d'avancement de la pathologie, des effets secondaires éventuels et au moyen de test d'évaluation des effets obtenus sur un échantillonnage de population, ces tests étant connus dans ces domaines d'applications.

Selon la description, lesdites compositions vaccinales seront de préférence en association avec un véhicule pharmaceutiquement acceptable et, le cas échéant, avec un ou plusieurs adjuvants de l'immunité appropriés.

Aujourd'hui, divers types de vaccins sont disponibles pour protéger l'animal ou l'homme contre des maladies infectieuses : micro-organismes vivants atténués (*M*. *bovis -* BCG pour la tuberculose), micro-organismes inactivés (virus de la grippe), des extraits acellulaires (*Bordetella pertussis* pour la coqueluche), protéines recombinées (antigène de surface du virus de l'hépatite B), des polyosides (pneumocoques). Des vaccins préparés à partir de peptides de synthèse ou de micro-organismes génétiquement modifiés exprimant des antigènes hétérologues sont en cours d'expérimentation. Plus récemment encore, des ADNs plasmidiques recombinés portant des gènes codant pour des antigènes protecteurs ont été proposés comme stratégie vaccinale alternative. Ce type de vaccination est réalisé avec un plasmide particulier dérivant d'un plasmide de *E. coli* qui ne se réplique pas *in vivo* et qui code uniquement pour la protéine vaccinante. Des animaux ont été immunisés en injectant simplement l'ADN plasmidique nu dans le muscle. Cette technique conduit à l'expression de la protéine vaccinale *in situ* et à une réponse immunitaire de type cellulaire (CTL) et de type humoral (anticorps). Cette double induction de la réponse immunitaire est l'un des principaux avantages de la technique de vaccination avec de l'ADN nu.

Les compositions vaccinales comprenant des séquences nucléotidiques ou des vecteurs dans lesquels sont insérées lesdites séquences, sont notamment décrites dans la demande internationale N° WO 90/11092 et également dans la demande internationale N° WO 95/11307.

La séquence nucléotidique constitutive de la composition vaccinale selon la description peut être injectée à l'hôte après avoir été couplée à des composés qui favorisent la pénétration de ce polynucléotide à l'intérieur de la cellule ou son transport jusqu'au noyau cellulaire. Les conjugués résultants peuvent être encapsulés dans des microparticules polymères, comme décrit dans la demande internationale N° WO 94/27238 (Medisorb Technologies International).

Selon un autre mode de réalisation de la composition vaccinale selon la description, la séquence nucléotidique, de préférence un ADN, est complexée avec du DEAE-dextran (Pagano et al., 1967) ou avec des protéines nucléaires (Kaneda et al., 1989), avec des lipides (Felgner et al., 1987) ou encapsulée dans des liposomes (Fraley et al., 1980) ou encore introduite sous la forme d'un gel facilitant sa transfection dans les cellules (Midoux et al., 1993, Pastore et al.,1994). Le polynucléotide ou le vecteur selon la description peut aussi être en suspension dans une solution tampon ou être associé à des liposomes.

Avantageusement, un tel vaccin sera préparé conformément à la technique décrite par Tacson et al. ou Huygen et al. en 1996 ou encore conformément à la technique décrite par Davis et al. dans la demande inter-nationale N° WO 95/11307.

Un tel vaccin peut être également préparé sous la forme d'une composition contenant un vecteur selon la description, placée sous le contrôle d'éléments de régulation permettant son expression chez l'homme ou l'animal. On pourra par exemple utiliser, en tant que vecteur d'expression *in vivo* de l'antigène polypeptidique d'intérêt, le plasmide pcDNA3 ou le plasmide pcDNA1/neo, tous les deux commercialisés par Invitrogen (R & D Systems, Abingdon, Royaume-Uni). On peut aussi utiliser le plasmide V1Jns.tPA, décrit par Shiver et al. en 1995. Un tel vaccin comprendra avantageusement, outre le vecteur recombinant, une solution saline, par exemple une solution de chlorure de sodium.

On entend désigner par véhicule pharmaceutiquement acceptable, un composé ou une combinaison de composés entrant dans une composition pharmaceutique ou vaccinale ne provoquant pas de réactions secondaires et qui permet par exemple la facilitation de l'administration du composé actif, l'augmentation de sa durée de vie et/ou de son efficacité dans l'organisme, l'augmentation de sa solubilité en solution ou encore l'amélioration de sa conservation. Ces véhicules pharmaceutiquement acceptables sont bien connus et seront adaptés par l'homme de l'art en fonction de la nature et du mode d'administration du composé actif choisi.

En ce qui concerne les formulations vaccinales, celles-ci peuvent comprendre des adjuvants de l'immunité appropriés qui sont connus de l'homme de l'art, comme par exemple l'hydroxyde d'aluminium, un représentant de la famille des muramyl peptides comme un des dérivés peptidiques du N-acétyl-muramyl, un lysat bactérien, ou encore l'adjuvant incomplet de Freund.

Ces composés peuvent être administrés par voie systémique, en particulier par voie intraveineuse, par voie intramusculaire, intradermique ou sous-cutanée, ou par voie orale. De manière plus préférée, la composition vaccinale comprenant des polypeptides selon la description, sera administrée par voie intramusculaire, au travers de l'alimentation ou par nébulisation à plusieurs reprises, de manière étalée dans le temps.

Leurs modes d'administration, posologies et formes galéniques optimaux peuvent être déterminés selon les critères généralement pris en compte dans l'établissement d'un traitement adapté à un animal comme par exemple l'âge ou le poids, la gravité de son état général, la tolérance au traitement et les effets secondaires constatés.

La présente invention a également pour objet l'utilisation des séquences nucléotidiques de circovirus MAP selon la description, pour la construction de vecteurs rétroviraux autoréplicatifs et les applications thérapeutiques de celles-ci, notamment dans le domaine de la thérapie génique humaine in vivo.

La faisabilité de la thérapie génique appliquée à l'homme n'est plus à démontrer et ceci concerne de nombreuses applications thérapeutiques comme les maladies génétiques, les maladies infectieuses et les cancers. De nombreux documents de l'art antérieur décrivent les moyens de mettre en oeuvre une thérapie génique, notamment par l'intermédiaire de vecteurs viraux. D'une manière générale, les vecteurs sont obtenus par délétion d'au moins une partie des gènes viraux qui sont remplacés par les gènes d'intérêt thérapeutique. De tels vecteurs peuvent être propagés dans une lignée de complémentation qui fournit en trans les fonctions virales délétées pour générer une particule de vecteur viral défective pour la réplication mais capable d'infecter une cellule hôte. A ce jour, les vecteurs rétroviraux sont parmi les plus utilisés et leur mode d'infection sont largement décrits dans la littérature accessible à l'homme de l'art.

Le principe de la thérapie génique est de délivrer un gène fonctionnel, dit gène d'intérêt, dont l'ARN ou la protéine correspondante produira l'effet biochimique désiré dans les cellules ou tissus ciblés. D'une part, l'insertion de gènes permet l'expression prolongée de molécules complexes et instables comme des ARNs ou des protéines qui peuvent être extrêmement difficiles voire impossibles à obtenir ou à administrer directement. D'autre part, l'insertion contrôlée du gène désiré à l'intérieur de cellules spécifiques ciblées permet de réguler le produit d'expression dans des tissus définis. Pour cela, il est nécessaire de pouvoir insérer le gène thérapeutique désiré à l'intérieur de cellules choisies et donc de disposer de méthode d'insertion capable de cibler spécifiquement les cellules ou les tissus choisis.

Parmi les méthodes d'insertion de gènes, comme par exemple la micro-injection, notamment l'injection d'ADN plasmidique nu (Derse, D. et al., 1995, et Zhao, T.M. et al., 1996), l'électroporation, la recombinaison homologue, l'utilisation de particules virales, comme les rétrovirus, est largement répandue. Cependant, appliqués in vivo, les systèmes de transfert de gène de type rétroviral recombinant présentent à la fois un faible pouvoir infectieux (concentration insuffisante de particules virales) et un manque de spécificité vis-à-vis des cellules cibles choisies.

La réalisation de vecteurs viraux cellules spécifiques, présentant un tropisme tissu-spécifique, et dont la transduction du gène d'intérêt peut être effectuée de manière adéquate par les cellules cibles, est réalisable par exemple en fusionnant un ligand spécifique des cellules hôtes cibles à la partie N-terminale d'une protéine de surface de l'enveloppe de circovirus MAP. On peut citer par exemple la construction de particules rétrovirales présentant la molécule CD4 à la surface de l'enveloppe de manière à cibler les cellules humaines infectées par le virus HIV (YOUNG, J.A.T. et al., Sciences 1990, 250, 1421-1423), de particules virales présentant une hormone peptidique fusionnée avec une protéine de l'enveloppe pour infecter spécifiquement les cellules exprimant le récepteur correspondant (KASAHARA, N. et al., Sciences 1994, 266, 1373-1376) ou bien encore de particules virales présentant un polypeptide fusionné capable de se fixer sur le récepteur du facteur de croissance de l'épiderme (EGF)(COSSET, F.L. et al., J. of Virology 1995, 69, 10, 6314-6322). Dans une autre approche, des fragments simple chaîne d'anticorps dirigés contre des antigènes de surface des cellules cibles, sont insérés par fusion à la partie N-terminale de la protéine d'enveloppe (VALSESIA-WITTMAN, S. et al., J. of Virology 1996, 70, 3, 2059-2064 ; TEARINA CHU, T. H. et al., J. of Virology 1997, 71, 1, 720-725).

Un gène d'intérêt en usage dans la description peut être obtenu d'un organisme eucaryote, procaryote ou d'un virus par toute technique conventionnelle. Il est, de préférence, capable de produire un produit d'expression ayant un effet thérapeutique et il peut s'agir d'un produit homologue à la cellule hôte ou, de manière alternative, hétérologue. Dans le cadre de la présente invention, un gène d'intérêt peut coder pour un produit (i) intracellulaire (ii) membranaire présent à la surface de la cellule hôte ou (iii) sécrété hors de la cellule hôte. Il peut donc comprendre des éléments additionnels appropriés comme, par exemple, une séquence codant pour un signal de sécrétion. Ces signaux sont connus de l'homme de l'art.

Conformément aux buts poursuivis par la présente invention, un gène d'intérêt peut coder pour une protéine correspondant à tout ou partie d'une protéine native telle que trouvée dans la nature. Il peut également s'agir d'une protéine chimérique, par exemple provenant de la fusion de polypeptides d'origines diverses ou d'un mutant présentant des propriétés biologiques améliorées et/ou modifiées. Un tel mutant peut être obtenu par des techniques de biologie classiques par substitution, délétion et/ou addition d'un ou plusieurs résidus acides aminés.

On préfère tout particulièrement mettre en oeuvre un gène d'intérêt thérapeutique codant pour un produit d'expression capable d'inhiber ou retarder l'établissement et/ou le développement d'une maladie génétique ou acquise. Un vecteur selon la description est particulièrement destiné à la prévention ou au traitement de la mucoviscidose, de l'hémophilie A ou B, de la myopathie de Duchenne ou de Becker, du cancer, du SIDA et d'autres bactéries ou maladies infectieuses dues à un organisme pathogène : virus, bactérie, parasite ou prion. Les gènes d'intérêt utilisables dans la présente invention, sont ceux qui codent par exemple pour les protéines suivantes :
- une cytokine et notamment une interleukine, un interféron, un facteur de nécrose tissulaire et un facteur de croissance et notamment hématopoïétique (G-CSF, GM-CSF),
- un facteur ou cofacteur impliqué dans la coagulation et notamment le facteur VIII, le facteur von Willebrand, l'antithrombine III, la protéine C, la thrombine et l'hirudine,
- une enzyme ou un inhibiteur d'enzyme tel que les inhibiteurs de protéases virales,
- un produit d'expression d'un gène suicide comme la thimidine kinase du virus HSV (virus de l'herpès) de type 1,
- un activateur ou un inhibiteur de canaux ioniques,
- une protéine dont l'absence, la modification ou la dérégulation de l'expression est responsable d'une maladie génétique, telle que la protéine CFTR, la dystrophine ou minidystrophine, l'insuline, l'ADA (adénosine diaminose), la glucocérébrosidase et la phénylhydroxylase,
- une protéine capable d'inhiber l'initiation ou la progression de cancers, telle que les produits d'expression des gènes suppresseurs de tumeurs, par exemple les gènes P53 et Rb,
- une protéine capable de stimuler une réponse immunitaire ou un anticorps, et
- une protéine capable d'inhiber une infection virale ou son développement, par exemple les épitopes antigéniques du virus en cause ou des variants altérés de protéines virales susceptibles d'entrer en compétition avec les protéines virales natives.

La description concerne ainsi les vecteurs caractérisés en ce qu'ils comprennent une séquence nucléotidique de circovirus MAP selon la description, et en ce qu'ils comprennent en outre un gène d'intérêt.

La présente description concerne également des particules virales générées à partir dudit vecteur selon la description. Elle concerne de plus des méthodes pour la préparation de particules virales selon la description, caractérisées en ce qu'elles mettent en oeuvre un vecteur selon la description, incluant les pseudoparticules virales (VLP, Virus-Like Particles).

La description a également pour objet des cellules animales transfectées par un vecteur selon la description.

Sont également comprises dans la description les cellules animales, notamment de mammifères, infectées par une particule virale selon la description.

La présente description concerne également un vecteur, une particule virale ou une cellule selon la description, pour le traitement et/ou la prévention d'une maladie génétique ou d'une maladie acquise comme le cancer ou une maladie infectieuse. La description s'adresse également à une composition pharmaceutique comprenant à titre d'agent thérapeutique ou prophylactique un vecteur ou une cellule selon la description, en association avec un véhicule acceptable d'un point de vue pharmaceutique.

D'autres caractéristiques et avantages de l'invention apparaissent dans les exemples et les figures suivants :

### Légendes des figures :

Figure 1 : Schéma expérimental ayant permis d'aboutir à l'isolement et à l'identification du circovirus associé au MAP de type A et B.
   Essai 1 : reproduction expérimentale du MAP par inoculation de broyats d'organes de porcs d'élevages atteints de MAP.
   Essai 2 : reproduction expérimentale du MAP.
   Essai 3 : reproduction expérimentale du MAP.
   Essai 4 : pas de reproduction expérimentale du MAP.
Figure 2 : Organisation du génome du circovirus associé au MAP de type A (PCVA)
   - brin de polarité (+) (SEQ ID N° 1) ;
   - brin de polarité (-) (SEQ ID N° 2, représentée suivant l'orientation 3' → 5');
   - séquences d'acides aminés des protéines codées par les deux brins d'ADN dans les trois cadres de lecture possibles.
Figure 3 : Alignement de la séquence nucléotidique SEQ ID N° 1 du circovirus MAP de type A (PCVA) et des circovirus souche MEEHAN et souche MANKERTZ des lignées cellulaires porcines.

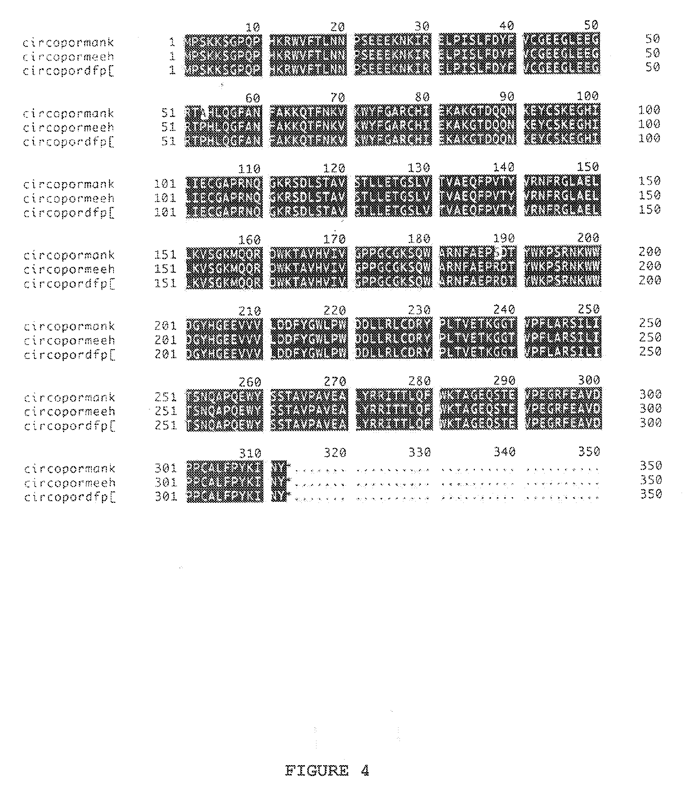

Figure 4 : Alignement de la séquence d'acides aminés SEQ ID N° 6 de polypeptide codé par la séquence nucléotidique SEQ ID N° 3 (ORF1) du circovirus MAP de type A (PCVA) et des séquences nucléotidiques correspondantes des circovirus souche MEEHAN et souche MANKERTZ des lignées cellulaires porcines.

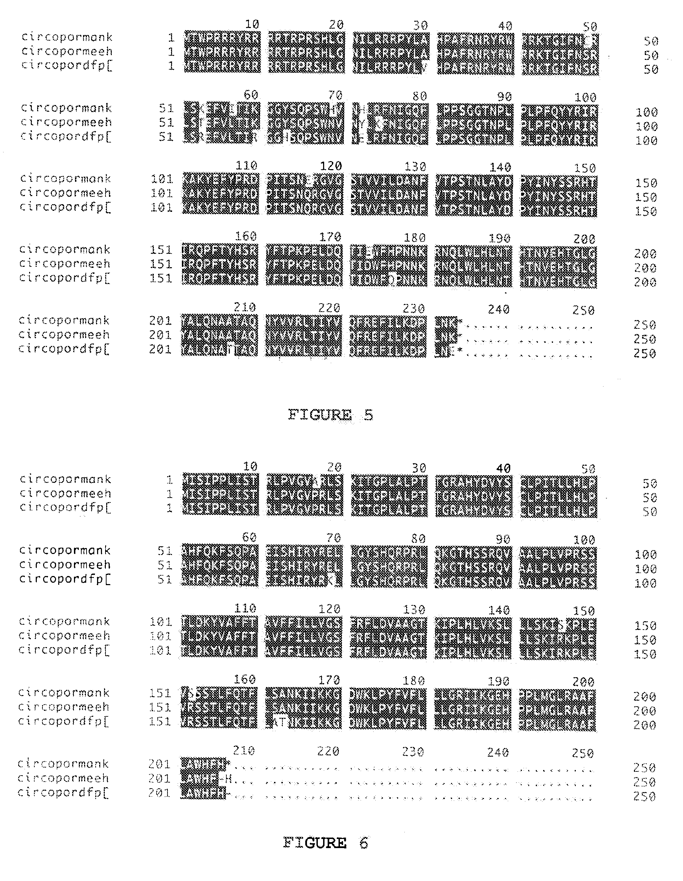

Figure 5 : Alignement de la séquence d'acides aminés SEQ ID N° 7 de polypeptide codé par la séquence nucléotidique SEQ ID N° 4 (ORF2) du circovirus MAP de type A (PCVA) et des séquences nucléotidiques correspondantes des circovirus souche MEEHAN et souche MANKERTZ des lignées cellulaires porcines.
Figure 6 : Alignement de la séquence d'acides aminés SEQ ID N° 8 de polypeptide codé par la séquence nucléotidique SEQ ID N° 5 (ORF3) du circovirus MAP de type A (PCVA) et des séquences nucléotidiques correspondantes des circovirus souche MEEHAN et souche MANKERTZ des lignées cellulaires porcines.
Figure 7 : Analyse par Western-Blot des protéines recombinantes du circovirus MAP de type A (PCVA).
   Les analyses ont été réalisées sur des extraits cellulaires de cellules Sf9 obtenus après infection par le baculovirus recombinant PCV ORF 1.
Figure 8 : Organisation du génome du circovirus associé au MAP de type B (PCVB)
   - brin de polarité (+) (SEQ ID N° 9) ;
   - brin de polarité (-) (SEQ ID N° 10, représentée suivant l'orientation 3' → 5');
   - séquences d'acides aminés des protéines codées par les deux brins d'ADN dans les trois cadres de lecture possibles.
Figure 9 : Evolution du gain moyen quotidien (GMQ) de porcs d'élevage atteints de maladie d'amaigrissement du porcelet (MAP ou DFP), placés dans les conditions expérimentales.
Figure 10 : GMQ comparée pour les 3 lots de porcs (F1, F3 et F4) calculée sur une période de 28 jours, après essai de vaccination.
Figure 11 : Hyperthermie supérieure à 41°C, exprimée en pourcentage comparée pour les 3 lots de porcs (F1, F3 et F4) calculée par semaine sur une période de 28 jours, après essai de vaccination.
Figure 12 : Membranes des spots peptides correspondant aux ORF2 révélées à l'aide d'un sérum de porc infecté, issu d'un élevage conventionnel.

Les numéros de peptides spécifiques du circovirus de type B ainsi que leurs homologues non réactifs (type A) sont indiqués en gras.

Les peptides immunogènes non spécifiques sont indiqués en italique.

Figure 13 : Alignement des séquences d'acides aminés des protéines codées par l'ORF2 du circovirus MAP de type A et par l'ORF'2 du circovirus MAP de type B. La position de 4 peptides correspondant à des épitopes spécifiques du circovirus MAP de type B est indiquée sur la séquence correspondante en trait gras, leur homologue sur la séquence du circovirus MAP de type A est également indiqué par un trait simple.

### EXEMPLES ILLUSTRATIFS

### EXEMPLE 1 : Clonage, séquençage et caractérisation du circovirus MAP de type A (PCVA)

### 1 - Procédures expérimentales

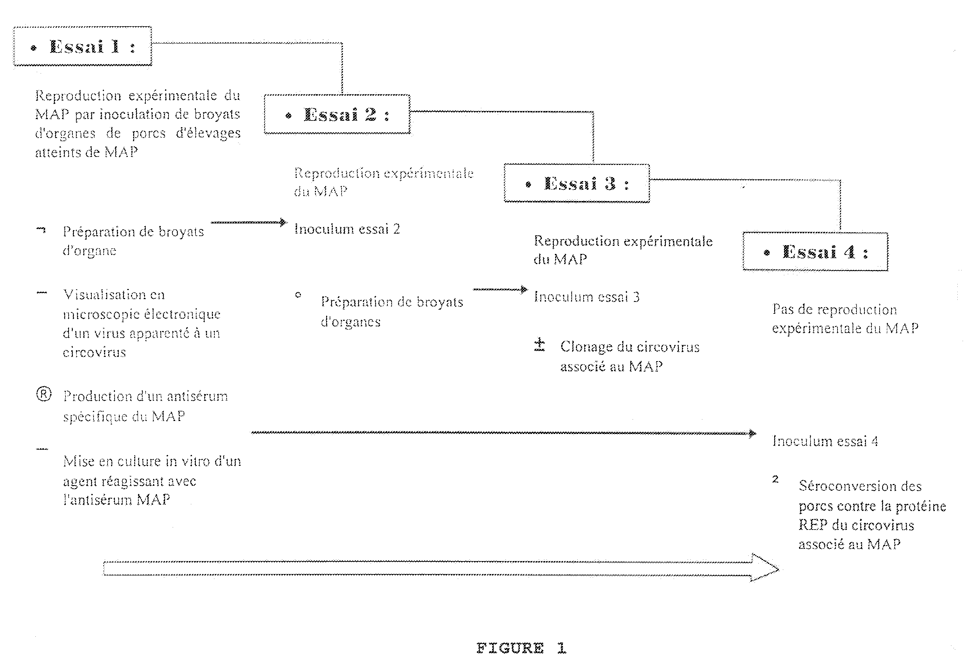

### Reproduction expérimentale de l'infection et de son syndrome (cf. figure 1).

Un premier essai a été effectué avec des porcs issus d'un élevage de très bonne tenue, mais atteint de la maladie de l'amaigrissement du porcelet (MAP) ou dénommée également DFP (Dépérissement Fatal du Porcelet). Des épreuves contact avec des porcs EOPS (Exempts d'organismes pathogènes spécifiés) ont montré un transfert de contaminant(s) se traduisant par une pathologie complexe associant hyperthermie, ralentissement de la croissance, diarrhée et conjonctivite. Le virus SDRP (syndrome dysgénésique et respiratoire porcin, maladie infectieuse due à un artérivirus) a été rapidement isolé à partir des porcs d'élevage et des porcs contact. L'ensemble des signes cliniques aurait pu être attribué à la présence du virus SDRP. Toutefois, deux porcs d'élevage ont présenté des signes de DFP sans que le virus SDRP soit isolé. Les analyses histologiques et formules sanguines ont cependant montré que ces porcs étaient atteints d'un processus infectieux d'origine virale.

Dans un deuxième essai, des porcs EOPS de 8 semaines ont été inoculés par voie intratrachéale avec les broyats d'organes provenant des deux porcs d'élevage atteints de DFP. Les porcs inoculés ont présenté de l'hyperthermie 8 à 9 jours post-infection, puis leur croissance a été ralentie. D'autres porcs EOPS, placés au contact, ont présenté des signes similaires, atténués, 30 jours après l'épreuve initiale. Aucune séroconversion vis-à-vis d'une souche européenne ou canadienne de virus SDRP n'a été enregistrée chez ces animaux.

Un troisième essai a permis de reproduire le syndrome à partir de prélèvements effectués sur les porcs du second essai.

### Conclusion

Le syndrome est reproduit dans les conditions expérimentales. Il est déterminé par au moins un agent infectieux, transmissible par contact direct. Les constantes cliniques sont une hyperthermie parfois élevée (supérieure ou égale 41,5°C) qui se développe 8 à 10 jours après infection. Un ralentissement de la croissance peut être observé. Les autres manifestations sont une inversion de la formule sanguine (inversion du rapport lymphocyte/polynucléaire de 70/30 à 30/70) et des lésions fréquentes sur les ganglions, notamment ceux drainant l'appareil respiratoire (hyper-trophie ganglionnaire, perte de structure avec nécrose et infiltration par des cellules mononucléées ou plurinucléées géantes).

### 2 - Etudes en laboratoire

Divers supports cellulaires incluant des cellules de rein de porc primaires ou en lignée, des cellules de testicule de porc, des cellules de rein de singe, des lymphocytes de porc, des macrophages alvéolaires de porc, des monocytes du sang circulant, ont été utilisés pour mettre en évidence la présence éventuelle d'un virus. Aucun effet cytopathique n'a été mis en évidence sur ces cellules. En revanche, l'utilisation d'un sérum de porc malade après infection expérimentale a permis de révéler un antigène intracellulaire dans les monocytes, les macrophages et environ 10 % des cellules de rein de porc (RP) infectées avec les broyats d'organe. Cette révélation indirecte a été faite en cinétique à différents temps de culture. Il en ressort que l'antigène apparaît initialement dans le noyau des cellules infectées avant de se répandre dans le cytoplasme. Les passages successifs en culture cellulaire n'ont pas permis d'amplifier le signal.

En microscopie électronique sur des broyats d'organes, il a été visualisé des particules sphériques marquées spécifiquement par le sérum de porcs malades, infectés dans les conditions expérimentales. La taille de ces particules est estimée à 20 nm.

Après deux passages de ces broyats d'organes sur lymphocytes de porc puis trois passages sur cellules de rein ou de testicule de porc, un effet cytopathique s'est développé et a été amplifié. Au microscope électronique a été visualisé un adénovirus qui, dans les conditions expérimentales, n'a pas reproduit le DFP (on note seulement un pic d'hyperthermie 24 à 48 heures après infection, puis plus rien).

Des bandes d'ADN dans certains prélèvements de porcs infectés dans les conditions expérimentales et ayant présenté des signes de la maladie ont pu être mis en évidence (résultats non représentés). Il existe une certaine correspondance entre les prélèvements donnant un résultat positif en culture cellulaire et ceux présentant une bande d'ADN.

### Conclusion

Au moins deux types de virus ont été mis en évidence dans les broyats d'organes issus de porcs malades de DFP. L'un est un adénovirus, mais il ne reproduit pas à lui seul la maladie. L'autre type de virus est un circovirus et est associés au DFP. Ce circovirus, dont deux types ont été isolés et séquencés, dénommés ci-après circovirus MAP de type A (ou PCVA) et circovirus MAP de type B (ou PCVB) présentent des mutations par rapport aux séquences connues de circovirus non pathogènes pour le porc.

### 3 - Clonage et séquençage de l'ADN du circovirus MAP de type A

Extraction de l'ADN forme réplicative (RF), clivage par l'enzyme Kpn I et amplification par un couple d'amorces flanquant le site de restriction Kpn I. Séquençage des deux brins au moins deux fois par la méthode de Sanger.

La séquence nucléique du brin de polarité (+) du génome du circovirus MAP de type A (ou PCVA), souche DFP, est représentée par la séquence SEQ ID N° 1 dans la liste des séquences, la séquence nucléique du brin de polarité(-) du génome du circovirus MAP de type A (ou PCVA) étant représentée par la séquence nucléique 3' → 5' de la figure 3 ou par la séquence SEQ ID N° 2 (représentée suivant l'orientation 5' → 3') dans la liste de séquences.

Les séquences d'acides aminés SEQ ID N° 6, SEQ ID N° 7 et SEQ ID N° 8 de la liste des séquences représentent respectivement les séquences des protéines codées par les séquences nucléiques des 3 cadres ouverts de lecture SEQ ID N° 3 (ORF1), correspondant à la protéine REP, SEQ ID N° 4 (ORF2) et SEQ ID N° 5 (ORF3), déterminées à partir de la séquence SEQ ID N° 1 du brin de polarité (+) ou de la séquence nucléique SEQ ID N° 2 du brin de polarité (-) du génome du circovirus MAP de type A.

### 4 - Comparaison des séquences nucléotidiques et d'acides aminés du circovirus MAP de type A (ou associé à la MAP) obtenues avec les séquences correspondantes de circovirus MEEHAN et MANKERTZ de lignées cellulaires porcines

### Utilisation du logiciel d'analyse de séquence d'ADN, DNASIS.

### Séquences des oligonucléotides utilisés comme amorces ou sondes dans les procédés de détection et/ou d'identification

1. détection spécifique du circovirus MAP de type A :
   amorce PCV 5 : 5' GTG TGC TCG ACA TTG GTG TG 3' ;
   amorce PCV 10 : 5' TGG AAT GTT AAC GAG CTG AG 3' ;
2. détection spécifique du circovirus des lignées cellulaires :
   amorce PCV 5 : 5' GTG TGC TCG ACA TTG GTG TG 3' ;
   amorce MEE1 : 5' TGG AAT GTT AAC TAC CTC AA 3' ;
3. détection différentielle:
   les couples d'amorces utilisés sont ceux par exemple décrits dans les paragraphes 1 et 2 ci-dessus ;
4. détection des formes réplicatives RF (replicative forms) circulaires monomériques :
   amorce PCV 5 : 5' GTG TGC TCG ACA TTG GTG TG 3' ;
   amorce PCV 6 : 5' CTC GCA GCC ATC TTG GAA TG 3' ;
5. détection des vecteurs portant les dimères en tandem :
   dimère Nar :
   amorce KS 620 : 5' CGC GCG TAA TAC GAC TCA CT 3' ;
   amorce PCV 5 : 5' GTG TGC TCG ACA TTG GTG TG 3' ;
   dimère Kpn :
   amorce KS 620 : 5' CGC GCG TAA TAC GAC TCA CT 3' ;
   amorce PCV 6 : 5' CTC GCA GCC ATC TTG GAA TG 3' ;
6. détection différentielle :
   les couples d'amorces utilisés sont ceux par exemple décrits dans les paragraphes 4 et 5 ci-dessus.

Les procédés utilisant les couples d'amorces décrits dans les paragraphes 4 et 5 sont particulièrement intéressants pour détecter de façon différentielle les formes monomériques circulaires de formes réplicatives spécifiques du virion ou de l'ADN en réplication et les formes dimériques retrouvées dans les constructions moléculaires dites en tandem.

Les constructions en tandem du génome viral (dimères) telles que les constructions utilisées pour la préparation du vecteur pBS KS+ Tandem PCV Kpn I, déposé à la CNCM sous le numéro I-1891, le 3 juillet 1997 (E-coli transformée par ledit vecteur) sont très intéressantes pour leur utilisation dans des méthodes de production en quantité suffisante d'un inoculum constitué d'ADN, destiné à la production de virus et ceci en l'absence de protocole satisfaisant de production de virus sur système cellulaire. Cesdites méthodes de production utilisant ces constructions en tandem du génome viral permettront d'étudier par mutation les facteurs de virulence et par voie de conséquence pourront être utilisées pour la fabrication d'une collection de virus portant les mutations indiquées dans la construction des vecteurs qui présenteront le tropisme et la virulence appropriés. Ces vecteurs à structure autoréplicative présentent des propriétés recherchées en transfert de gènes, notamment pour leurs applications en thérapie génique, et en vaccinologie.

### Analyse par Western-Blot des protéines recombinantes du circovirus MAP de type A

Les résultats ont été obtenus en utilisant un antisérum spécifique du circovirus MAP produit lors de l'essai 1 (cf. figure 1).

### Type de produits analysés

Les analyses ont été réalisées sur des extraits cellulaires de cellules Sf9 obtenus après infection par le baculovirus recombinant PCV ORF 1.

La culture des cellules Sf9 a été réalisée sur boîte de Pétri de 25 cm² selon les méthodes de culture standard pour ces cellules. Après centrifugation, les culots cellulaires sont repris par 300 µl de tampon PBS (tampon phosphate salin).

### Electrophorèse (PAGE- SDS)

L'électrophorèse est réalisée sur les extraits cellulaires de cellules Sf9 obtenus précédemment sur 5 échantillons (cf. tableau 1 ci-dessous) dans les conditions suivantes :
% gel de polyacrylamide : 8 % ; Conditions : dénaturantes
Voltage : 80 V ; Durée : 135 mn.

**Tableau 1 : Nature des échantillons soumis à l'électro- phorèse**

| N° puits | 1 | 2 | 3 | 4 | 5 |
|---|---|---|---|---|---|
| Dépôts | PM Raoul | Raoul 24 h | Raoul 48 h | Raoul 72 h | Raoul 96 h |
| µl échant. | 10 | 15 | 15 | 15 | 15 |
| µl Laemli 4X | 0 | 5 | 5 | 5 | 5 |

### Légendes du tableau 1 :

Laemli 4X : tampon de charge
PM Rainbow : marqueurs de poids moléculaire (35, 52, 77, 107, 160 et 250 kD)
Raoul 24 h, 48h, 72h et 96h : produits d'expression de l'ORF1 du circovirus MAP de type A.

### Western-blot

Après électrophorèse, les bandes obtenues dans les différents puits sont transférées sur membrane de nitrocellulose pendant 1 h à 100 v dans un tampon TGM (Tris-glycine-méthanol).

Le Western-blot est réalisé dans les conditions suivantes :
1) Saturation par une solution contenant 5 % de lait écrémé ; 0,05 % Tween 20 dans un tampon TBS 1X (Tris buffer saline) pendant 30 min.
2) 1er anticorps :
   10 ml d'anticorps anticircovirus MAP de type A sont ajoutés dilués au 1/100, puis le milieu réactionnel est incubé une nuit à 4°C. Trois lavages de 10 min. en TBS 1X sont effectués.
3) 2ème anticorps :
   10 ml d'anticorps P164 de lapin anti-immunoglobulines de porc, couplés à la péroxydase (Dakopath) sont ajoutés dilués au 1/100, puis le milieu réactionnel est incubé 3 heures à 37°C. Trois lavages de 10 min. en TBS 1X sont effectués.
4) Révélation

Le substrat 4-Chloro-1-Naphtol, en présence d'eau oxygénée est utilisé pour la révélation.

### Résultats

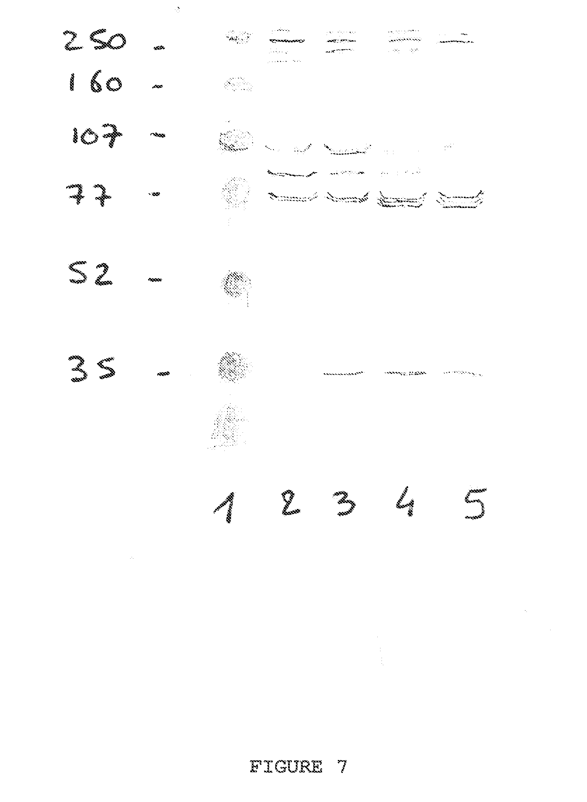

Les résultats sont représentés à la figure 7.

### Cinétique d'apparition des anticorps spécifiques de la protéine recombinante REP du circovirus MAP de type A exprimé en baculovirus après infection des porcs par le circovirus MAP de type A (essai 4, cf. figure 1)

Après infection des porcs, un échantillon de sérum de chacun des porcs infectés est prélevé à différentes périodes exprimées dans le tableau par la date du prélèvement (effectué ici la même année) puis est analysée par Western-blot.

La révélation des anticorps spécifiques est réalisée de la manière décrite précédemment.

Les résultats obtenus sont représentés par le tableau 2 ci-dessous.

**²Tableau 2 : Cinétique d'apparition des anticorps spécifiques**

| Echan. | Porcs | 10/06 | 16/06 | 23/06 | 01/07 | 08/07 | 15/07 | 21/07 |
|---|---|---|---|---|---|---|---|---|
| A3 | 1 | | | | | | Nég. | |
| Témoin- | 2 | | | | | | Nég. | |
| B2 | 1 | Nég. | Nég. | Nég. | + | + | ++ | +++ |
| Infec. | 2 | Nég. | Nég. | Nég. | Nég. | Nég. | Nég. | Nég. |
| RP+ | 3 | Nég. | Nég. | Nég. | Nég. | + | + | + |
| | 4 | Nég. | Nég. | Nég. | Nég. | Nég. | Nég. | ++ |

### Légendes du tableau 2 :

A3 Témoin : animaux témoins non infectés ;
B2 Infec. RP+ : animaux infectés avec des cellules de rein de porcs (RP) contenant le circovirus ;
Nég. : négatif ;
+, ++, +++ : échelle d'intensité de la réaction positive ;
10/06, 16/06, 23/06, 01/07, 08/07, 15/07, 21/07 : dates exprimées en jour/mois auxquelles ont été effectuées les différents prélèvements de sérum.

### EXEMPLE 2 : Clonage, séquençage et caractérisation du circovirus MAP type B (PCVB)

Les techniques utilisées pour le clonage, le séquençage et la caractérisation du circovirus MAP type B (PCVB) sont celles utilisées à l'exemple 1 ci-dessus pour le circovirus MAP type A (PCVA).

La séquence nucléique du brin de polarité (+) du génome du circovirus MAP de type B (ou PCVB) est représentée par la séquence SEQ ID N° 9 dans la liste des séquences, la séquence nucléique du brin de polarité (-) du génome du circovirus MAP de type B (ou PCVB) étant représentée par la séquence nucléique 3' → 5' de la figure 8. ou par la séquence SEQ ID N° 10 (représentée suivant l'orientation 5' → 3') dans la liste de séquences.

Les séquences d'acides aminés SEQ ID N° 14, SEQ ID N° 15 et SEQ ID N° 16 de la liste des séquences représentent respectivement les séquences des protéines codées par les séquences nucléiques des 3 cadres ouverts de lecture SEQ ID N° 11 (ORF'1), correspondant à la protéine REP, SEQ ID N° 12 (ORF'2) et SEQ ID N° 13 (ORF'3), déterminées à partir de la séquence SEQ ID N° 9 du brin de polarité (+) ou de la séquence nucléique SEQ ID N° 10 du brin de polarité (-) du génome du circovirus MAP de type B.

### EXEMPLE 3 : Analyse comparative des séquences nucléotidiques (ORF1, ORF2 et génomique) et des séquences d'acides aminés codés par l'ORF1 et l'ORF2 des circovirus MAP de type A (PCVA) et de type B (PCVB)

Les résultats exprimés en % d'homologie sont représentés dans les tableaux 3 et 4 ci-après.

**Tableau 3 : Analyse comparée des séquences d'acides aminés**

| % homologie | ORF1 | ORF2 |
|---|---|---|
| PCVA / PCVB | 80,4 | 56,2 |

**Tableau 4 : Analyse comparée des séquences nucléotidiques**

| % homologie | Génomique | ORF1 | ORF2 | Le reste |
|---|---|---|---|---|
| PCVA/PCVB | 70,4 | 80,4 | 60,1 | 66,1 |

### EXEMPLE 4 : Observation de la maladie et reproduction de la maladie dans les conditions expérimentales

### a) Essai N° 1 : Observation de la maladie

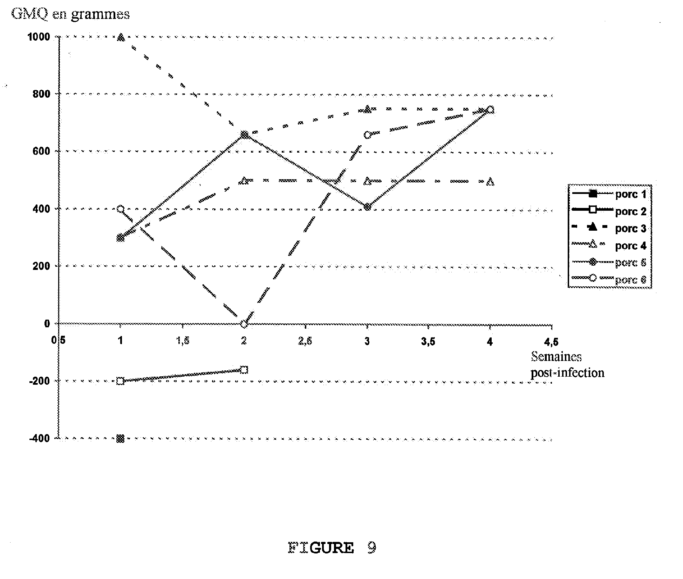

L'objectif est de prendre des animaux d'élevage en début de maladie et de les placer dans les conditions expérimentales pour suivre l'évolution de la pathologie et en décrire toutes les manifestations cliniques. Ce premier essai a été effectué sur 3 porcs d'élevage âgés de 10 semaines dont 2 étaient déjà malades (atteints de dépérissement), et sur 3 autres porcs âgés de 13 semaines, ne présentant pas de signes de maladie. L'observation clinique s'est étalée sur une période de 37 jours. Deux porcs de 10 semaines ont rapidement dépéri (porc 1 et 2, figure 9) et ont dû être euthanasiés 5 et 6 jours après leur arrivée. Un seul a présenté de l'hyperthermie sur 5 jours et de la diarrhée. Deux autres porcs ont présenté de la dyspnée et de la toux dont un a fait en outre de l'hyperthermie, supérieure à 41°C, les deux premiers jours de son séjour. Un autre porc a eu une croissance ralentie à la deuxième semaine (porc 6, figure 9), sans qu'aucun autre signe clinique ne soit relevé. Sur le plan lésionnel, 5 porcs sur 6 ont présenté des lésions macroscopiques de pneumonie grise, le sixième présentait des lésions cicatricielles sur le poumon.

### b) Essai N° 2 : Reproduction de la maladie à partir d'inoculums préparés sur porcs d'élevage.

Les deux porcs malades dans l'essai 1 ont servi à préparer des inocula que l'on a testé dans l'essai 2 sur des porcs exempts d'organismes pathogènes spécifiques (EOPS, SPF en version anglo-saxonne). Les porcs EOPS étaient âgés de 9 semaines au moment de l'inoculation. Les résultats cliniques et lésionnels sont présentés dans le tableau 5.

**Tableau 5 : Récapitulatif des mesures effectuées lors des reproductions expérimentales de la MAP (entre parenthèses sont reportées les valeurs des animaux témoins, les valeurs soulignées indiquent une différence entre animaux infectés et animaux témoins)**

| Essai | 2 | 3 | 4 | 5 | 6 | 7 |
|---|---|---|---|---|---|---|
| Mesure | | | | | | |
| Statut des porcs | EOPS CNEVA | EOPS terrain | EOPS CNEVA | EOPS CNEVA | Conventionnels | Conventionnels |
| Age | 9 semaines | 6 semaines | 5 semaines | 5 semaines | 5 semaines | 6-7 semaines |
| Nombre | 4 | 6 | 12 | 8 | 8 | 8 |
| Voie d'inoculation | Voie intratrachéale | Voie intratrachéale | Voie intratrachéale + intramusculaire | Voie intratrachéale + intramusculaire | Voie intratrachéale + intramusculaire | Voie intratrachéale + intramusculaire |
| Titre inoculum par porc | ND * | ND* | 10^{4.53} TCID₅₀ par ml: 1 ml IM + 5 ml IT | 10^{4.53} TCI₅₀ par ml: 1 ml IM + 5 ml IT | 10^{4.53} TCID₅₀ par ml: 1 ml IM + 5 ml IT | 10^{4.53} TCID₅₀ par ml: 1 ml IM + 5 ml IT |
| Début des hyperthermies | 10 jours post-infection | 9-13 jours post-infection | 12-13 jours post-infection | 9-14 jours post-infection | 8-12 jours post-infection | 12 jours post-infection |
| % de porcs en hyperthermie ** | 100 % | 83% | 92% | 100 % | 75% | 88% |
| Nb de jours d'hyperthermie par porc ** | 7 | 4,5 | 3,3 | 5,8 | 7,5 | 11,6 |
| Températures maximales *** | 40,4 à 41,7 °C | 40,6 à 42,3 °C | 40,2 à 41,6 °C | 40,3 à 40,8°C | 40,6 à 42 °C | 40,2 à 41,9°C |
| Hyperthermie **** % par semaine | | | | | | |
| S1 | 3,5 (3,5) | 17 (36) | 7 (5) | 37 (17) | 16 (17) | 20 (28) |
| S2 | *42 (3,5)* | 7 (13) | *13 (1)* | *21 (3)* | *52 (10)* | 37 (28) |
| S3 | *35 (3.5)* | *33 (10)* | *28 (7)* | *62* (*2*) | *34 (12)* | *79 (17)* |
| S4 | *21 (3.5)* | 28(*7*) | 5 (0) | 6 (3) | 25 (22) | *55 (3)* |
| GMQ: | | | | | | |
| S1 | 928 (1053) | 417 (357) | 564 (620) | 650 (589) | 401 (407) | 509 (512) |
| S2 | *678 (1028)* | *428 (617)* | *503 (718)* | 612 (584) | *294 (514)* | 410 (310) |
| S3 | *661 (1000)* | 771 (642) | *381 (657)* | *520 (851)* | *375 (586)* | 435 (440) |
| S4 | *786 (1100)* | *550 (657)* | 764 (778) | 641 (696) | *473 (610)* | *451* (*681*) |
| Transmission porcs contacts | Oui à 100 % | Oui à 75 % | Non testé | Non testé | Non testé | Non testé |
| % lésions pulmonaires | 25 | 75 | 0 | 25 | 25 | 12 |
| % lésions ganglionnaires | 17 | 33 | 67 | 25 | 50 | 12 |

| | | | | | | |
|---|---|---|---|---|---|---|
| * ND : non déterminé, ** hyperthermie lorsque la température est supérieure à 40°C, *** éventail des températures maximales relevées au niveau individuel, **** le pourcentage correspond au nombre de relevés de température supérieure à 40°C divisé par le nombre total de relevés de température dans la semaine sur l'ensemble des porcs. | | | | | | |

Dans cet essai, il n'y a pas eu dépérissement, tout au plus un ralentissement de la croissance à la deuxième, troisième ou quatrième semaine après infection. Ces données illustrent que certaines conditions d'élevage favorisent probablement l'expression de la maladie.

### c) Essais N° 3 à N° 7 : Reproduction des essais expérimentaux

La multiplication des essais expérimentaux sur porcs a eu pour objectif de maîtriser et de mieux caractériser le modèle expérimental. L'ensemble des résultats est présenté dans le tableau 5.

Dans les conditions expérimentales, la MAP se caractérise ainsi par une incubation longue, de 8 à 14 jours, des hyperthermies franches sur 2 à 8 jours, une diminution de la consommation alimentaire et un ralentissement de la croissance pondérale à la deuxième, troisième ou quatrième semaine post-infection. Le tableau lésionnel associé à cette expression clinique comprend pour l'essentiel, des hypertrophies ganglionnaires et des lésions de pneumonie.

### Conclusion

La mise au point de ce modèle expérimental permet de mettre en évidence de manière indiscutable le rôle étiologique direct du circovirus MAP dans la maladie. De plus, ce modèle est l'outil indispensable pour la compréhension des mécanismes pathogéniques et l'étude de futurs candidats vaccins.

### EXEMPLE 5 : Mise en évidence de l'efficacité protectrice de composition vaccinale réalisée à partir de fragments nucléiques de séquence de circovirus MAP

### 1) Animaux utilisés pour l'étude

Des porcelets présentant la maladie MAP, reproduite dans les conditions expérimentales décrite au paragraphe c) de l'Exemple 4, ont été utilisés dans un protocole d'évaluation de l'efficacité de composition vaccinale comprenant des fragments nucléiques de séquence de circovirus MAP.

### 2) Composition vaccinale testée et protocole de vaccination

### a) Composants utilisés pour l'étude

Les plasmides ont été obtenus à partir du plasmide pcDNA3 de INVITROGENE

### - Plasmides pcDNA3 ORF-

Ces plasmides sont des plasmides ne portant pas d'insert d'acide nucléique de circovirus MAP et sont utilisés à titre de plasmide témoin négatif.

### - Plasmide pcDNA3ORF1+ et plasmide pcDNA3ORF2+

Les plasmides pcDNA3ORF1+ et pcDNA3ORF2+ sont des plasmides qui portent un insert d'acide nucléique de la séquence du circovirus MAP de TYPE B, respectivement un insert comprenant le fragment d'acide nucléique SEQ ID N° 11 (ORF'1) codant pour la protéine Rep de séquence SEQ ID N° 14 et un insert comprenant le fragment d'acide nucléique SEQ ID N° 12 (ORF'2) codant pour la protéine de séquence SEQ ID N° 15, correspondant probablement à la protéine de capside, ces constructions nucléiques comprenant le codon ATG d'initition de la séquence codante de la protéine correspondante.

### - Plasmide GMCSF+

Le GM-CSF (Granulocyte/macrophage-colony stimulating factor) est une cytokine qui intervient dans le développement, la maturation et l'activation des macrophages, des granulocytes et des cellules dendritiques présentatrices d'antigène. L'apport bénéfique du GM-CSF dans la vaccination est estimé être une activation cellulaire avec notamment le recrutement et la différenciation de cellules présentatrices d'antigène.

Ce plasmide pcDNA3-GMCSF+ porte un insert d'acide nucléique codant pour le facteur de stimulation de colonies granulocytes/macrophage, la protéine GM-CSF.

Le gène codant pour cette protéine GM-CSF a été cloné et séquencé par Inumaru et al. (Immunol. Cell Biol., 1995, 73 (5), 474-476). Le plasmide pcDNA3-GMCSF+ a été obtenu auprès du Dr. B. Charley de L'INRA de Jouy-en-Josas (78, France).

### - Baculovirus recombinants

Les baculovirus dits ORF- sont des virus ne portant pas d'insert comprenant un fragment d'acide nucléique capable d'exprimer une protéine de circovirus MAP.

Les baculovirus dits ORF1+ (BAC ORF1+) ou ORF2+ (BAC ORF2+) sont des baculovirus recombinants portant respectivement un insert comprenant un fragment d'acide nucléique SEQ ID N° 11 (ORF'1) et un insert comprenant le fragment d'acide nucléique SEQ ID N° 12 (ORF'2).

### - Adjuvant

L'adjuvant fourni par la Société Seppic filiale de AIR LIQUIDE est l'adjuvant correspondant à la référence AIF SEPPIC.

### b) Protocole de vaccination

Des porcelets sevrés âgés de 3 semaines sont répartis en quatre lots A, B, C et D comprenant chacun 8 porcelets.

Les lots A, B et C, âgés de 3 semaines, reçoivent chacun une première injection (injection M1) de 1 ml contenant 200 microgrammes de plasmides (ADN nu) en PBS, pH : 7,2, par voie intramusculaire pour chacun des plasmides mentionnés ci-après pour chaque lot, puis, à l'âge de 5 semaines une deuxième injection (injection M2) comprenant ces mêmes plasmides. Une troisième injection est pratiquée simultanément de l'autre côté du cou. Cette troisième injection comprend 1 ml d'une suspension contenant 5.10⁶ cellules infectées par baculovirus recombinants et 1 ml d'adjuvant AIF SEPPIC.

### Lot A (F1) (Lot témoin) :

### - première injection

Plasmide pcDNA3ORF1-, plasmide pcDNA3ORF2- et plasmide GMCSF+.

### - deuxième et troisième injection (simultanées)

Plasmide pcDNA3ORF1-, plasmide pcDNA3ORF2- et plasmide GMCSF+ ;
Cellules transformées par baculovirus ne contenant pas d'insert d'acide nucléique codant pour une protéine de circovirus MAP ;
Adjuvant AIF SEPPIC.

### Lot B (F2) (Lot témoin) :

### - première injection

Plasmide pcDNA3ORF1-, plasmide pcDNA3ORF2- et plasmide GMCSF+ ;

### - deuxième et troisième injection (simultanées)

Plasmide pcDNA3ORF1-, plasmide pcDNA3ORF2- et plasmide GMCSF+ ;
Cellules transformées par baculovirus ne contenant pas d'insert d'acide nucléique codant pour une protéine de circovirus MAP ;
Adjuvant AIF SEPPIC.

### Lot C (F3) :

### - première injection

Plasmide pcDNA3ORF1+, plasmide pcDNA3ORF2+ et plasmide GMCSF+ ;

### - deuxième et troisième injection (simultanées)

Plasmide pcDNA3ORF1+, plasmide pcDNA3ORF2+ et plasmide GMCSF+ ;

Cellules transformées par des baculovirus recombinants BAC ORF1+ et BAC ORF2+ capables d'exprimer respectivement la protéine Rep de séquence SEQ ID N° 14 et la protéine de séquence SEQ ID N° 15 du circovirus MAP de TYPE B.

### Lot D (F4) (lot témoin) : aucune injection

Les lots de porcelets B, C et D sont infectés (mis à l'épreuve) à l'âge de 6 semaines alors que le lot A n'est pas soumis à l'épreuve.

### 3) Suivi des lots

- comptage toux-éternuement : 15 minutes / lot / jour ;
- consistance matières fécales : tous les jours ;
- enregistrements habituels : Prise de sang hebdomadaire, pesées ;
- pesées des refus alimentaires : 3 fois par semaine ;
- calcul du gain moyen quotidien en poids (gmq) ;

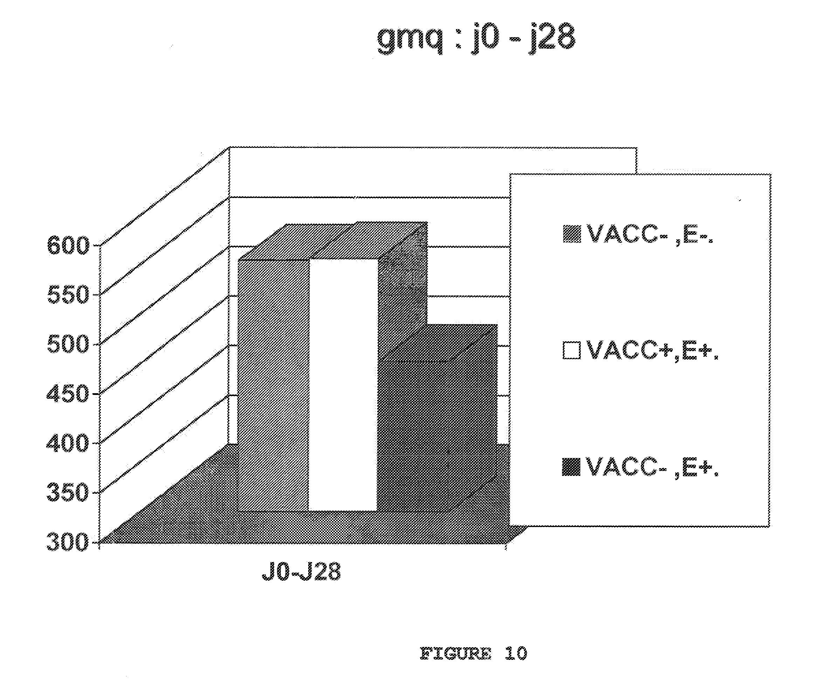

Les gains moyens quotidiens ont été calculés pour chacun des lots sur une période de 28 jours suivant la mise à l'épreuve (cf. figure 10), un calcul intermédiaire du gmq a été également effectué pour chacun des lots sur la première et la seconde période de 14 jours. Les résultats obtenus sont reportés ci-après dans le tableau 6.

**Tableau 6 : Gains moyens quotidiens**

| | F1 | F2 | F3 | F4 |
|---|---|---|---|---|
| j0-j14 | 411 g | 450 g | 511 g | 461 g |
| j14-j28 | 623 g | 362 g | 601 g | 443 g |
| j0-j28 | 554 g | 406 g | 556 g | 452 g |

### - Mesure de l'hyperthermie

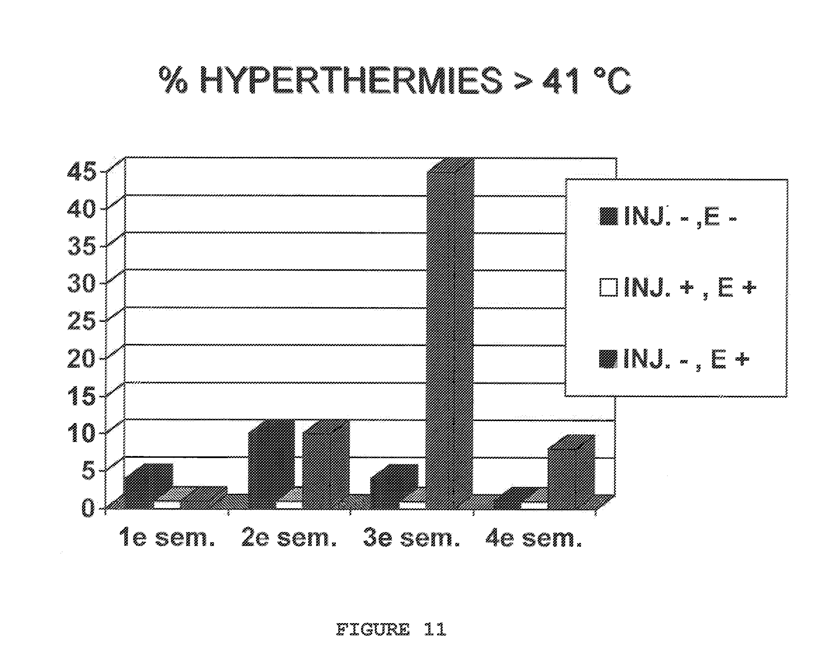

La mesure de l'hyperthermie, supérieure à 41°C (cf. figure 11) et supérieure à 40,2°C, a été effectuée pour chacun des lots sur une période totale de 28 jours suivant la mise à l'épreuve. Les résultats obtenus, correspondant au rapport exprimé en pourcentage entre le nombre de relevés thermiques supérieurs à 41°C (ou supérieurs à 40,2°C) et entre le nombre total de relevés thermiques effectués sur l'ensemble des porcs par période d'une semaine, sont reportés ci-après dans les tableaux 7 et 8, respectivement pour les mesures d'hyperthermie supérieure à 41°C et supérieure à 40,2°C.

**Tableau 7 : Hyperthermies > 41°C**

| | F1 | F2 | F3 | F4 |
|---|---|---|---|---|
| S1 | 4.1 | 0. | 0. | 0. |
| S2 | 10.7 | 16. | 0. | 8.9 |
| S3 | 4.7 | 27. | 0. | 45. |
| S4 | 0. | 0. | 0. | 7.5 |

**Tableau 8 : Hyperthermies > 40.2**

| | F1 | F2 | F3 | F4 |
|---|---|---|---|---|
| S1 | 29.1 | 10.41 | 29.1 | 20.8 |
| S2 | 28.5 | 39.2 | 10.7 | 37.5 |
| S3 | 14.3 | 68.7 | 25.0 | 81.2 |
| S4 | 3.3 | 17.5 | 20.0 | 55 |

### 4) Conclusion

Les enregistrements effectués montrent clairement que les animaux qui ont reçu les trois injections d'une composition vaccinale comprenant des fragments d'acide nucléique de circovirus MAP selon la description et /ou capables d'exprimer des protéines recombinantes de circovirus MAP, en particulier de type B, n'ont pas présenté d'hyperthermie (cf. figure 10). Ces animaux n'ont pas connu en outre de baisse de leur croissance, les gmq étant comparables à ceux des animaux témoins non infectés (cf. figure 9). Ils n'ont présenté aucun signe clinique particulier.

Ces résultats mettent en évidence la protection efficace des porcelets contre l'infection par un circovirus MAP de la description, agent primaire responsable de la MAP ou DFP, apportée par une composition vaccinale préparée à partir de fragment d'acide nucléique de la séquence nucléique de circovirus MAP selon la description, en particulier de type B, et/ou à partir de protéines recombinantes codées par ces fragments d'acides nucléiques.

Ces résultats montrent en particulier que les protéines codées par les ORF1 et ORF2 de circovirus MAP selon la description sont des protéines immunogènes induisant une réponse protectrice efficace pour la prévention de l'infection par un circovirus MAP.

### EXEMPLE 6 : Diagnostic sérologique de Circovirus MAP par immunodosage utilisant des protéines recombinantes ou peptides de synthèse de Circovirus MAP

### A - Diagnostic Sérologique par protéines recombinantes

L'identification et le séquençage de circovirus porcin MAP permettent de produire par les techniques de recombinaison génétique bien connues de l'homme des protéines recombinantes de circovirus MAP.

Par ces techniques, des protéines recombinantes codées en particulier par l'ORF'2 du circovirus MAP, type B, ont été exprimées par des cellules d'insecte Sf9 transformées puis isolées.

Ces protéines recombinantes codées par l'ORF'2 sont extraites, après culture des cellules sf9 transformées, par lyse cellulaire thermique grâce à 3 cycles de surgélation/décongélation -70°C/+37°C. Des cellules Sf9 saines ou Sf9 témoins non transformées sont également lysées.

Ces deux fractions antigéniques issues de cellules Sf9 témoins non transformées et de cellules Sf9 exprimant l'ORF'2 sont précipitées à 4°C par une solution à 60 % plus ou moins 5 % de sulfate d'ammonium saturé. Un dosage de protéines totales est réalisé à l'aide du kit Biorad. 500ng de protéines Sf9 témoin et de protéines Sf9 exprimant l'ORF'2 semi-purifiées, en solution dans du tampon bicarbonate 0,05 M pH 9,6 sont adsorbées passivement au fond de 3 cupules différentes d'une microplaque Nunc Maxisorp par incubation une nuit à +4°C.

La réactivité de sérums de porcs vis-à-vis de chacune de ces fractions antigéniques est évaluée par une réaction ELISA indirecte dont le protocole expérimental est détaillé ci-dessous :
- Etape de saturation : 200 µl/cupule de PBS1X/Lait demi- écrémé 3 %, incubation 1 h 30 à 37°C.
- Lavage : 200 µl/cupule de PBS1X/Tween 20 : 0,05 %, 3 lavages rapides.
- Etape d'incubation des sérums : 100 µl/cupule de sérum dilué au 1/100 en PBS1X/Lait demi-écrémé, 1 %/Tween 20 : 0,05 %, incubation 1 h à 37°C.
- Lavage : 200 µl/cupule de PBS1X/Tween 20 : 0,05 %, 2 lavages rapides suivis de 2 lavages de 5 min.
- Etape d'incubation du conjugué : 50 µl/cupule de conjugué lapin anti-porc dilué au 1/1000 en PBS1X/Lait demi-écrémé, 1 %/Tween 20 : 0,05 %, incubation 1 h à 37°C.
- Lavage : 200 µl/cupule de PBS1X/Tween 20 : 0,05 %, 2 lavages rapides suivis de 2 lavages de 5 min.
- Etape de Révélation : 100 µl/cupule de substrat OPD / Tampon Citrate / H₂O₂, incubation 15 min à 37°C.
- Arrêt de Réaction : 50 µl/cupule H₂SO₄ 1N.
- Lecture au spectrophotomètre à 490 nm.

### Résultats

Les résultats obtenus sont présentés ci-dessous dans le tableau 9.

**Tableau 9 :**

| Antigènes | Réactivité Sérum Porc non inoculé par le Circovirus | Réactivité Sérum Porc inoculé par le Circovirus |
|---|---|---|
| Sf9 témoin purifié | 0,076 | 0,088 |
| Sf9 exprimant ORF'2 purifié | 0,071 | 1,035 |

Les résultats sont exprimés en densité optique mesurée au spectrophotométre à 490 nm lors de l'analyse par ELISA de la réactivité de sérums de porc inoculé ou non par le circovirus MAP type B selon le protocole indiqué ci-dessus.

### B - Diagnostic Sérologique par Peptide de Synthèse

La cartographie épitopique des protéines codées par exemple par les séquences nucléiques ORF1 et ORF2 des deux types de circovirus MAP (types A et B) a permis en autre d'identifier des épitopes circoviraux immunogènes sur les protéines codées par les séquences nucléiques ORF'1 et ORF'2 ainsi que les épitopes spécifiques de la protéine codée par la séquence nucléique ORF'2 du circovirus MAP type B. Quatre épitopes spécifiques du circovirus MAP type B et un épitope commun aux deux types de circovirus MAP situés sur la protéine codée par la séquence nucléique ORF'2 ont été synthétisés sous forme de peptide. Les peptides équivalents chez le circovirus de type A ont également été synthétisés. Tous ces peptides ont été évalués comme antigènes de diagnostic dans le cadre de la réalisation d'un test sérologique.

### Résultats

Les résultats obtenus sont représentés dans le tableau 10 ci-après.

**Tableau 10 : Résultats de l'évaluation comme antigène de diagnostic de peptides synthétiques codés par les séquences nucléiques ORF2 et ORF'2 de circovirus MAP de type A et B.**

| | | | | **Réactivité Sérum Porc Infecté Circovirus B** | | | |
|---|---|---|---|---|---|---|---|
| **Peptide** | **Type Circovirus MAP** | **Position** | **Séquence AA** | **EOPS J0/J54** | **Conventionnel 1 J0/J42** | **Conventionnel 2 J0/J42** | **Spécificité Epitopique** |
| 121 | B | 71-85 | VDMMRFNINDFLPPG | +/-, +++ | +/-, +++ | -, +++ | Circovirus B |
| 177 | A | 70-84 | NVNELRFNIGQFLPP | +/-, + | +/-, +/- | +/-, - | |
| 132 | B | 115-129 | QGDRGVGSSAVILDD | +/-, +/- | ++, ++ | +/-, + | Circovirus B |
| 188 | A | 114-127 | TSNQRGVGSTVVIL | +/-,- | -, +/- | +/-, +/- | |
| 133 | B | 119-134 | GVGSSAVILDDNVFTK | -, ++ | ++, +++ | +/-, ++ | |
| 189 | A | 118-132 | RGVGSTVVILDANFV | +/-,- | -, +/- | +/-, +/- | |
| 146 | B | 171-185 | FTIDYFQPNNKRNQL | -, +/- | -, ++ | -, ++ | Circovirus A&B |
| 202 | A | 170-184 | DQTIDWFQPNNKRNQ | +++, +++ | +/-, ++ | +, ++ | |
| 152 | B | 195-209 | VDHVGLGTAFENSIY | -, ++ | +++; +++ | +/-, + | Circovirus B |
| 208 | A | 194-208 | NVEHTGLGYALQNAT | **-,-** | **-, -** | **-, -** | |

| | | | | | | | |
|---|---|---|---|---|---|---|---|
| +/-, +, ++, +++. Intensités croissantes des réactivités observées en Spot-peptides sur membrane de nitrocellulose. Les sérums porcins testés sont issus d'animaux expérimentalement infectés par le circovirus de type B au sein des animaleries du CNEVA. Les animaux sont prélevés avant inoculation à J0 et 42 jours ou 54 jours après inoculation J42, J54. | | | | | | | |

### EXEMPLE 7 : Caractérisation des épitopes spécifiques du circovirus MAP de type B

Les protéines codées par l'ORF2 des circovirus porcins de type A et B ont été choisies pour cette étude. Pour chacune des ORF2 (types A et B), 56 peptides de 15 acides aminés qui se chevauchent tous les 4 acides aminés ont été synthétisés, recouvrant ainsi la totalité de la protéine (cf tableau 11 ci-après).

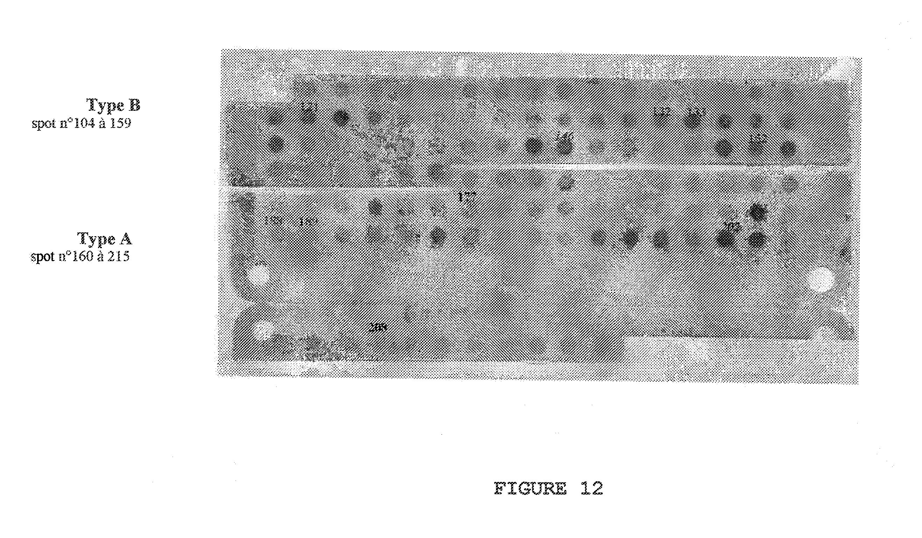

**Tableau 11 : Séquence d'acides aminés des 56 peptides de 15 acides aminés synthétisés à partir de la séquence nucléique ORF'2 (type B) et ORF2 (type A) de circovirus MAP avec leur numéro de spot correspondant (cf. figure 12)**

| **ORF'2 type B** | | **ORF2 type A** | |
|---|---|---|---|
| Spot n° | Séquence | Spot n° | Séquence |
| 104 | MTYPRRRYRRRRHRP | 160 | MTWPRRRYRRRRTRP |
| 105 | RRRYRRRRHRPRSHL | 161 | RRRYRRRRTRPRSHL |
| 106 | RRRRHRPRSHLGQIL | 162 | RRRRTRPRSHLGNIL |
| 107 | HRPRSHLGQILRRRP | 163 | TRPRSHLGNILRRRP |
| 108 | SHLGQILRRRPWLVH | 164 | SHLGNILRRRPYLVH |
| 109 | QILRRRPWLVHPRHR | 165 | NILRRRPYLVHPAFR |
| 110 | RRPWLVHPRHRYRWR | 166 | RRPYLVHPAFRNRYR |
| 111 | LVHPRHRYRWRRKNG | 167 | LVHPAFRNRYRWRRK |
| 112 | RHRYRWRRKNGIFNT | 168 | AFRNRYRWRRKTGIF |
| 113 | RWRRKNGIFNTRLSR | 169 | RYRWRRKTGIFNSRL |
| 114 | KNGIFNTRLSRTFGY | 170 | RRKTGIFNSRLSREF |
| 115 | FNTRLSRTFGYTVKR | 171 | GIFNSRLSREFVLTI |
| 116 | LSRTFGYTVKRTTVR | 172 | SRLSREFVLTIRGGH |
| 117 | FGYTVKRTTVRTPSW | 173 | REFVLTIRGGHSQPS |
| 118 | VKRTTVRTPSWAVDM | 174 | LTIRGGHSQPSWNVN |
| 119 | TVRTPSWAVDMMRFN | 175 | GGHSQPSWNVNELRF |
| 120 | PSWAVDMMRFNINDF | 176 | QPSWNVNELRFNIGQ |
| 121 | VDMMRFNINDFLPPG | 177 | NVNELRFNIGQFLPP |
| 122 | RFNINDFLPPGGGSN | 178 | LRFNIGQFLPPSGGT |
| 123 | NDFLPPGGGSNPRSV | 179 | IGQFLPPSGGTNPLP |
| 124 | PPGGGSNPRSVPFEY | 180 | LPPSGGTNPLPLPFQ |
| 125 | GSNPRSVPFEYYRIR | 181 | GGTNPLPLPFQYYRI |
| 126 | RSVPFEYYRIRKVKV | 182 | PLPLPFQYYRIRKAK |
| 127 | FEYYRIRKVKVEFWP | 183 | PFQYYRIRKAKYEFY |
| 128 | RIRKVKVEFWPCSPI | 184 | YRIRKAKYEFYPRDP |
| 129 | VKVEFWPCSPITQGD | 185 | KAKYEFYPRDPITSN |
| 130 | FWPCSPITQGDRGVG | 186 | EFYPRDPITSNQRGV |
| 131 | SPITQGDRGVGSSAV | 187 | RDPITSNQRGVGSTV |
| 132 | QGDRGVGSSAVILDD | 188 | TSNQRGVGSTVVILD |
| 133 | GVGSSAVILDDNFVT | 189 | RGVGSTVVILDANFV |
| 134 | SAVILDDNFVTKATA | 190 | STVVILDANFVTPST |
| 135 | LDDNFVTKATALTYD | 191 | ILDANFVTPSTNLAY |
| 136 | FVTKATALTYDPYVN | 192 | NFVTPSTNLAYDPYI |
| 137 | ATALTYDPYVNYSSR | 193 | PSTNLAYDPYINYSS |
| 138 | TYDPYVNYSSRHTIT | 194 | LAYDPYINYSSRHTI |
| 139 | YVNYSSRHTITQPFS | 195 | PYINYSSRHTIRQPF |
| 140 | SSRHTITQPFSYHSR | 196 | YSSRHTIRQPFTYHS |
| 141 | TITQPFSYHSRYFTP | 197 | HTIRQPFTYHSRYFT |
| 142 | PFSYHSRYFTPKPVL | 198 | QPFTYHSRYFTPKPE |
| 143 | HSRYFTPKPVLDFTI | 199 | YHSRYFTPKPELDQT |
| 144 | FTPKPVLDFTIDYFQ | 200 | YFTPKPELDQTIDWF |
| 145 | PVLDFTIDYFQPNNK | 201 | KPELDQTIDWFQPNN |
| 146 | FTIDYFQPNNKRNQL | 202 | DQTIDWFQPNNKRNQ |
| 147 | YFQPNNKRNQLWLRL | 203 | DWFQPNNKRNQLWLH |
| 148 | NNKRNQLWLRLQTAG | 204 | PNNKRNQLWLHLNTH |
| 149 | NQLWLRLQTAGNVDH | 205 | RNQLWLHLNTHTNVE |
| 150 | LRLQTAGNVDHVGLG | 206 | WLHLNTHTNVEHTGL |
| 151 | TAGNVDHVGLGTAFE | 207 | NTHTNVEHTGLGYAL |
| 152 | VDHVGLGTAFENSIY | 208 | NVEHTGLGYALQNAT |
| 153 | GLGTAFENSIYDQEY | 209 | TGLGYALQNATTAQN |
| 154 | AFENSIYDQEYNIRV | 210 | YALQNATTAQNYVVR |
| 155 | SIYDQEYNIRVTMYV | 211 | NATTAQNYVVRLTIY |
| 156 | QEYNIRVTMYVQFRE | 212 | AQNYVVRLTIYVQFR |
| 157 | IRVTMYVQFREFNFK | 213 | VVRLTIYVQFREFIL |
| 158 | MYVQFREFNFKDPPL | 214 | TIYVQFREFILKDPL |
| 159 | VQFREFNFKDPPLNP | 215 | YVQFREFILKDPLNE |

Ces peptides ont été synthétisés selon la méthode «spot» qui consiste en une synthèse simultanée d'un grand nombre de peptides sur un support solide de cellulose, chaque lieu de synthèse d'un peptide constituant un spot (Synt:em, NIMES). Cette méthode implique une orientation des peptides sur la plaque, ceux-ci étant fixés de façon covalente par l'extrémité carboxy-terminale. Un spot représente environ 50 nmole de peptide.

La référence des spots et des séquences peptidiques correspondantes est donnée dans le tableau 11.

Ces membranes ont été utilisées pour des tests d'immunoréactivité vis-à-vis de sérum de porcs EOPS infectés ou non expérimentalement par la souche circovirale MAP type B ainsi que vis-à-vis de sérums de porcs infectés, issus d'élevages conventionnels (élevages conventionnels 1 ou 2). Cette étude a permis de mettre en évidence des peptides immunoréactifs spécifiques du circovirus de type B correspondant aux spots N° 121, N° 132, N° 133 et N° 152 (respectivement de séquences d'acides aminés SEQ ID N° 17, SEQ ID N° 18, SEQ ID N° 19 et SEQ ID N° 20). Une illustration est présentée dans la figure 12 où les membranes sont révélées avec un sérum de porc infecté, provenant d'un élevage conventionnel. Des peptides immunoréactifs non spécifiques de type ont également été mis en évidence parmi lesquels nous retiendrons le peptide N° 146 qui est fortement immunogène.

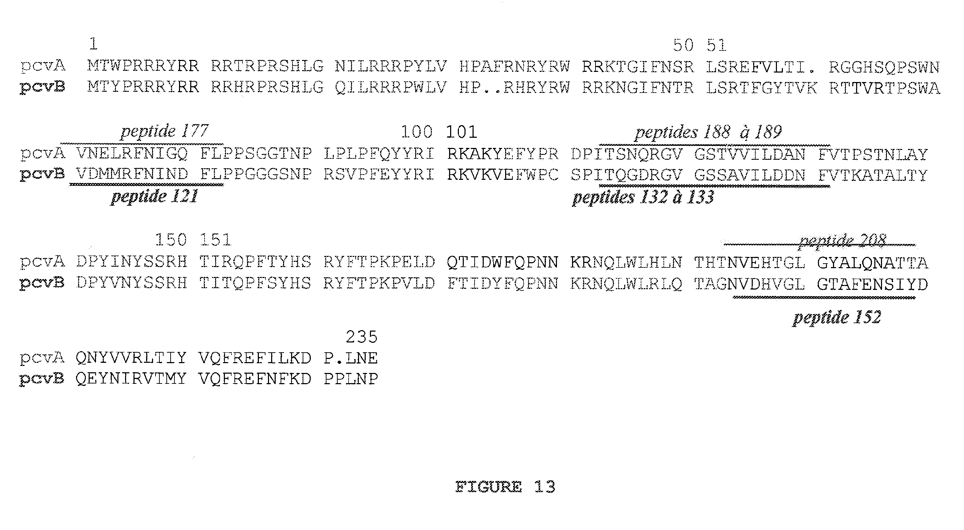

Une comparaison entre les séquences peptidiques des circovirus de type A et B (figure 13) indique une divergence allant de 20 à 60 % pour les peptides immunoréactifs spécifiques du type B, et une divergence plus faible (13 %) entre les peptides non spécifiques.

### Références bibliographiques

Allan, G. M. et al., 1995, Vet. Microbiol., 44 : 49-64.
Barany, F., 1911, PNAS. USA, 88 : 189-193.
Boulton, L.H. et al., 1997, J. Gen. Virol., 78 (Pt 6), 1265-1270.
Buckholz, R.G., 1993, Yeast systems for the expression of heterologous gene products. Curr. Op. Biotechnology 4 : 538-542.
Burg, J.L. et al., 1996, Mol. and Cell. Probes, 10 : 257-271.
Chu, B.C.F. et al., 1986, NAR, 14 : 5591-5603.
Chu, P.W.G. et al., 1993, Virus Research, 27 : 161-171.
Clark, E.G., 1997, American Association of Swine Practitioners, 499-501.
Daft, B. et al., 1996, American Association of Veterinary Laboratory Diagnosticians, 32.
Derse, D. et al., 1995, J. Virol., 69(3): 1907-1912.
Duck, P. et al., 1990, Biotechniques, 9 : 142-147.
Dulac, G.C. et al., 1989, Can. J. Vet. Res., 53 : 431-433.
Edwards, C.P., and Aruffo, A., 1993, Current applications of COS cell based transient expression systems. Curr. Op. Biotechnology 4 : 558-563.
Edwards, S. et al., 1994, Vet. Rec., 134 :680-681.
Erlich, H.A., 1989, In PCR Technology. Principles and Applications for DNA Amplification. New York: Stockton Press.
Felgner, et al., 1987, Proc. Natl. Acad. Sci., 84 : 7413.
Fontes, E.P.B. et al., 1994, J. Biol. Chem., Vol. 269, N° 11 : 8459-8465.
Fraley et al., 1980, J. Biol. Chem., 255 : 1043 1.
Guateli, J.C. et al., 1990, PNAS. USA, 87 : 1874-1878.
Hackland, A.F. et al., 1994, Arch. Virol., 139: 1-22.
Hanson, S.F. et al., 1995, Virology, 211 : 1-9.
Harding, J.C., 1997, American Association of Swine Practitioners, 503.
Harding, R.M. et al., 1993, Journal of General Virology, 74 : 323-328.
Harding, J.C. et Clark, E.G., 1997, Swine Health and Production, Vol. 5, N° 5 201-203.
Heyraud-Nitschke, F. et al., 1995, Nucleic Acids Research, Vol. 23, N° 6.
Homer, G.W., 1991, Surveillance 18(5): 23.
Houbenweyl, 1974, in Meuthode der Organischen Chemie, E. Wunsch Ed., Volume 15-I et 15-11, Thieme, Stuttgart.
Huygen, K. et al., 1996, Nature Medicine, 2(8): 893-898.
Innis, M.A. et al., 1990, in PCR Protocols. A guide to Methods and Applications. San Diego : Academic Press.
Kaneda, et al., 1989, Science, 243 : 375.
Kievitis, T. et al., 1991, J. Virol. Methods, 35 : 273-286.
Kohler, G. et al., 1975, Nature, 256(5517) : 495-497.
Kwoh, D.Y. et al., 1989, PNAS. USA, 86: 1173-1177.
Ladany, S. et. al., 1989, J. Clin. Microbiol. 27 : 2778-2783.
Lazarowitz, S. G. et al., 1989, The EMBO Journal, Vol. 8 N° 4: 1023-1032.
Luckow, V.A., 1993, Baculovirus systems for the expression of human gene products. Curr. Op. Biotechnology 4 : 564-572.
Mankertz, A. et al., 1997, J. Virol., 71 : 2562-2566.
Matthews, J.A. et al., 1988, Anal. Biochem., 169: 1-25.
McNeilly, F. et al., 1996, Vet. Immunol. Immunopathol., 49: 295-306.
Meehan, B.M. et al., 1997, J. Gen. Virol., 78 : 221-227.
Merrifield, R.D., 1966, J. Am. Chem. Soc., 88(21) : 5051-5052.
Midoux, 1993, Nucleic Acids Research, 21 : 871-878.
Miele, E.A. et al., 1983, J. Mol. Biol., 171 :281-295.
Murphy, F.A. et al., 1995, Sixth Report of the International Committee on Taxonomy of Viruses. Springer-Verlag Wien New York.
Nayar, G.P. et al., 1997, Can. Vet. J. 38(6) : 385-386.
Olins, P.O., and Lee, S.C., 1993, Recent advances in heterologous gene expression in E. coli. Curr. Op. Biotechnology 4 520-525.
Pagano et al., 1967, J. Virol., 1 : 891.
Rolfs, A. et al., 1991, In PCR Topics. Usage of Polymerase Chain reaction in Genetic and Infectious Disease. Berlin : Springer-Verlag.
Sambrook, J. et al., 1989, In Molecular cloning : A Laboratory Manual. Cold Spring Harbor, NY : Cold Spring Harbor Laboratory Press.
Sanchez-Pescador, R., 1988, J. Clin. Microbiol., 26(10): 1934-1938.
Segev D., 1992, in «Non-radioactive Labeling and Detection of Biomolecules». Kessler C. Springer Verlag, Berlin, New-York : 197-205.
Shiver, J.W., 1995, in Vaccines 1995, eds Chanock, R.M. Brown, F. Ginsberg, H.S. & Norrby, E., pp.95-98, Cold Spring Harbor Laboratory Press, Cold Spring Harbor, New York.
Tascon, R.E et al., 1996, Nature Medicine, 2(8) : 888-892.
Tischer, 1. et al., 1982, Nature, 295 : 64-66.
Tischer, 1. et al., 1986, Arch. Virol., 91 :271-276.
Tischer, 1. et al., 1988, Zentralbl Bakteriol Mikrobiol Hyg [A] 270 : 280-287.
Tischer, I. et al., 1995, Arch. Virol., 140 : 737-743.
Urdea, M.S., 1988, Nucleic Acids Research, 11 : 4937-4957.
Walker, G.T. et al., 1992, NAR 20: 1691-1696.
Walker, G.T. et al., 1992, PNAS. USA, 89: 392-396.
White, B.A. et al., 1997, Methods in Molecular Biology, 67, Humana Press, Towota.
Zhao, T.M. et al., 1996, Proc. Natl. Adac. Sci., USA, 93(13) : 6653-6658.

## Revendications

1. Anticorps mono- ou polyclonal capable de reconnaître de manière spécifique un polypeptide isolé ayant la séquence SEQ ID N°17.

2. Anticorps mono- ou polyclonal capable de reconnaître de manière spécifique un polypeptide isolé ayant la séquence SEQ ID N°18.

3. Anticorps mono- ou polyclonal capable de reconnaître de manière spécifique un polypeptide isolé ayant la séquence SEQ ID N°19.

4. Anticorps mono- ou polyclonal capable de reconnaître de manière spécifique un polypeptide isolé ayant la séquence SEQ ID N°20.

5. Anticorps mono- ou polyclonal selon l'une des revendications 1 à 4, **caractérisé en ce qu'**il s'agit d'un anticorps monoclonal.

6. Procédé de détection et/ou d'identification d'un circovirus MAP de type B dans un échantillon biologique **caractérisé en ce qu'**il comprend la mise en contact de l'échantillon biologique avec un anticorps mono- ou polyclonal selon l'une des revendications 1 à 5 et la mise en évidence des complexes antigène-anticorps éventuellement formés.

7. Kit ou nécessaire de détection et/ou d'identification d'un circovirus MAP de type B dans un échantillon biologique comprenant :
a) un anticorps mono- ou polyclonal selon l'une des revendications 1 à 5,
b) un réactif pour la constitution d'un milieu propice à la réalisation d'une réaction antigénique.

## Patentansprüche

1. Mono- oder polyklonaler Antikörper, der zur speziellen Erkennung eines isolierten Polypeptids mit der Sequenz SEQ ID Nr. 17 fähig ist.

2. Mono- oder polyklonaler Antikörper, der zur speziellen Erkennung eines isolierten Polypeptids mit der Sequenz SEQ ID Nr. 18 fähig ist.

3. Mono- oder polyklonaler Antikörper, der zur speziellen Erkennung eines isolierten Polypeptids mit der Sequenz SEQ ID Nr. 19 fähig ist.

4. Mono- oder polyklonaler Antikörper, der zur speziellen Erkennung eines isolierten Polypeptids mit der Sequenz SEQ ID Nr. 20 fähig ist.

5. Mono- oder polyklonaler Antikörper gemäß einem der Ansprüche 1 bis 4, **dadurch gekennzeichnet, dass** es sich um einen monoklonalen Antikörper handelt.

6. Verfahren zur Detektion und/oder Identifikation eines Circovirus MAP des Typs B in einer biologischen Probe, **dadurch gekennzeichnet, dass** es das Inkontaktbringen der biologischen Probe mit einem mono-oder polyklonalen Antikörper gemäß einem der Ansprüche 1 bis 5 und den Nachweis von gegebenenfalls gebildeten Antigen-Antikörper-Komplexen umfasst.

7. Kit oder Testsatz zur Detektion und/oder Identifikation eines Circovirus MAP des Typs B in einer biologischen Probe, Folgendes umfassend:
a) ein mono- oder polyklonaler Antikörper gemäß einem der Ansprüche 1 bis 5,
b) ein Reagenz für die Herstellung eines geeigneten Milieus zur Durchführung einer Antigen-Reaktion.

## Claims

1. Mono- or polyclonal antibody, capable of specifically recognizing an isolated polypeptide having the sequence SEQ ID N°17.

2. Mono- or polyclonal antibody, capable of specifically recognizing an isolated polypeptide having the sequence SEQ ID N° 18.

3. Mono- or polyclonal antibody capable of specifically recognizing an isolated polypeptide having the sequence SEQ ID N° 19.

4. Mono- or polyclonal antibody, capable of specifically recognizing an isolated polypeptide having the sequence SEQ ID N°20.

5. Mono- or polyclonal antibody according to any one of claims 1 to 4, **characterized in that** it is a monoclonal antibody.

6. Method for the detection and/or the identification of PWD circovirus of type B in a biological sample, **characterized in that** it comprises contacting said biological sample with a mono- or a polyclonal antibody according to any one of claims 1 to 5, and demonstrating the antigen-antibody complexes potentially formed.

7. Kit or set for the detection and/or the identification of a PWD circovirus of type B in a biological sample, comprising:
a) A mono- or a polyclonal antibody according to any one of claims 1 to 5,
b) A reagent for the constitution of a medium adapted to an antigenic reaction.
